(19) **Europäisches Patentamt / European Patent Office / Office européen des brevets**

(11) **EP 4 134 091 B1**

(12) **EUROPEAN PATENT SPECIFICATION**

(45) Date of publication and mention
of the grant of the patent:
**10.12.2025 Bulletin 2025/50**

(21) Application number: **21785025.4**

(22) Date of filing: **07.04.2021**

(51) International Patent Classification (IPC):
*A61K 36/53* *(2006.01)*   *A61K 31/192* *(2006.01)*
*A61K 31/216* *(2006.01)*   *A61P 3/04* *(2006.01)*
*A61P 27/02* *(2006.01)*   *A61P 17/06* *(2006.01)*
*A61P 15/02* *(2006.01)*   *A61P 35/00* *(2006.01)*
*A61P 35/04* *(2006.01)*   *A61P 9/10* *(2006.01)*

(52) Cooperative Patent Classification (CPC):
(C-Sets available)
**A61P 9/10; A61K 31/192; A61K 31/216;
A61K 36/53; A61P 3/04; A61P 15/02; A61P 17/06;
A61P 27/02; A61P 35/00; A61P 35/04**   (Cont.)

(86) International application number:
**PCT/KR2021/004375**

(87) International publication number:
**WO 2021/206455 (14.10.2021 Gazette 2021/41)**

(54) **FRACTION EXTRACT OF MELISSA OFFICINALIS LEAVES AND NOVEL PHARMACEUTICAL COMPOSITION INCLUDING SAME**

FRAKTIONSEXTRAKT AUS MELISSA OFFICINALIS-BLÄTTERN UND NEUARTIGE PHARMAZEUTISCHE ZUSAMMENSETZUNG DAMIT

EXTRAIT DE FRACTION DE FEUILLES DE MÉLISSE-CITRONNELLE ET NOUVELLE COMPOSITION PHARMACEUTIQUE LE COMPRENANT

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **08.04.2020  KR 20200042856
12.03.2021  KR 20210032830**

(43) Date of publication of application:
**15.02.2023 Bulletin 2023/07**

(73) Proprietors:
• **ANGIOLAB, INC.**
  **Daejeon 34016 (KR)**
• **Kim, Min Young**
  **Daejeon 34053 (KR)**

(72) Inventors:
• **PARK, Byung Young**
  **Daejeon 35228 (KR)**
• **PARK, Eun Kyu**
  **Pyeongtaek-si Gyeonggi-do 17896 (KR)**

• **LEE, Hee Suk**
  **Daejeon 34681 (KR)**
• **YUH, Hyung Soo**
  **Sejong  30126 (KR)**
• **KIM, Min Young**
  **Daejeon 34053 (KR)**

(74) Representative: **Louis Pöhlau Lohrentz
Patentanwälte
Postfach 30 55
90014 Nürnberg (DE)**

(56) References cited:
WO-A1-2014/014271   WO-A2-2009/025532
KR-A- 20090 020 285   KR-A- 20140 011 209
KR-A- 20150 035 245   KR-B1- 102 229 760
US-A1- 2004 009 244

**(Cont. next page)**

- **PEREIRA ROMAIANA PICADA ET AL: "Chemical composition, antioxidant and anticholinesterase activity of Melissa officinalis", INDUSTRIAL CROPS AND PRODUCTS, vol. 53, 1 February 2014 (2014-02-01), NL, pages 34 - 45, XP055796499, ISSN: 0926-6690, DOI: 10.1016/ j.indcrop.2013.12.007**
- **KIM MYUNGSUK, YOO GYHYE, RANDY AHMAD, SON YANG-JU, HONG CHI RAC, KIM SANG MIN, NHO CHU WON: "Lemon Balm and Its Constituent, Rosmarinic Acid, Alleviate Liver Damage in an Animal Model of Nonalcoholic Steatohepatitis", NUTRIENTS, vol. 12, no. 4, pages 1166, XP055856155, DOI: 10.3390/ nu12041166**
- **KIM, S. ; YUN, E.J. ; BAK, J.S. ; LEE, H. ; LEE, S.J. ; KIM, C.T. ; LEE, J.H. ; KIM, K.H.: "Response surface optimised extraction and chromatographic purification of rosmarinic acid from Melissa officinalis leaves", FOOD CHEMISTRY, ELSEVIER LTD., NL, vol. 121, no. 2, 15 July 2010 (2010-07-15), NL , pages 521 - 526, XP026916260, ISSN: 0308-8146, DOI: 10.1016/j.foodchem.2009.12.040**

(52) Cooperative Patent Classification (CPC): (Cont.)

C-Sets
**A61K 31/192, A61K 2300/00;**
**A61K 31/216, A61K 2300/00**

**Description**

**[Technical Field]**

The present invention relates to a fractional extract of *Melissa officinalis* leaf and a novel pharmaceutical and a food composition comprising the same as effective component.

**[Background Art]**

*Melissa officinalis* is an herbaceous perennial plant, which belongs to the *Labiataefamily,* and is also nicknamed Lemon Balm.

*A Melissa officinalis* leaf extract contains flavonoids, triterpene acids, volatile oils, glycosides of the alcoholic and phenolic compounds and caffeic acid derivatives. In particular, the flavonoids contained in *Melissa officinalis* leaves are cynaroside, cosmosin, rhamnocitrin, isoquercitrin, etc., and ursolic acid as triterpene acid. A *Melissa officinalis* leaf extract contains hydroxycinnamic acid derivatives such as rosmarinic acid, one of non-volatile ingredients which has been attracting attention recently, and geraniol, neral, citronellal and eugenol as volatile oils.

Non-alcoholic steatohepatitis (NASH) is a disease in which fat accumulates in the liver, causing hepatocyte ballooning and inflammation, followed by fibrosis which can lead to liver cirrhosis, and to further complication such as liver cancer in some cases.

The number of patients with NASH is rapidly increasing, but the pathogenesis of the disease is not clear and various causes are at work, making it difficult to treat with one mechanism or control of the cause, and there is no approved drug.

NASH progresses chronically, so there are generally no specific symptoms, but liver functions gradually deteriorate, such as increased activity of alkaline phosphatase (ALP) or aminotransferase, which is a measure of liver function.

NASH progresses to liver fibrosis or cirrhosis if left unattended, resulting in poor prognosis. Thus, it is necessary to suppress fat accumulation in the liver and additional inflammation and fibrosis to prevent NASH from progressing to liver cirrhosis, but no such treatment is suggested. Therefore, there is a need to develop effective therapeutics that can treat NASH.

Non-alcoholic fatty liver disease (NAFLD) refers to a condition in which triglycerides are excessively accumulated in the liver regardless of alcohol intake. If the triglyceride content in the liver is above the top 95% among healthy and thin people, or the proportion of triglyceride particles in the cytoplasm within the liver cell is above 5%, it is defined as a simple fatty liver among non-alcoholic fatty liver disease (NAFLD). NAFLD has been reported to occur in 10-24% of the general population and 58-74% of obese people.

Angiogenesis is a process through which new capillaries form from pre-existing microvessels. Normal angiogenesis occurs during embryonic development, tissue regeneration and wound healing, and the development of the corpus luteum, which is a periodic change in the female reproductive system, and in these cases it is tightly regulated. [Folkman and Cotran, Relation of vascular proliferation to tumor growth, Int Rev Exp Pathol 16, 207 - 248(1976)].

In adults, the vascular endothelial cells grow very slowly and do not divide relatively well as compared with other types of cells. However, the angiogenesis in adults is provoked depending on the stimulation of angiogenesis-stimulating factors, the release of pro-angiogenic cytokines from inflammatory cells, and the activation of hydrolytic enzymes that release the angiogenic mediators sequestered within the extracellular matrix.

Generally, the process of angiogenesis is the degradation of basement membrane of blood vessels by proteases, the migration of vascular endothelial cells, and the lumen formation via proliferation and differentiation of endothelial cells. One of the major events in the process of angiogenesis is a breakdown of the basement membrane surrounding vessels by enzymes, and the most important enzymes of matrix degradation are those belonging to the family of Matrix metalloproteinase (MMP).

It is known that pathological angiogenesis takes place when the failure in the regulation of angiogenesis occurs or MMPs, important enzymes in angiogenesis are highly activated, which is associated with many diseases (Ref. Polverini PJ, Critical Reviews in Oral Biology, 6(3), 1995, 230-247; Arup Das, et al., Progress in Retinal and Eye Research, 22, 2003, 721-748 ; Nick Di Girolamo, et al., IOVS, Vol. 42, No.9, August 2001, 1963-1968; Patricia Lee, et al., Survey of ophthalmology, vol 43, No. 3, Nov-Dec 1998, 245-269; D.B. Holland, et al., British Journal of Dermatology, 150, 2004, 72-81; Anthony H Vagnucci Jr, et al., The Lancet, vol 361, Feb. 15, 2003, 605-608; Berislav V. Zlokovic, Trends in Neuroscience, Vol. 28, No.4, April 2005, 202-208; Jaap G. Neels, et al., The FASEB Journal express article 10. 1096/fj.03-1101fje. Published online April 14, 2004 ; D.L. Crandall, et al., Microcirculation, 4, 1997, 211-232; G. Voros, et al., Endocrinology, 146, 2005, 4545-4554; M.A. Rupnick, et al., PNAS, 99, 2002, 10730-10735; E. Brakenhielm, et al., Circ. Res., 94, 2004, 1579-1588; H.R. Lijnen, et al., Arterioscler Thromb Vasc Biol., 22, 2002, 374-379; D. Demeulemeester, et al., Biochem. Biophys. Res. Commun., 329, 2005, 105-110).

An example of angiogenesis-related disease or MMP (Matrix metalloproteinase)-mediated disease is obesity in which excess fat accumulates in the body due to an imbalance in energy intake and consumption that can cause various health problems. The cause is most likely due to lifestyle such as excessive intake of nutrients and lack of physical activity, and in rare cases, it can occur secondary due to drug use or disease.

According to the World Health Organization (WHO) statistics of 2014, 1.9 billion adults over the age of 18 were overweight, of which 600 million people were obese worldwide. Obesity is one of the leading factors that increase the incidence of cardiovascular diseases, Type 2 diabetes and several types of cancer which lead to the death of more than 2.8 million people each year due to overweight or obesity.

As the living standards of people improve, obesity has become a global social problem. According to the National Health and Nutrition Examination Survey of 2015, the fat intake of Koreans increased by 5.9 g over the past 10 years, and the prevalence of obesity increased by 1.9% compared with 2005. In particular, the prevalence of obesity in men increased significantly to 39.7%, and the prevalence of hypercholesterolemia increased by 9.9% to 17.9%.

Several drugs have been developed to improve obesity, which are largely divided into appetite suppressants and fat absorption inhibitors according to their mechanism of action. However, drugs such as Phentermine and Diethylpropion that suppress appetite have side effects such as increase in blood pressure, dizziness, headache, tremor, and dry mouth. In the case of orlistat, a fat absorption inhibitor, the absorption of fat-soluble vitamins is inhibited, and has side effects such as steatorrhea, fat excretion, frequent defecation, and fecal incontinence.

Therefore, the need for the development of safe drug with fewer side effects to prevent or treat obesity has been raised.

Macular degeneration, an example of angiogenesis-related disease or matrix metalloproteinase (MMP)-mediated disease, is a disease that causes visual impairment due to degeneration by various causes in the macula lutea where the concentration of photoreceptor cells is very high and receives light most clearly and accurately. Macular degeneration is one of the three leading causes of blindness along with glaucoma and diabetic retinopathy. The biggest cause of macular degeneration is age and other causes are family history, race, and smoking. When the macular is damaged, the eye loses the ability to recognize details such as small print, facial features, or small objects.

There are two types of macular degeneration: non-exudative (dry) macular degeneration and exudative (wet) macular degeneration, and 90% of people with macular degeneration have the dry form. In dry macular degeneration, waste material collects under the macula, forming yellow deposits called drusen. The presence of drusen interferes with blood flow to the retina, particularly the macula, and when the blood flow is reduced, the supply of nutrients to the macula is reduced, thereby stopping or atrophying the efficient action of photosensitive cells. In wet macular degeneration, new weak blood vessels grow in or under the retina, causing fluid and blood to leak into the space below the macula.

Although macular degeneration was considered a disease that occurs frequently among the elderly, it is known that the number of patients in their 40s and 50s is increasing rapidly in recent years. Westernization of diet, such as the increase in fat intake, has been pointed out as one of the main reasons for the decrease in the age of onset of macular degeneration.

Lutein is a naturally occurring carotenoid that exists in a natural state without vitamin A activity, and is contained in large amounts in green and yellow vegetables. Lutein, one of the most abundant carotenoids in food products and human blood, is known to play a role in antioxidant effects and protecting plants from UV rays. For humans, it is known as the main constituent of the macula and lens of the eye, which plays an important role in improving eye health and vision.

However, a research team at the Moran Eye Center of the University of Utah reported in 'JAMA Ophthalmology', a journal of the American Medical Association, that a round, yellowish crystal-like substance was found inside the fovea of a woman who took an excessive amount of lutein for a long period. In the case of the patient above, it has been reported that she took 20mg of lutein supplement on a daily basis for the last 8 years and that she also consumed lutein-rich foods such as spinach, broccoli, kale, and avocado. Therefore, the research team assumed that lutein precipitated in the eyeball, crystals were formed, and macular degeneration occurred.

Therefore, although lutein has an effect of improving vision in macular degeneration, side effects such as macular degeneration appear when consumed for a long period of time or in excess, so it is necessary to develop a method for preventing, alleviating or suppressing it.

Psoriasis, an example of angiogenesis-related or matrix metalloproteinase (MMP)-mediated disease, is a chronic inflammatory disease that is covered with white or silvery scales, and has a clear boundary of red papule or plate-like rash of various sizes repeatedly occurring on the skin of the body. It is a dermatologic disease characterized by epidermal proliferation and dermis inflammation histologically, which occurs at a frequency of 1 to 2% of the population. It is a chronic skin disease in which small millet-like rashes form on the skin and white dandruff-like dead skin cells build up on top of the rash. So, if they spread a lot, almost all the skin of the body is covered with rashes.

In psoriasis, as the number of skin cells that make dead skin cells increase rapidly, dandruff-like dead skin cells build up on top of the skin. The exact cause of psoriasis isn't fully understood, but it is thought to be an immune system problem and besides that, genetic factors, environmental factors, drugs, skin irritation, dryness, upper respiratory tract inflammation, mental stress, etc. are mentioned as factors that cause or worsen psoriasis.

Vitamin D analogues, narrow-band UVB treatment, Psoralen plus ultraviolet A (Bath-PUVA), and cyclosporine are used to treat psoriasis. There is still no known causative therapy for psoriasis. This is because the pathological cause of the disease is not clearly identified. Treatment methods such as vitamin D derivative ointment, narrow-band UVB treatment, bath-PUVA, and cyclosporin are only temporary symptom relief treatment, so they cannot be called causative therapy because psoriasis recurs.

Endometriosis, is an example of angiogenesis-related disease or matrix metalloproteinase (MMP)-mediated disease, in which the endometrium or similar tissue occurs outside the uterus depending on the increase of estrogen. It is a benign disease that causes pain including menstrual cramps and decreases fertility, which leads to a significant decline in the quality of life for women in social and reproductive activities. In endometriosis, dysmenorrhea appears to be very frequent, and pain symptoms such as lower abdominal pain, back pain, dyspareunia, and defecation pain other than menstruation are also confirmed with high frequency.

Many patients with endometriosis recur or repeat recurrence until menopause unless radical surgery is performed, which requires long-term treatment and management. Drug therapy is often the first choice for the treatment of endometriosis. It is largely divided into symptomatic therapy and endocrine therapy. For symptomatic therapy, analgesic drugs are mainly used for improving the pain caused by endometriosis. For endocrine therapy, low-dose estrogen-progestin preparations, dienogest, and gonadotropin-releasing hormone (GnRH) agonists are used for suppressing estrogen-dependent endometrial proliferation in addition to pain relief.

However, it is said that pain associated with endometriosis cannot be controlled with analgesic drugs in 10% to 30% of endometriosis patients. Also, caution is required for thrombosis and liver dysfunction in the use of low-dose estrogen-progestin preparations. It has been reported that dienogest has a side effect of irregular genital bleeding of 71.9% in a long-term administration study and may lead to severe anemia. GnRH agonists cannot be administered for more than 6 months in principle due to the decrease of bone mineral density based on an estrogen-lowering action in some cases.

As described above, in drug therapy for endometriosis treatment, there are many patients who find it difficult to continue administration because it has been confirmed that each drug has its own side effect. Therefore, there is a demand for the development of a new drug that can be administered over a long period of time with reduced side effects.

Cancer, an example of angiogenesis-related disease or matrix metalloproteinase (MMP)-mediated disease, is one of the most common causes of death worldwide. About 10 million new cases occur each year, accounting for about 12% of all deaths, makes it the third leading cause of death.

Among cancers, breast cancer is the most common malignant tumor in women that causes more than 40,000 deaths annually, and early diagnosis is very important. Despite the many known anticancer drugs, the survival rate has not improved if the cancer is very advanced or has metastasized

Chemotherapy, a representative anticancer therapy has been used as the most effective treatment for cancer when used by itself or in combination with other therapies. However, although the efficacy of anticancer drugs in chemotherapy depends on the ability to kill cancer cells, there is a problem that drugs can act not only on cancer cells but also on normal cells, requiring drugs to treat cancer efficiently.

Arteriosclerosis also called atherosclerosis, is an example of angiogenesis-related or matrix metalloproteinase (MMP)-mediated disease, in which the arteries harden due to thickening or tissue degeneration of the walls of the arteries. As the intima of the arteries thicken, the inner diameter narrows and disturbs the blood flow that supplies oxygen and various nutrients, leading to various diseases. Arteriosclerosis is likely to occur in the cerebral artery or coronary artery. In the case of cerebral arteriosclerosis, headache, dizziness, and mental disorders appear and cause encephalomalacia. In the case of coronary arteriosclerosis, it is known to cause pain and arrhythmia in the heart, causing angina pectoris and myocardial infarction. In addition, arteriosclerosis is deeply related to the occurrence and progression of diseases such as metabolic syndromes including truncal obesity, obesity (overweight), thrombosis, hypercoagulation, and thrombotic conditions (arterial and venous), or dyslipidemia. It also causes or exacerbates diabetic complications.

In addition, when the blood triglyceride concentration is increased due to obesity, hyperlipidemia, etc., macrophages are converted into foam cells, or the rate of death increases by triglycerides. Thus, as macrophages die, their immune function and triglyceride metabolism function are lowered, which lowers the removal function of the foam cells, increasing the possibility of developing or worsening arteriosclerosis. Therefore, to prevent the onset or exacerbation of arteriosclerosis, it is important to ensure that macrophages maintain their function of removing foam cells or to minimize death by triglycerides even when blood fat concentration is high.

Currently, statin-based drugs such as rosuvastatin and simvastatin are mainly used for the treatment of arteriosclerosis, but it is known that it has a side effect of reducing the activity of macrophages that remove foam cells. In addition, statin-based drugs have been pointed out as a problem in that they cause side effects such as diabetes, muscular dystrophy, and hyperglycemia, so there is a need for research on natural products with fewer side effects.

Arthritis, an example of angiogenesis-related disease or matrix metalloproteinase (MMP)-mediated disease, is largely divided into rheumatoid arthritis and osteoarthritis (degenerative arthritis). Rheumatoid arthritis is a chronic, inflammatory, multisystem disorder that typically involves the joints. Symptoms include pain and swelling with inflammation of various joints such as fingers, hands, feet, wrists, ankles, and knees, and also cause abnormalities in various organs such as muscles, skin, lungs, and eyes. Genetic factors, pathogenic infections, and immune abnormalities have been suggested as the causes of arthritis but they are not precisely known.

As for the pathogenesis, due to hypersensitivity to autologous or foreign antigens interlerkin-1 (IL-1), tumor necrosis factor (TNF), prostaglandin E2 (PGE2), collagenase, proteinase, substance-P, etc. released from immune cells reduce cartilage and collagen, and induce the proliferation and inflammation of synovial fluids, which eventually leads to destruction of cartilage and bone.

Osteoarthritis refers to a degenerative condition of the synovial joint and is also called degenerative arthritis. It is a disease that occurs in middle-aged or old ages as a phenomenon of aging and causes movement disorders after the age of 65. The main causes are thought to be caused by obesity, hyperkinesia syndrome, recurrent dislocation, recurrent hemarthrosis, internal joint dislocation, inflammatory arthritis, and gout, etc.

Osteoarthritis is mainly involved in progressive damage of the articular cartilage. As arthritis progresses, the production of inflammatory cytokines such as tumor necrosis factor-a (TNF-$\alpha$), and interleukin-1$\beta$ (IL-1$\beta$), and nitric oxide (NO), etc., increase which causes severe pain in tissues of muscles, tendons, and ligaments.

The treatment methods for arthritis currently used are largely divided into non-pharmaceutical treatment, drug treatment, and surgical treatment. Non-pharmaceutical treatment methods include reduction of joint load, patella fixation, heat treatment, and exercise. Drug treatment includes oral medications and intra-articular injections. The drugs used in initial treatment are non-steroidal anti-inflammatory drugs (NSAIDs). Although these NSAIDs simply reduce pain and relieve symptoms, they cannot prevent joint cartilage loss or disease progression. When taken for a long time, it may cause heartburn and gastric bleeding due to side effects of gastrointestinal tract, kidney, heart, and liver as well as blood clotting mechanisms. Therefore, there is a need for research on natural products with fewer side effects.

Inflammatory bowel disease (IBD), an example of angiogenesis-related disease or matrix metalloproteinase (MMP)-mediated disease, causes chronic inflammation or ulceration in the mucous membranes of the large intestine and small intestine, and it is an incurable disease in which diarrhea and bloody stools continue and recur in the long-term.

Inflammatory bowel disease is a more common disease among Westerners, but it has been increasing rapidly in Korea since the 1980s. The prevalence of IBD is 15-35 years old and is reported by all age group, of which 15% are over 60 years old, and about 15% of IBD patients have a family history in an immediate family.

Although the exact etiology of inflammatory bowel disease is still unclear, inflammatory mediators and the activation of immune cells are presumed to be important etiologies due to autoimmune diseases along with environmental or genetic factors.

Inflammatory bowel disease is classified into two conditions, ulcerative colitis and Crohn's disease, which are clinically similar but differ from each other in histological, endoscopic and immunological aspects. Inflammatory mediators and the activation of immune cells are known to be an important etiology for this disease.

The persistent or inappropriate activation of the intestinal immune system plays an important role in the pathophysiology of chronic mucositis inflammation, and in particular, infiltration of neutrophils, macrophages, lymphocytes and mast cells eventually leads to mucosal destruction and ulceration.

In the course of the pathogenesis of inflammatory bowel disease, inflammatory cytokines such as TNF-$\alpha$ (Tumor necrosis factor-a), interleukin-6 (IL-6) and interleukin-8 (IL-8) play a major role.

In particular, TNF-$\alpha$ is highly expressed in the colon lumen and colonic epithelial cells of ulcerative colitis patients, and according to recent studies TNF-$\alpha$ is known to play an important role in the pathogenesis of ulcerative colitis. Infliximab, an anti-TNF-$\alpha$ antibody, is known to be effective in the treatment of Crohn's disease which was incurable. However, these treatments are expensive and cause side effects such as fluid reactions or infectious complications in some patients.

Current treatments for inflammatory bowel disease use 5-aminosalicylic acid (5-ASA) drugs that block the production of prostaglandins such as sulfasalazine, mesalazine or immunosuppressants of steroids. In case of not responding to steroid treatment immunosuppressants such as azathioprine, 6-mercaptopurine, and cyclosporine are also used, but there is no drug that can be expected to cure.

Alzheimer's disease, an example of angiogenesis-related disease or matrix metalloproteinase (MMP)-mediated disease, is the most common cause of dementia which accounts for about 60 to 80% of dementia. About 33.9 million people worldwide have Alzheimer's disease, and after 40 years, the prevalence is expected to triple with increasing life expectancy. Currently, there is no drug that can be expected to provide a clear improvement in Alzheimer's disease, and since the onset of the disease goes through a process of worsening for several decades, research around the world is attracting attention on the prevention of the Alzheimer's disease.

Alzheimer's disease is known to be caused by a complex interaction of various risk factors. Along with genetic risk factors, demographic risk factors such as age, sex, and educational background and environmental risk factors such as smoking, drinking, nutrition and social activity are known so far. Representative genetic markers for Alzheimer's include apolipoprotein E (APOE) $\varepsilon$4, but these genetic markers are not altered or modified. However, acquired factors (e.g., vascular risk factors, lifestyle) are modifiable factors, and thus, in addition to studies investigating how these modifiable factors regulate the onset of dementia, it is possible to reduce or delay the onset of dementia by inversely regulating these factors.

Among Alzheimer's treatment drugs, Memantine was developed in the 1980s by Merz Pharmaceuticals in Germany as a treatment for Parkinson's disease and movement disorders and also has been marketed as a treatment for dementia in Germany. As a study result was announced in 1999 that it was effective in treating severe Alzheimer's disease, it was approved as a treatment for Alzheimer's disease in Europe in 2002. In October 2003, it was approved by the US FDA as a treatment for moderate to severe Alzheimer's disease. Recently, it is also marketed as EBIXA® by Lundbeck in Korea. Evixa commercially available contains memantine hydrochloride in tablet or liquid form, and its administration is made within a range that does not exceed the maximum required dose of 20 mg per day in consideration of both efficacy and tolerability.

Memantine's mechanism of action is to block glutamate, an excitatory neurotransmitter. Glutamate overstimulates the nerves in the brain and causes excessive calcium influx into the cranial nerve cells, causing damage and death of the cranial nerves and memantine is known to prevent this. It is known that the calcium ion channel of the NMDA receptor is always opened even during the resting phase due to excessive secretion of glutamate in Alzheimer's patients, resulting in excessive calcium influx into the cell, which is associated with the death of cranial nerves. Memantine blocks the influx of calcium into cells by acting as a low-affinity antagonist in the calcium channel located at the center of the NMDA receptor. It reduces the excessive excitability of nerve cells due to excessive calcium influx into cells and when physiological actions such as memorization and learning are needed, it exits the calcium channel and causes depolarization to occur normally so that it stabilizes physiological nerve signals to normalize the function of nerve cells.

However, as side effects of memantine, loss of appetite, diarrhea, weight loss, dry mouth, dizziness, sleep disturbance, and fatigue, etc. have been reported. In addition, it has been reported to rarely cause dizziness, headache, constipation, drowsiness, and high blood pressure. Therefore, there is a need for research on natural products with fewer side effects.

Periodontal disease, an example of angiogenesis-related disease or - matrix metalloproteinase (MMP)-mediated disease, is a type of inflammation that occurs in supporting tissues around teeth by toxins which are metabolites of microorganisms living in the oral cavity. These periodontal diseases start from gingivitis in the early stages, and if left untreated, gingivitis develops into periodontal disease accompanied by swelling of the gums, bleeding and severe bad breath. In addition, persistent periodontal disease develops into progressive periodontal disease in which collagen supporting the periodontal membrane is destroyed and the alveolar bone supporting the teeth is dissolved, the periodontal ligaments are separated to form a periodontal pocket which can damage the teeth in severe cases.

The etiology of periodontal disease is different depending on gender, race, and age. This periodontal disease worsens for several years and repeats a lulled state as the infection state continues. Its incidence rate is high in patients with systemic diseases such as diabetes, AIDS, neutropenia, and Down's syndrome.

The most effective method for preventing periodontal disease can be achieved by using a substance that inhibits the activity of these microorganisms and facilitates blood flow to the inflamed gums. However, among the drugs used for the prevention and/or treatment of periodontal disease, antibiotics such as penicillin, erythromycin, and tetracycline are effective in sterilization and growth inhibition, but have serious side effects such as causing the emergence of resistant bacteria. In the case of antibacterial agents like chlorhexidine, it has an effect of inhibiting the formation of caries, but can be a problem because of strong toxicity. Therefore, there is a need for research on natural products with fewer side effects.

Diabetic retinopathy, an example of angiogenesis-related disease or matrix metalloproteinase (MMP)-mediated disease, is more specific for hyperglycemia than other chronic diabetes-related complications.

Depending on the degree of progression, diabetic retinopathy can be divided into early non-proliferative diabetic retinopathy (NPDR) and late proliferative diabetic retinopathy (PDR). In addition, when accompanied by diabetic macular edema (DME), severe visual impairment may be seen even at this stage.

If diabetic retinopathy is detected early, proper management can prevent the progression and worsening of retinopathy, but if the condition is not properly managed, it can lead to severe vision loss or blindness. Currently, diabetic retinopathy is considered a major cause of blindness in adults, and despite its clinical significance, drugs for preventing or treating diabetic retinopathy is not being actively developed.

Therefore, the need for drug development for preventing or treating diabetic retinopathy has been raised.

Sjogren's syndrome, an example of angiogenesis-related disease or matrix metalloproteinase (MMP)-mediated disease, is a chronic disease that causes secretion disturbance due to the invasion of lymphocytes into exocrine glands. The characteristic symptoms of this disease are dry eye and dry mouth caused by lymphocytic infiltrates in the lacrimal and salivary glands. With the loss of tears and saliva, characteristic changes in the eye (called aqueous lacrimal deficiency or keratoconjunctivitis sicca) and characteristic changes in the oral cavity (this results in damage to teeth, increase in oral infections, difficulty of swallowing, and mouth sores) may cause pain. The patient may also have joint inflammation (arthritis), muscle inflammation (myositis), nerve inflammation (neuropathy), thyroid inflammation (thyroiditis), kidney inflammation (nephritis), lung inflammation, or inflammation of other parts of the body, or lymph nodes swelling. Also, patients may experience fatigue and sleep disturbances. Sjogren's syndrome mainly affects middle-aged women.

Currently, there is no known treatment for Sjogren's syndrome or an effective treatment to restore glandular secretion. Conventional treatment is usually symptomatic and supportive which includes rehydration therapy (e.g. to relieve symptoms of dry eyes and mouth) and various forms of lubrication. Prescription medications are available, including cyclosporine that helps treat chronic dry eye, and cevimeline or pilocarpine that helps stimulate flow of saliva. Anti-inflammatory agents such as methotrexate and hydroxychloroquine have also been prescribed for amelioration of musculoskeletal symptoms. However, none of the currently available drugs are ideal due to the wide range of serious side effects.

Glaucoma, an example of angiogenesis-related disease or matrix metalloproteinase (MMP)-mediated disease, is a disease in which the optic nerve-responsible for transmitting visual information to the brain is damaged, that makes the optic nerve atrophied and field of view is narrow. As types of glaucoma, primary open-angle glaucoma (POAG), normal tension glaucoma, primary angle-closure glaucoma, developmental glaucoma, secondary glaucoma are known. In addition, although visual field is normal, ocular hypertension, a condition in which intraocular pressure is chronically high, is one of the risk factors for glaucoma.

In the treatment of glaucoma, lowering the intraocular pressure and preventing further visual field disturbances are considered the top priority, and drug treatment, laser treatment, and surgery are performed to lower the intraocular pressure, but these conventional treatments are not satisfactory to all patients. There is a demand for a glaucoma treatment drug containing an active ingredient with a new mechanism of action or a new structure that is not found in existing therapeutic drugs.

[Prior **Art References**]

**[Patent Documents]**

    (Patent Document 1) KP No. 10-1055920
    (Patent Document 2) KP No. 10-1292931

KR 2015 0035245 A relates to a method for preparing a tablet comprising a melissa officinalis leaf extract.
WO 2014/014271 A1 discloses tablet comprising melissa officinalis folium extract for preventing or treating angiogenesis or matrix metalloproteinase (MMP) activity-mediated disease.

**[Disclosure of Invention]**

**[Technical Problem]**

An object of the present invention is to provide a fractional extract of melissa leaf comprising caffeic acid, EDPA (Ethyl 2-(3,4-dihydroxyphenyl) acetate), RME (Rosmarinic acid methyl ester) and rosmarinic acid.
Another object of the present invention is to provide a pharmaceutical composition for use in preventing or treating non-alcoholic steatohepatitis comprising a fractional extract of melissa leaf as effective component.
Another object of the present invention is to provide a pharmaceutical composition for use in preventing or treating non-alcoholic fatty liver disease
comprising a fractional extract of melissa leaf as effective component.
An object of the present invention is to provide a pharmaceutical composition for use in the prevention or treatment of angiogenesis-related diseases or MMP (Matrix metalloproteinase)-mediated diseases comprising a fractional extract of melissa leaf as effective component containing caffeic acid, EDPA, RME and rosmarinic acid.

**[Technical Solution]**

The present inventors have recognized the need for research on the prevention of treatment of non-alcoholic steatohepatitis and non-alcoholic fatty liver, and angiogenesis-related diseases/MMP-mediated diseases e.g. obesity, age-related macular degeneration, psoriasis, endometriosis, cancer growth or cancer metastasis, arteriosclerosis, arthritis, inflammatory bowel disease, Alzheimer's disease, periodontal disease, diabetic retinopathy, Sjogren's syndrome, and glaucoma. The inventors tried to achieve the purpose of research on natural substances that can prevent or treat these diseases.
Specifically, when a novel fractional extract of melissa leaf is included as an active ingredient, the inventors of the present invention discovered that it has a preventive and therapeutic effect on non-alcoholic steatohepatitis, non-alcoholic fatty liver, and angiogenesis-related diseases/MMP-mediated diseases such as obesity, age-related macular degeneration, psoriasis, endometriosis, cancer growth or cancer metastasis, arteriosclerosis, arthritis, inflammatory bowel disease, Alzheimer's disease, periodontal disease, diabetic retinopathy, Sjogren's syndrome, and glaucoma, and have completed the present invention.

Hereinafter, the present invention will be described in detail.

## Fractional extract of Melissa leaf

The present invention provides a fractional extract of melissa leaf comprising caffeic acid, EDPA, RME and rosmarinic acid.

According to the present invention, a fractional extract of melissa leaf comprises 0.1 to 5 % by weight of caffeic acid, 0.05 to 6% by weight of EDPA, 0.01 to 2% by weight of RME, and 5 to 50% by weight of rosmarinic acid, based on the total fractional extract of melissa leaf. Specifically, (1) 0.2 to 4.0 weight % of caffeic acid, 0.1 to 5.0 weight % of EDPA, 0.02 to 1.5 weight % of RME, and 6 to 40 weight % of rosmarinic acid, (2) 0.3 to 3.0 weight % of caffeic acid, 0.15 to 4.0 weight % of EDPA, 0.03 to 1.0 weight % of RME, and 7 to 35 weight % of rosmarinic acid, (3) 0.4 to 2.0 weight % of caffeic acid, 0.2 to 3.0 weight % of EDPA , 0.04 to 0.7 weight % of RME and 8 to 30 weight % of rosmarinic acid, but is not limited thereto. Hereinafter, in this embodiment, a fractional extract of melissa leaf of the present invention may comprise caffeic acid, EDPA, RME, and rosmarinic acid in the above-described weight % unless otherwise specified.

According to the present invention, the fractional extract of melissa leaf contains 0.05 to 6% by weight of EDPA (Ethyl 2-(3,4-dihydroxyphenyl) acetate), specifically (1) 0.1 to 5% by weight of EDPA %, (2) 0.15 to 4% by weight of EDPA, and (3) 0.2 to 3% by weight of EDPA, but is not limited thereto.

In an embodiment of the present invention, the pharmaceutical composition with the highest content of EDPA contained in a fractional extract of melissa leaf is ALS-T20003 (0.85% by weight), and it has been confirmed that ALS-T20003 has effects of preventing or treating non-alcoholic steatohepatitis and non-alcoholic fatty liver disease through animal experiments.

The fractional extract of melissa leaf of the present invention is effective for the prevention and treatment of non-alcoholic steatohepatitis and non-alcoholic fatty liver disease, and has excellent pharmacological effects with low side effects.

The fractional extract of melissa leaf of the present invention does not contain rutin.

In an embodiment of the present invention, the fractional extract of melissa leaf may be obtained by extracting the melissa leaf with alcohol, then concentrating the alcohol extract, suspending the concentrate in water, and then drying the fraction obtained by fractionation with ethyl acetate.

Specifically, in an embodiment of the present invention, the fractional extract of melissa leaf may be obtained by an extraction process comprising extracting and concentrating the melissa leaf with 50% to 100% alcohol, suspending in water, and fractionating with ethyl acetate, but is not limited thereto.

In an embodiment of the present invention, the fraction may be dried using a hot air drying, a freeze drying, or a spray drying method. For example, the fraction may be dried by hot air using a hot air dryer, freeze-dried by reducing air pressure after freezing the fraction, or spray-dried by spraying the fraction with hot air.

In the preparation of the fractional extract of melissa leaf of the present invention, dried or undried melissa leaves or a mixture thereof may be used. For effective extraction, melissa leaves may be used after crushing.

In an embodiment of the present invention, the alcohol may be 70 to 80% (v/v) alcohol.

In the present invention, the term "alcohol" refers to a compound in which a hydroxyl group is bonded to a carbon atom of an alkyl or substituted alkyl group, and the term "alkyl" refers to a linear saturated hydrocarbon group or branched saturated hydrocarbon group. The term "substituted alkyl group" means that a substituent bonded to the carbon of the alkyl group is hydroxy, cyano, or halides, etc.

The alcohol refers to a C1-C6 alcohol including 70 to 80% (v/v) ethanol, methanol, etc., and preferably may be ethanol or methanol.

In the present invention, the term "C1-C6" means a functional group or main chain having 1 or more and 6 or less carbon atoms.

In an embodiment of the present invention, the alcohol extract may be obtained by the following steps, but is not limited thereto.

(S1) the first extraction step to obtain the first extract by reflux extraction of melissa leaves with 50-100% (v/v) alcohol at 80 to 85°C for 2 to 6 hours;
(S2) the second extraction step to obtain the second extract by reflux extraction of the melissa residue remaining after the first extraction with 50-100% (v/v) alcohol at 80 to 85°C for 2 to 6 hours; and
(S3) the step of mixing the first extract and the second extract.

The alcohol extract can be obtained by mixing the first and second extracts obtained through the above the first and the second extraction steps.

The alcohol extract may be prepared using 50-100% alcohol (v/v) of 5 to 15 times (v/w) of Melissa leaves, preferably 70-80% alcohol (v/v), but is not limited thereto.

According to the embodiment of the present invention, the concentrate may be obtained by the following steps, but is not limited thereto.

(S4) the first concentration step to obtain the first concentrate by concentrating the alcohol extract for 5 to 15 hours under a temperature condition of 50 to 60 °C, and a pressure condition of -0.066 to -0.070 MPa, and
(S5) the second concentration step to obtain the second concentrate by concentrating the first concentrate for 3 to 10 hours under a temperature condition of 55 to 60°C and a pressure condition of -0.063 to -0.065 MPa.

According to the embodiments of the present invention, before drying the fraction, the following concentration step may be further included, but is not limited thereto.

(S6) the third concentration step of concentrating the fraction.

The third concentration step of concentrating the fraction for 3 to 10 hours under a temperature condition of 55 to 60 °C, and a pressure condition of -0.063 to - 0.065 MPa.

In the present invention, the term "fraction" refers to a resultant product obtained by performing the fractionation to separate specific components or a specific component group from a mixture containing various constituents.

In the present invention, the fractionation method for obtaining the fractional extract of melissa leaf is not particularly limited, and may be performed according to a method commonly used in the art. Specifically, solvent fractionation performed by treating various solvents, ultrafiltration fractionation performed by passing through an ultrafiltration membrane having a constant molecular weight cut-off value, chromatographic fractionation by various chromatography (prepared for separation according to size, electric charge, hydrophobicity or affinity) and any combination thereof. Specifically, there may be a method to obtain a fraction from the extract by treating the extract obtained by extracting the melissa leaf of the present invention with a certain solvent.

In the present invention, the type of the fractionation solvent used to obtain the fraction is not particularly limited, and any solvent known in the art may be used, and specifically, ethyl acetate which is a non-polar solvent may be used.

The fractional extract of melissa leaf may be an ethyl acetate fraction of the ethanol extract of melissa leaf.

The method effectively extracts soluble substances and insoluble substances by using 50-100% (v/v) alcohol, and has the effect of extracting insoluble substances with low solubility in water but high solubility in ethyl acetate.

The ethyl acetate fraction of the melissa leaf ethanol extract may comprise 0.1 to 5 weight % of caffeic acid, 0.05 to 6 weight % of EDPA, 0.01 to 2 weight % of RME, and 5 to 50 weight % of rosmarinic acid based on the total weight of the fraction. Specifically (1) 0.2 to 4.0 weight % of caffeic acid, 0.1 to 5.0 weight % of EDPA, 0.02 to 1.5 weight % of RME and 6 to 40 weight % of rosmarinic acid, (2) 0.3 to 3.0 weight % of caffeic acid, 0.15-4.0 weight % of EDPA, 0.03-1.0 weight % of RME, and 7-35 weight % of rosmarinic acid, (3) 0.4-2.0 weight % of caffeic acid, 0.2-3.0 weight % of EDPA, 0.04-0.7 weight % of RME and 8 to 30 weight % of rosmarinic acid.

In the embodiment of the invention, the ethyl acetate fraction of the melissa leaf ethanol extract may comprise 0.05 to 6 weight % of EDPA (Ethyl 2-(3,4-dihydroxyphenyl) acetate), specifically (1) 0.1 to 5 weight % of EDPA, (2) 0.15 to 4 weight % of EDPA, and (3) 0.2 to 3 weight % of EDPA, but is not limited thereto.

Advantages and features of the present invention, and methods for achieving them, will become apparent with reference to the embodiments described below in detail. However, the present invention is not limited to the embodiments disclosed below, but will be implemented in a variety of different forms, and only these embodiments allow the disclosure of the present invention to be complete, and provide to fully indicate the scope of the invention to those of ordinary skill in the art to which the present invention pertains, and the present invention is only defined by the scope of the claims.

**Pharmaceutical composition for use in the prevention or treatment of non-alcoholic steatohepatitis comprising a fractional extract of melissa leaf as effective component**

It is an object of the present invention to provide a pharmaceutical composition for use in the prevention or treatment of non-alcoholic steatohepatitis comprising a fractional extract of melissa leaf as effective component.

The fractional extract of melissa leaf and its preparation method are the same as previously examined.

According to the present invention, the term "non-alcoholic steatohepatitis" refers to a disease that exhibits a form similar to alcoholic liver disease despite no drinking history.

According to the present invention, the term "effective component" includes substances or groups of substances expected to directly or indirectly express the efficacy of the composition by its intrinsic pharmacological action (e.g. herbal medicines for which pharmacologically active ingredients, etc. are not identified) as a means to include the main component.

According to the present invention the pharmaceutical composition for use in the prevention or treatment of non-alcoholic steatohepatitis comprising a fractional extract of melissa leaf as effective component, has an effect of preventing or treating non-alcoholic steatohepatitis by exhibiting significant inhibitory effect on fibrosis and inhibitory effect on protein expression levels of Col1A2, TGF beta, IL-6 and IL-10.

The pharmaceutical composition of the present invention for use in the prevention or treatment of non-alcoholic steatohepatitis comprising a fractional extract of melissa leaf as effective component comprises a fractional extract of melissa leaf as effective component, and may further comprise a pharmaceutically acceptable carrier. According to a conventional method, it may be formulated in oral dosage forms of powders, granules, tablets, capsules, suspensions, emulsions, syrups, aerosols, etc., external preparations, and sterile injection solutions.

According to the present invention, the term "pharmaceutically acceptable" refers to a composition that is physiologically acceptable and does not normally cause allergic reactions such as gastrointestinal disorders, dizziness, or similar reactions when administered to humans.

According to the present invention, the term "pharmaceutically acceptable carrier" typically includes a liquid or non-liquid basis of a pharmaceutical composition. If a pharmaceutical composition is provided in liquid form, the carrier comprises typically water without pyrogen; isotonic saline or buffered (aqueous) solutions, for example, phosphate, citric acid, etc. The injection buffer may be hypertonic, isotonic or hypotonic in a particular reference medium, i.e. the buffer may have a high, equal or low salt content in the particular reference medium, preferably such concentrations of the aforementioned salts, which do not induce cell damage by osmotic pressure or other concentration effect, may be used.

The reference medium occurs in an "in vivo" method, for example, such as blood, lymph, cytoplasmic liquid, or other bodily fluid, or a bodily fluid that can be used as a reference medium in an "ex vivo" method, for example as a general buffer or liquid. Such general buffers or liquids are known to the person skilled in the art.

The pharmaceutically acceptable carriers include those commonly used in the art for example, lactose, dextrose, sucrose, sorbitol, mannitol, xylitol, erythritol, maltitol, starch, gum acacia, alginate, gelatin, calcium phosphate, calcium silicate, cellulose, methyl cellulose, microcrystalline cellulose, polyvinyl pyrrolidone, water, methylhydroxybenzoate, propylhydroxybenzoate, talc, magnesium stearate and mineral oil or the like, but not limited to thereto.

In addition, the pharmaceutical composition of the present invention may comprise a diluent or excipient such as a filler, an extender, a binder, a humectant, a disintegrant, a surfactant, and other pharmaceutically acceptable additives.

The pharmaceutical composition of the present invention may be manufactured in the form of liquid, suspension, powder, granule, tablet, capsule, pill or extract.

The composition of the present invention may be administered orally or parenterally (e.g. liniments or intravenous, subcutaneous, intraperitoneal injection).

According to the present invention, the term "oral administration" is a method of injecting a drug by mouth for alleviating pathological symptoms, and according to the present invention, the term "parenteral administration" refers to a method of subcutaneous, intramuscular, intravenous, or intraperitoneal administration using a tube, except for administration by mouth.

Solid dosage forms for oral administration may include powder, granule, tablet, capsule, soft capsule, pill, and the like. Liquid dosage forms for oral administration may include suspension, liquid for internal use, emulsion, syrup, aerosol, and the like, and may include various additives, for example, humectant, sweetening agent, flavoring agent, preservative and the like in addition to water and liquid paraffin, which are frequently used simple diluents.

For dosage forms for parenteral administration, external preparations such as sterilized aqueous solution, liquid preparation, non-aqueous solvent, suspending agent, emulsion, eye drop, eye ointment, syrup, suppository, aerosol, etc., and sterile injection preparation can be formulated and used according to the usual method, preferably cream, gel, patch, spray, ointment, plaster, lotion, liniment, eye ointment, eye drop, pasta, or cataplasma pharmaceutical composition can be formulated, but not limited thereto. Compositions for topical administration may be anhydrous or aqueous, depending on the clinical prescription. Propylene glycol, polyethylene glycol, vegetable oils such as olive oil, and injectable esters such as ethyl oleate may be used as non-aqueous solvent and suspending agent. As a base of suppository, witepsol, macrogol, tween 61, cacao butter, laurin, glycerogelatin, and the like may be used.

The pharmaceutically acceptable additive according to the present invention may be included in 0.1 to 99.9 parts by weight, specifically 0.1 to 50 parts by weight, based on the composition, but is not limited thereto.

The pharmaceutical composition for use in preventing or treating non-alcoholic steatohepatitis comprising a fractional extract of melissa leaf as effective component has a fractional extract of melissa leaf in an amount of 20 weight % to 80 weight % based on the total weight.

According to the present invention the pharmaceutical composition for use in the prevention or treatment of non-alcoholic steatohepatitis comprising a fractional extract of melissa leaf as effective component, has an effect of preventing or treating non-alcoholic steatohepatitis by exhibiting significant inhibitory effect on fibrosis and on protein expression levels of ColiA2, TGF beta, IL-6 and IL-10.

The pharmaceutical composition for use in preventing or treating non-alcoholic steatohepatitis comprising the fractional extract of melissa leaf as effective component of the present invention may be administered in a conventional manner via oral, rectal, intravenous, intra-arterial, intraperitoneal, intramuscular, intra-sternal, transdermal, topical, intraocular, or intradermal routes, specifically oral route.

The pharmaceutical composition for use in preventing or treating non-alcoholic steatohepatitis comprising a fractional extract of melissa leaf as effective component of the present invention is preferably administered at a dose of 0.001 mg/kg to 50 mg/kg when administered once to several times a day.

## Pharmaceutical composition for use in preventing or treating non-alcoholic fatty liver disease comprising a fractional extract of melissa leaf as effective component

Another object of the present invention is to provide a pharmaceutical composition for use in preventing or treating non-alcoholic fatty liver disease comprising a fractional extract of melissa leaf as effective component.

The fractional extract of melissa leaf and its preparation method are the same as previously examined.

According to the present invention, the term "non-alcoholic fatty liver disease " refers to a disease that exhibits a form similar to alcoholic fatty liver disease despite no drinking history.

According to the present invention a pharmaceutical composition for use in the prevention or treatment of non-alcoholic fatty liver disease comprising a fractional extract of melissa leaf as effective component, is effective in preventing or treating non-alcoholic fatty liver disease by exhibiting significant suppression of weight, blood triglycerides, blood sugar and total cholesterol.

The pharmaceutical composition for use in the prevention or treatment of non-alcoholic fatty liver disease comprising the fractional extract of melissa leaf as effective component has a fractional extract of melissa leaf in an amount of 20 weight % to 80 weight % based on the total weight.

The pharmaceutical composition for use in preventing or treating non-alcoholic fatty liver disease comprising the fractional extract of melissa leaf as effective component of the present invention may be administered in a conventional manner via oral, rectal, intravenous, intra-arterial, intraperitoneal, intramuscular, intra-sternal, transdermal, topical, intraocular, or intradermal routes, specifically oral route.

The pharmaceutical composition for use in the prevention or treatment of non-alcoholic fatty liver disease comprising the fractional extract of melissa leaf as effective component of the present invention is preferably administered at a dose of 0.001 mg/kg to 50 mg/kg when administered once to several times a day.

The matters mentioned in the pharmaceutical composition for use in the prevention or treatment of non-alcoholic steatohepatitis comprising the fractional extract of melissa leaf as effective component are equally applicable to the pharmaceutical composition for the prevention or treatment of non-alcoholic fatty liver disease comprising the fractional extract of melissa leaf as effective component as long as they do not contradict each other.

## Food composition comprising a fractional extract of melissa leaf as effective component

The present invention can be used to provide a food composition comprising a fractional extract of melissa leaf as effective component.

The fractional extract of melissa leaf and the preparation method thereof are the same as described above.

The composition can be used for preventing or ameliorating any one of non-alcoholic steatohepatitis and non-alcoholic fatty liver disease.

In the food composition, when the fractional extract of melissa leaf is used as an additive in the food composition, it can be added as it is or used with other foods or food ingredients, and can be used appropriately according to a conventional method. The mixing amount of the effective component may be appropriately determined according to each purpose of use, such as prevention, health or treatment.

The formulation of the food composition may be possible in any form of general food or beverage as well as powder, granule, pill, tablet, and capsule.

There is no particular limitation on the type of the food, and examples of the food to which the substance can be added include meat, sausage, bread, chocolate, candy, snacks, confectionery, pizza, ramen, other noodles, gums, and dairy products including ice cream, various soups, beverages, teas, drinks, alcoholic beverages, and vitamin complex, and may include all foods in a conventional sense.

In general, in the manufacture of food or beverage, the fractional extract of melissa leaf may be added in an amount of 15 parts by weight or less, preferably 10 parts by weight or less, based on 100 parts by weight of the raw material. However, in the case of long-term intake for health and hygiene or health control, the amount may be less than the above range. In addition, since there is no problem in terms of safety using a fractional extract from a natural product in the present invention, it can also be used in an amount above the range.

Beverages in the food composition may comprise various flavoring agents or natural carbohydrates as additional ingredients like conventional beverages. The natural carbohydrates may be monosaccharides such as glucose and fructose, disaccharides such as maltose and sucrose, polysaccharides such as dextrin and cyclodextrin, and sugar alcohols such as xylitol, sorbitol, and erythritol. As the sweetener, natural sweeteners such as thaumatin and stevia extract

or synthetic sweeteners such as saccharin and aspartame, and the like may be used. The ratio of the natural carbohydrate may be about 0.01 to 0.04 g, preferably about 0.02 to 0.03 g per 100 mL of the beverage according to the present invention.

In addition to the above, the food composition mayinclude various nutrients, vitamins, electrolytes, flavoring agents, coloring agents, pectic acid and its salts, alginic acid and its salts, organic acids, protective colloidal thickeners, pH adjusters, stabilizers, preservatives, glycerin, alcohol, and carbonating agents used in carbonated beverages. In addition, the food composition comprising the fractional extract of melissa leaf of the present invention as effective component may comprise natural fruit juice, and fruit pulp for making fruit juice and vegetable beverage. These components may be used independently or in combination. The ratio of these additives is not limited, but is generally selected in the range of 0.01 to 0.1 parts by weight relative to 100 parts by weight of the food composition

## Method for prevention or treatment

The present invention can be used in a method for preventing or treating non-alcoholic steatohepatitis by administering a therapeutically effective amount of a fractional extract of melissa leaf to a subject in need of treatment.

The present invention can be used in a method for preventing or treating non-alcoholic fatty liver disease by administering a therapeutically effective amount of a fractional extract of melissa leaf to a subject in need of treatment.

According to the embodiments of the present invention, the term "subject in need of treatment" refers to mammals including humans, and the term "administration" refers to providing a predetermined substance to a patient by any suitable method. In the present invention, the pharmaceutical composition comprising a fractional extract of melissa leaf as effective component of the present invention may be administered in a conventional manner by oral, rectal, intravenous, intra-arterial, intraperitoneal, intramuscular, intra-sternal, transdermal, topical, intraocular or intradermal routes.

In addition, in the method for preventing or treating non-alcoholic steatohepatitis, the fractional extract of melissa leaf of the present invention may be administered in a conventional manner by oral, rectal, intravenous, intra-arterial, intraperitoneal, intramuscular, intra-sternal, transdermal, topical, intraocular or intradermal routes, and specifically it can be administered orally.

Also, in the method for preventing or treating non-alcoholic fatty liver disease, the fractional extract of melissa leaf of the present invention may be administered in a conventional manner by oral, rectal, intravenous, intra-arterial, intraperitoneal, intramuscular, intra-sternal, transdermal, topical, intraocular or intradermal routes, and specifically, it can be administered orally.

According to the embodiments of the present invention, the term "therapeutically effective amount" refers to the amount of an effective component or a pharmaceutical composition that induces a biological or medical response in a tissue system, animal or human that is considered by researchers, veterinarians, medical doctors or other clinicians, which includes an amount that induces amelioration of the symptoms of the disease or disorder being treated. It is apparent to those skilled in the art that the therapeutically effective dosage and frequency of administration for the effective component of the present invention will vary depending on the desired effect. Therefore, the optimal dosage of administration can be easily determined by those skilled in the art, and can be adjusted by various factors including the type of disease, the severity of the disease, the content of effective components and other ingredients comprised in the composition, the type of formulation, and patient's age, weight, general health, sex and diet, administration time, administration route and secretion rate of the composition, treatment period, and concomitant drugs.

In the method for preventing or treating non-alcoholic steatohepatitis, the fractional extract of melissa leaf of the present invention is preferably to be administered to adults at a dose of 0.001 mg/kg to 50 mg/kg when administered once to several times a day.

In the method for preventing or treating non-alcoholic fatty liver disease, the fractional extract of melissa leaf of the present invention is preferably to be administered to adults at a dose of 0.001 mg/kg to 50 mg/kg when administered once to several times a day.

According to the present invention, the term "prevention" refers to any action of suppressing or delaying non-alcoholic steatohepatitis and non-alcoholic fatty liver disease by administration of the fractional extract of melissa leaf according to the present invention.

According to the present invention, the term "treatment" refers to any action for improving or beneficially changing non-alcoholic steatohepatitis and non-alcoholic fatty liver disease by administration of the fractional extract of melissa leaf according to the present invention.

## A pharmaceutical composition comprising a fractional extract of melissa leaf as effective component for use in prevention or treatment

A pharmaceutical composition comprising a fractional extract of melissa leaf as effective component can be used for the prevention or treatment of non-alcoholic steatohepatitis.

A pharmaceutical composition comprising a fractional extract of melissa leaf as effective component can be used for the prevention or treatment of non-alcoholic fatty liver disease.

**Use of a pharmaceutical composition comprising a fractional extract of melissa leaf as effective component for the manufacture of a medicament**

A pharmaceutical composition comprising a fractional extract of melissa leaf as effective component can be used for the manufacture of a medicament for preventing or treating non-alcoholic steatohepatitis.

A pharmaceutical composition comprising a fractional extract of melissa leaf as effective component can be used for the manufacture of a medicament for preventing or treating non-alcoholic fatty liver disease.

The pharmaceutical composition comprising the fractional extract of melissa leaf as effective component for the manufacture of a medicament may be admixed with an acceptable carrier or the like, and may further comprise other agents.

**[Angiogenesis-related disease or MMP (Matrix metalloproteinase)-mediated disease]**

**Pharmaceutical composition for the prevention or treatment of angiogenesis-related diseases or MMP (Matrix metalloproteinase)-mediated diseases**

The present invention is to provide a pharmaceutical composition for use in preventing or treating angiogenesis-related diseases or MMP (Matrix metalloproteinase)-mediated diseases, comprising a fractional extract of melissa leaf containing caffeic acid, EDPA, RME and rosmarinic acid as active ingredients.

According to the present invention, the term "angiogenesis-related disease" refers to a disease occurring in association with the pathologically excessive formation of new capillaries from existing microvessels.

According to the present invention, the term "MMP-mediated disease" refers to a disease caused by excessive activation of matrix metalloprotease (MMP).

According to the present invention, the term "effective component" refers to a substance or group of substances (including herbal medicines and the like for which pharmacologically active ingredients are not identified) expected to directly or indirectly express the efficacy of the composition by its intrinsic pharmacological action, which means to include the main component.

According to the present invention, a fractional extract of melissa leaf may comprise 0.1 to 5% by weight of caffeic acid, 0.05 to 6% by weight of EDPA, 0.01 to 2% by weight of RME, and 5 to 50% by weight of rosmarinic acid, based on the total fractional extract of melissa leaf. Specifically it may comprise (1) caffeic acid 0.2 to 4.0 weight %, EDPA 0.1 to 5.0 weight %, RME 0.02 to 1.5 weight %, and rosmarinic acid 6 to 40 weight %, (2) caffeic acid 0.3 to 3.0 weight %, EDPA 0.15 to 4.0 weight %, RME 0.03 to 1.0 weight %, and rosmarinic acid 7 to 35 weight %, (3) caffeic acid 0.4 to 2.0 weight %, EDPA 0.2 to 3.0 weight %, RME 0.04 to 0.7 weight % and rosmarinic acid 8 to 30 weight %, but is not limited thereto. Hereinafter, in the exemplary embodiments, the fractional extract of melissa leaf of the present invention may comprise caffeic acid, EDPA, RME, and rosmarinic acid in the above-described weight%, unless otherwise specified.

According to the present invention, the fractional extract of melissa leaf may comprise 0.05 to 6% by weight of EDPA (Ethyl 2-(3,4-dihydroxyphenyl) acetate), specifically (1) 0.1 to 5% by weight of EDPA, (2) 0.15 to 4% by weight of EDPA, and (3) 0.2 to 3% by weight of EDPA, but is not limited thereto.

According to the embodiment of the present invention, the pharmaceutical composition with the highest content of EDPA comprised in the fractional extract of melissa leaf is ALS-T20003 (0.85 weight %), and it can be confirmed that ALS-T20003 used in animal experiments inhibits angiogenesis and has an effect of preventing or treating angiogenesis and MMP-mediated diseases.

The pharmaceutical composition of the present invention comprising the fractional extract of melissa leaf as effective component is effective in preventing and treating angiogenesis-related diseases or matrix metalloproteinase (MMP) mediated diseases, such as obesity, age-related macular degeneration, psoriasis, endometriosis, cancer growth or cancer metastasis, atherosclerosis, arthritis, inflammatory bowel disease, Alzheimer's disease, periodontal disease, diabetic retinopathy, Sjogren's syndrome, and glaucoma, and has excellent pharmacological effects with few side effects.

The fractional extract of melissa leaf, which is effective component comprised in the pharmaceutical composition of the present invention, does not contain rutin.

According to the embodiment of the present invention, the fractional extract of melissa leaf may be obtained by extracting the melissa leaf under reflux with alcohol, then concentrating the alcohol extract, suspending the concentrate in water, and then fractionating with ethyl acetate and drying the fraction.

Specifically, according to the embodiment of the present invention, the fractional extract of melissa leaf may be obtained by an extraction process which includes extracting melissa leaf with 50% to 100% alcohol, concentrating, suspending in water, and fractionating with ethyl acetate, but is not limited thereto.

According to the embodiment of the present invention, the fraction may be dried using a hot air drying, a freeze drying, or a spray drying method. For example, the fraction may be hot-air dried using a hot-air dryer, or freeze-dried by freezing the fraction and lowering the air pressure, or spray-dried by spraying the fraction and blowing hot air.

In the preparation of the fractional extract of melissa leaf of the present invention, dried or undried melissa leaves or a mixture thereof may be used. For effective extraction, melissa leaves can be finely crushed and used.

According to the embodiment of the present invention, the alcohol may be 70 to 80% (v/v) alcohol, but is not limited thereto.

According to the present invention, the term "alcohol" refers to a compound in which a hydroxyl group is bonded to a carbon atom of an alkyl or substituted alkyl group, and the term "alkyl" refers to a linear saturated hydrocarbon group or branched saturated hydrocarbon group, and the term "substituted alkyl group" means that a substituent bonded to the carbon of the alkyl group is hydroxy, cyano, halides, etc.

The alcohol refers to a C1-C6 alcohol including 70 to 80% (v/v) ethanol, methanol, etc., preferably ethanol or methanol.

According to the present invention, the term "C1-C6" means a functional group or main chain having 1 to 6 carbon atoms.

According to the embodiment of the present invention, the alcohol extract may be obtained by the following steps, but is not limited thereto.

(S1) the first extraction step of obtaining the first extract by reflux extraction of melissa leaves with 50-100% (v/v) alcohol at 80 to 85°C for 2 to 6 hours;

(S2) the second extraction step of obtaining the second extract by reflux extraction of the melissa residue remaining after the first extraction with 50-100% (v/v) alcohol at 80 to 85°C for 2 to 6 hours; and

(S3) the step of mixing the first extract and the second extract.

The alcohol extract can be obtained by mixing the first and second extracts obtained through the above first and second extraction steps.

The alcohol extract may be prepared using 50-100% alcohol (v/v) of 5 to 15 times (v/w) of Melissa leaves, preferably 70-80% alcohol (v/v), but is not limited thereto.

According to the embodiment of the present invention, the concentrate may be obtained by the following steps, but is not limited thereto.

(S4) the first concentration step to obtain the first concentrate by concentrating the alcohol extract for 5 to 15 hours under a temperature condition of 50 to 60 °C, and a pressure condition of -0.066 to -0.070 MPa, and

(S5) the second concentration step to obtain the second concentrate by concentrating the first concentrate for 3 to 10 hours under a temperature condition of 55 to 60 °C and a pressure condition of -0.063 to -0.065 MPa.

According to the embodiment of the present invention, before drying the fraction, the following concentration step may be further included, but is not limited thereto.

(S6) the third concentration step of concentrating the fraction.

The third concentration step of concentrating the fraction for 3 to 10 hours under a temperature condition of 55 to 60 °C, and a pressure condition of -0.063 to - 0.065 MPa.

According to the present invention, the term "fraction" refers to a result obtained by performing fractionation in order to separate a specific component or a specific component group from a mixture containing various constituents.

According to the present invention, the fractionation method for obtaining the fractional extract of melissa leaf is not particularly limited, and may be performed according to a method commonly used in the art. Specifically, solvent fractionation performed by treating various solvents, ultrafiltration fractionation performed by passing through an ultrafiltration membrane having a constant molecular weight cut-off value, chromatographic fractionation performed by various chromatography (prepared for separation according to size, charge, hydrophobicity or affinity), and combinations of these thereof. Specifically, there may be a method of obtaining a fraction from the extract by treating the extract obtained by extracting the melissa leaf of the present invention with a predetermined solvent.

According to the present invention, the type of the fractionation solvent used to obtain the fraction is not particularly limited, and any solvent known in the art may be used, and specifically ethyl acetate which is a non-polar solvent may be used.

The fractional extract of melissa leaf may be an ethyl acetate fraction of the ethanol extract of melissa leaf.

The method effectively extracts soluble substances and insoluble substances by using 50-100% (v/v) alcohol, and has the effect of extracting insoluble substances with low solubility in water but high solubility in ethyl acetate.

The ethyl acetate fraction of the melissa leaf ethanol extract may comprise 0.1 to 5 weight % of caffeic acid, 0.05 to 6 weight % of EDPA, 0.01 to 2 weight % of RME, and 5 to 50 weight % of rosmarinic acid based on the total weight of the fraction. Specifically (1) 0.2 to 4.0 weight % of caffeic acid, 0.1 to 5.0 weight % of EDPA, 0.02 to 1.5 weight % of RME and 6 to 40 weight % of rosmarinic acid, (2) 0.3 to 3.0 weight % of caffeic acid, 0.15-4.0 weight % of EDPA, 0.03-1.0 weight % of RME, and 7-35 weight % of rosmarinic acid, (3) 0.4-2.0 weight % of caffeic acid, 0.2-3.0 weight % of EDPA, 0.04-0.7 weight % of RME and 8 to 30 weight % of rosmarinic acid.

The pharmaceutical composition comprising the fractional extract of melissa leaf as effective component for use in the prevention or treatment of angiogenesis-related diseases or MMP (Matrix metalloproteinase)-mediated diseases of the present invention comprises a fractional extract of melissa leaf as effective component, and may further comprise a pharmaceutically acceptable carrier to be formulated in oral dosage form such as powder, granule, tablet, capsule, suspension, emulsion, syrup, aerosol, etc., and for external use, and sterile injection solution according to a conventional method.

The contents of the formulation can be equally applied to the pharmaceutical composition for use in the prevention or treatment of angiogenesis-related diseases or MMP (Matrix metalloproteinase)-mediated diseases, e.g. obesity, age-related macular degeneration, psoriasis, endometriosis, cancer growth or cancer metastasis, arteriosclerosis, arthritis, inflammatory bowel disease, Alzheimer's disease, periodontal disease, diabetic retinopathy, Sjogren's syndrome and glaucoma.

According to the present invention, the term "pharmaceutically acceptable" refers to a composition that is physiologically acceptable and does not normally cause allergic reactions such as gastrointestinal disorders, dizziness, or similar reactions when administered to humans.

According to the present invention, the term "pharmaceutically acceptable carrier" typically includes a liquid or non-liquid basis of a pharmaceutical composition. If a pharmaceutical composition is provided in liquid form, the carrier typically includes pyrogen-free water; Isotonic saline or buffered (aqueous) solutions, for example phosphate, citric acid, etc. The injection buffer may be hypertonic, isotonic or hypotonic in a particular reference medium, i.e. the buffer may have a high, equal or low salt content in the particular reference medium, preferably such concentrations of the aforementioned salts, which do not induce cell damage by osmotic pressure or other concentration effect, may be used. The reference medium occurs in an "in vivo" method, for example, such as blood, lymph, cytoplasmic liquid, or other bodily fluid, or a bodily fluid that can be used as a reference medium in an "ex vivo" method, for example as a general buffer or liquid. Such general buffers or liquids are known to a person skilled in the art.

The pharmaceutically acceptable carriers include those commonly used in the art for example, lactose, dextrose, sucrose, sorbitol, mannitol, xylitol, erythritol, maltitol, starch, acacia gum, alginate, gelatin, calcium phosphate, calcium silicate, cellulose, methyl cellulose, microcrystalline cellulose, polyvinyl pyrrolidone, water, methylhydroxybenzoate, propylhydroxybenzoate, talc, magnesium stearate and mineral oil or the like, but is not limited thereto.

In addition, the pharmaceutical composition of the present invention may comprise a diluent or excipient such as a filler, an extender, a binder, a humectant, a disintegrant, a surfactant, and other pharmaceutically acceptable additives.

The pharmaceutical composition of the present invention may be manufactured in the form of liquid, suspension, powder, granule, tablet, capsule, pill or extract.

The composition of the present invention may be administered orally or parenterally (e.g. liniments or intravenous, subcutaneous, intraperitoneal injection). According to the present invention, the term "oral administration" is a method of injecting a drug by mouth for alleviating pathological symptoms, and according to the present invention, the term "parenteral administration" refers to a method of subcutaneous, intramuscular, intravenous, or intraperitoneal administration using a tube, except for administration by mouth.

Solid dosage form for oral administration may include powder, granule, tablet, capsule, soft capsules, pill, and the like. Liquid dosage form for oral administration may include suspension, liquid for internal use, emulsion, syrup, aerosol, and the like, and may include various additives, for example, humectant, sweetening agent, flavoring agent, preservative and the like in addition to water and liquid paraffin, which are frequently used simple diluents.

For dosage forms of parenteral administration, external preparations such as sterilized aqueous solution, liquid preparation, non-aqueous solvent, suspending agent, emulsion, eye drop, eye ointment, syrup, suppository, aerosol, etc., and sterile injection preparation can be formulated and used according to the conventional method, preferably cream, gel, patch, spray, ointment, plaster, lotion, liniment, eye ointment, eye drop, pasta or cataplasma pharmaceutical preparation can be formulated, but not limited thereto. Compositions for topical administration may be anhydrous or aqueous, depending on the clinical prescription. As non-aqueous solvent and suspending agent, propylene glycol, polyethylene glycol, vegetable oils such as olive oil, and injectable esters such as ethyl oleate may be used. As a base of suppository, witepsol, macrogol, tween 61, cacao butter, laurin, glycerogelatin, and the like may be used.

The pharmaceutically acceptable additive according to the present invention may be included in 0.1 to 99.9 parts by weight, specifically 0.1 to 50 parts by weight, based on the pharmaceutical composition, but is not limited thereto.

Advantages and features of the present invention, and methods for achieving them, will become apparent with reference to the embodiments described below in detail. However, the present invention is not limited to the exemplary embodiments disclosed below, but will be implemented in a variety of different forms. These exemplary embodiments just make the disclosure of the present invention complete, and are provided to inform the scope of the invention completely to those of ordinary skill in the art to which the present invention pertains, and the present invention is only defined by the scope of the claims.

## Angiogenesis-related disease or MMP-mediated disease: obesity

According to the embodiment of the present invention, the angiogenesis-related disease or MMP-mediated disease may be obesity.

According to the present invention, the term "obesity" refers to a state in which body weight is increased beyond the limits of skeletal and physical requirements due to excessive accumulation of fat in the body.

When the angiogenesis-related disease or MMP-mediated disease is obesity, a pharmaceutical composition for use in the prevention or treatment of angiogenesis-related disease or MMP (Matrix metalloproteinase)-mediated disease comprising the fractional extract of melissa leaf as effective component comprises a fractional extract of melissa leaf in an amount of 20 weight % to 80 weight % based on the total weight.

According to the present invention, a pharmaceutical composition comprising the fractional extract of melissa leaf as effective component for use in the prevention or treatment of angiogenesis-related diseases or MMP (Matrix metalloproteinase)-mediated diseases is effective in preventing or treating obesity by exhibiting significant suppression of weight, blood triglycerides, blood sugar, and total cholesterol.

When the angiogenesis-related disease or MMP-mediated disease is obesity, a pharmaceutical composition for use in the prevention or treatment of angiogenesis-related diseases or MMP (Matrix metalloproteinase)-mediated diseases comprising the fractional extract of melissa leaf as effective component can be administered in the conventional way by oral, rectal, intravenous, intra-arterial, intraperitoneal, intramuscular, intra-sternal, transdermal, topical, intraocular or intradermal routes, and specifically by oral route.

When the angiogenesis-related disease or MMP-mediated disease is obesity, a pharmaceutical composition for use in the prevention or treatment of angiogenesis-related disease or MMP (Matrix metalloproteinase)-mediated disease comprising the fractional extract of melissa leaf as effective component is preferably to be administered at a dose of 0.001 mg/kg to 50 mg/kg when administered once or several times a day.

The matters mentioned in the pharmaceutical composition for use in the prevention or treatment of angiogenesis-related diseases or matrix metalloproteinase (MMP)-mediated diseases are equally applied to obesity, which is an example of angiogenesis-related diseases or MMP-mediated diseases, unless they contradict each other.

## Angiogenesis-related disease or MMP-mediated disease: age-related macular degeneration

According to the embodiment of the present invention, the angiogenesis-related disease or MMP-mediated disease may be age-related macular degeneration.

According to the present invention, the term "macular degeneration" refers to a disease in which the macula deteriorates due to aging, genetic factors, toxicity, inflammation, etc. leading to complete loss of vision in severe cases.

According to the present invention, a pharmaceutical composition for use in the prevention or treatment of angiogenesis-related disease or MMP (Matrix metalloproteinase)-mediated disease comprising the fractional extract of melissa leaf as effective component is effective in preventing or treating age-related macular degeneration by suppressing the size of macular degeneration lesions.

When the angiogenesis-related disease or MMP-mediated disease is age-related macular degeneration, a pharmaceutical composition for use in the prevention or treatment of angiogenesis-related disease or MMP (Matrix metalloproteinase)-mediated disease comprising the fractional extract of melissa leaf as effective component comprises a fractional extract of melissa leaf in an amount of 20 weight % to 80 weight % based on the total weight.

When the angiogenesis-related disease or MMP-mediated disease is age-related macular degeneration, a pharmaceutical composition for use in the prevention or treatment of angiogenesis-related disease or MMP (Matrix metalloproteinase)-mediated disease comprising the fractional extract of melissa leaf as effective component can be administered in the conventional way by oral, rectal, intravenous, intra-arterial, intraperitoneal, intramuscular, intra-sternal, transdermal, topical, intraocular or intradermal routes, and specifically by oral route.

When the angiogenesis-related disease or MMP-mediated disease is age-related macular degeneration, a pharmaceutical composition for use in the prevention or treatment of angiogenesis-related disease or MMP (Matrix metalloproteinase)-mediated disease comprising the fractional extract of melissa leaf as effective component is preferably to be administered at a dose of 0.001 mg/kg to 50 mg/kg when administered when administered once or several times a day.

The matters mentioned in the pharmaceutical composition for use in the prevention or treatment of angiogenesis-related diseases or matrix metalloproteinase (MMP)-mediated diseases are equally applied to age-related macular degeneration, which is an example of angiogenesis-related disease or MMP-mediated disease, unless they contradict each other.

### Angiogenesis-related disease or MMP-mediated disease: psoriasis

According to the embodiment of the present invention, the angiogenesis-related disease or MMP-mediated disease may be psoriasis.

According to the present invention, the term "psoriasis" refers to a chronic inflammatory skin disease characterized by well-demarcated papules and plaques covered with silvery-white scales.

According to the present invention, a pharmaceutical composition for use in the prevention or treatment of angiogenesis-related disease or MMP (Matrix metalloproteinase)-mediated disease comprising the fractional extract of melissa leaf as effective component is effective in preventing or treating psoriasis by suppressing significant epidermal thickness, inflammatory cell infiltration of ear tissue, and mRNA expression level in ear tissue.

According to the embodiment of the present invention, when the angiogenesis-related disease or MMP-mediated disease is psoriasis, a pharmaceutical composition for use in the prevention or treatment of angiogenesis-related disease or MMP (Matrix metalloproteinase)-mediated disease comprising the fractional extract of melissa leaf as effective component comprises a fractional extract of melissa leaf in an amount of 20 weight % to 80 weight % based on the total weight.

When the angiogenesis-related disease or MMP-mediated disease is psoriasis, a pharmaceutical composition for use in the prevention or treatment of angiogenesis-related disease or MMP (Matrix metalloproteinase)-mediated disease comprising the fractional extract of melissa leaf as effective component can be administered in the conventional way by oral, rectal, intravenous, intra-arterial, intraperitoneal, intramuscular, intra-sternal, transdermal, topical, intraocular or intradermal routes, and specifically by oral route.

When the angiogenesis-related disease or MMP-mediated disease is psoriasis, a pharmaceutical composition for use in the prevention or treatment of angiogenesis-related disease or MMP (Matrix metalloproteinase)-mediated disease comprising the fractional extract of melissa leaf as effective component is preferably to be administered at a dose of 0.001 mg/kg to 50 mg/kg when administered once or several times a day.

The matters mentioned in the pharmaceutical composition for the prevention or treatment of angiogenesis-related diseases or matrix metalloproteinase (MMP)-mediated diseases are equally applied to psoriasis, which is an example of angiogenesis-related disease or MMP-mediated disease, unless they contradict each other.

### Angiogenesis-related disease or MMP-mediated disease: Endometriosis

According to the embodiment of the present invention, the angiogenesis-related disease or MMP-mediated disease may be endometriosis.

According to the present invention, the term "endometriosis" refers to a disease in which endometrial tissue is present in the ovaries, posterior wall, uterine ligament, pelvic wall, etc., and various symptoms such as pain and bleeding, etc. occur.

According to the present invention, a pharmaceutical composition for use in the prevention or treatment of angiogenesis-related disease or MMP (Matrix metalloproteinase)-mediated disease comprising the fractional extract of melissa leaf as effective component is effective in preventing or treating endometriosis by reducing the size of the uterine lesions significantly.

According to the embodiment of the present invention, when the angiogenesis-related disease or MMP-mediated disease is endometriosis, a pharmaceutical composition for use in the prevention or treatment of angiogenesis-related disease or MMP (Matrix metalloproteinase)-mediated disease comprising the fractional extract of melissa leaf as effective component can be administered in the conventional way by oral, rectal, intravenous, intra-arterial, intraperitoneal, intramuscular, intra-sternal, transdermal, topical, intraocular or intradermal routes, and specifically by oral route.

According to the embodiment of the present invention, when the angiogenesis-related disease or MMP-mediated disease is endometriosis, a pharmaceutical composition for use in the prevention or treatment of angiogenesis-related disease or MMP (Matrix metalloproteinase)-mediated disease comprising the fractional extract of melissa leaf as effective component is preferably to be administered at a dose of 0.001 mg/kg to 50 mg/kg when administered once or several times a day.

The matters mentioned in the pharmaceutical composition for use in the prevention or treatment of angiogenesis-related diseases or matrix metalloproteinase (MMP)-mediated diseases are equally applied to endometriosis, which is an example of angiogenesis-related disease or MMP-mediated disease, unless they contradict each other.

### Angiogenesis-related disease or MMP-mediated disease: Cancer

According to the embodiment of the present invention, the angiogenesis-related disease or MMP-mediated disease may be cancer growth or cancer metastasis.

According to the present invention, the term "cancer" refers to a disease in which cell cycle is not regulated and cell division continues.

According to the present invention, a pharmaceutical composition for use in the prevention or treatment of angiogenesis-related disease or MMP (Matrix metalloproteinase)-mediated disease comprising the fractional extract of melissa leaf as effective component is effective in preventing or treating cancer by reducing the size of cancerous tumors significantly.

According to the embodiment of the present invention, when the angiogenesis-related disease or MMP-mediated disease is cancer growth or cancer metastasis, a pharmaceutical composition for use in the prevention or treatment of angiogenesis-related disease or MMP (Matrix metalloproteinase)-mediated disease comprising the fractional extract of melissa leaf as effective component comprises a fractional extract of melissa leaf in an amount of 20 weight % to 80 weight % based on the total weight.

The cancer may be any one of lung cancer, non-small cell lung cancer (NSCL), bronchoalveolar cell lung cancer, stomach cancer, gastrointestinal cancer, liver cancer, bone cancer, pancreatic cancer, skin cancer, head and neck cancer, skin or eye melanoma, ovarian cancer, rectal cancer, colorectal cancer, colon cancer, breast cancer, fallopian tube carcinoma, endometrial carcinoma, vaginal carcinoma, vulvar carcinoma, esophageal cancer, laryngeal cancer, small intestine cancer, thyroid cancer, parathyroid cancer, soft tissue sarcoma, urethral cancer, penile cancer, prostate cancer, multiple myeloma, chronic or acute leukemia, childhood solid tumor, lymphoma, bladder cancer, kidney cancer, renal cell carcinoma, renal pelvic carcinoma, axial contraction tumors, brainstem glioma, or pituitary adenoma, and specifically colorectal cancer.

According to the embodiment of the present invention, when the angiogenesis-related disease or MMP-mediated disease is cancer growth or cancer metastasis, a pharmaceutical composition for use in the prevention or treatment of angiogenesis-related disease or MMP (Matrix metalloproteinase)-mediated disease comprising the fractional extract of melissa leaf as effective component can be administered in the conventional way by oral, rectal, intravenous, intra-arterial, intraperitoneal, intramuscular, intra-sternal, transdermal, topical, intraocular or intradermal routes, and specifically by oral route.

When the angiogenesis-related disease or MMP-mediated disease is cancer growth or cancer metastasis, a pharmaceutical composition for use in the prevention or treatment of angiogenesis-related disease or MMP (Matrix metalloproteinase)-mediated disease comprising the fractional extract of melissa leaf as effective component is preferably to be administered at a dose of 0.001 mg/kg to 50 mg/kg when administered once or several times a day.

The matters mentioned in the pharmaceutical composition for use in the prevention or treatment of angiogenesis-related diseases or matrix metalloproteinase (MMP)-mediated diseases are equally applied to cancer, which is an example of angiogenesis-related disease or MMP-mediated disease, unless they contradict each other.

### Angiogenesis-related disease or MMP-mediated disease: Arteriosclerosis

According to the embodiment of the present invention, the angiogenesis-related disease or MMP-mediated disease may be arteriosclerosis.

According to the present invention, the term "arteriosclerosis" refers to a disease in which blood vessels are narrowed and hardened and blocked when cholesterol or triglycerides are accumulated in the innermost lining of blood vessels.

According to the present invention, a pharmaceutical composition for use in the prevention or treatment of angiogenesis-related disease or MMP (Matrix metalloproteinase)-mediated disease comprising the fractional extract of melissa leaf as effective component is effective in preventing or treating arteriosclerosis by inhibiting the increase in atherosclerotic plaques significantly

According to the embodiment of the present invention, when the angiogenesis-related disease or MMP-mediated disease is arteriosclerosis, a pharmaceutical composition for use in the prevention or treatment of angiogenesis-related disease or MMP (Matrix metalloproteinase)-mediated disease comprising the fractional extract of melissa leaf as effective component comprises a fractional extract of melissa leaf in an amount of 20 weight % to 80 weight % based on the total weight.

When the angiogenesis-related disease or MMP-mediated disease is arteriosclerosis, a pharmaceutical composition for use in the prevention or treatment of angiogenesis-related disease or MMP (Matrix metalloproteinase)-mediated disease comprising the fractional extract of melissa leaf as effective component can be administered in the conventional way by oral, rectal, intravenous, intra-arterial, intraperitoneal, intramuscular, intra-sternal, transdermal, topical, intraocular or intradermal routes, and specifically by oral route.

When the angiogenesis-related disease or MMP-mediated disease is arteriosclerosis, a pharmaceutical composition for use in the prevention or treatment of angiogenesis-related disease or MMP (Matrix metalloproteinase)-mediated disease comprising the fractional extract of melissa leaf as effective component is preferably to be administered at a dose of 0.001 mg/kg to 50 mg/kg when administered once or several times a day.

The matters mentioned in the pharmaceutical composition for use in the prevention or treatment of angiogenesis-related diseases or matrix metalloproteinase (MMP)-mediated diseases are equally applied to arteriosclerosis, which is an example of angiogenesis-related disease or MMP-mediated disease, unless they contradict each other.

## Angiogenesis-related disease or MMP-mediated disease: arthritis

According to the embodiment of the present invention, the angiogenesis-related disease or MMP-mediated disease may be arthritis.

According to the present invention, the term "arthritis" refers to a disease in which injury or inflammation occur at the joints where bones meet by multiple causes.

According to the present invention, a pharmaceutical composition for use in the prevention or treatment of angiogenesis-related disease or MMP (Matrix metalloproteinase)-mediated disease comprising the fractional extract of melissa leaf as effective component is effective in preventing or treating arthritis by exhibiting excellent arthritis treatment effect and excellent weight-bearing effect.

According to the embodiment of the present invention, when the angiogenesis-related disease or MMP-mediated disease is arthritis, a pharmaceutical composition for use in the prevention or treatment of angiogenesis-related disease or MMP (Matrix metalloproteinase)-mediated disease comprising the fractional extract of melissa leaf as effective component comprises a fractional extract of melissa leaf in an amount of 20 weight % to 80 weight % based on the total weight.

The arthritis may be any one of osteoarthritis, degenerative arthritis, dissociative osteochondritis, joint ligament injury, psoriatic arthritis, ankylosing spondylitis, and rheumatoid arthritis, specifically osteoarthritis and rheumatoid arthritis.

When the angiogenesis-related disease or MMP-mediated disease is arthritis, a pharmaceutical composition for use in the prevention or treatment of angiogenesis-related disease or MMP (Matrix metalloproteinase)-mediated disease comprising the fractional extract of melissa leaf as effective component can be administered in the conventional way by oral, rectal, intravenous, intra-arterial, intraperitoneal, intramuscular, intra-sternal, transdermal, topical, intraocular or intradermal routes, and specifically by oral route.

When the angiogenesis-related disease or MMP-mediated disease is arthritis, a pharmaceutical composition for use in the prevention or treatment of angiogenesis-related disease or MMP (Matrix metalloproteinase)-mediated disease comprising the fractional extract of melissa leaf as effective component is preferably to be administered at a dose of 0.001 mg/kg to 50 mg/kg when administered once or several times a day.

The matters mentioned in the pharmaceutical composition for use in the prevention or treatment of angiogenesis-related diseases or matrix metalloproteinase (MMP)-mediated diseases are equally applied to arthritis, which is an example of angiogenesis-related disease or MMP-mediated disease, unless they contradict each other.

## Angiogenesis-related disease or MMP-mediated disease: inflammatory bowel disease

According to the embodiment of the present invention, the angiogenesis-related disease or MMP-mediated disease may be inflammatory bowel disease.

According to the present invention, the term "inflammatory bowel disease" refers to a disease in which abnormal chronic inflammation in the intestinal tract repeats improvement and recurrence.

According to the present invention, a pharmaceutical composition for use in the prevention or treatment of angiogenesis-related disease or MMP (Matrix metalloproteinase)-mediated disease comprising the fractional extract of melissa leaf as effective component is effective in preventing or treating inflammatory bowel disease by showing a low disease activity index.

According to the embodiment of the present invention, when the angiogenesis-related disease or MMP-mediated disease is inflammatory bowel disease, a pharmaceutical composition for use in the prevention or treatment of angiogenesis-related disease or MMP (Matrix metalloproteinase)-mediated disease comprising the fractional extract of melissa leaf as effective component comprises a fractional extract of melissa leaf in an amount of 20 weight % to 80 weight % based on the total weight.

The inflammatory bowel disease may be any one of ulcerative colitis, Crohn's disease, intestinal tuberculosis, ischemic colitis, and intestinal ulcer according to Behcet's disease, specifically ulcerative colitis.

When the angiogenesis-related disease or MMP-mediated disease is inflammatory bowel disease, a pharmaceutical composition for use in the prevention or treatment of angiogenesis-related disease or MMP (Matrix metalloproteinase)-mediated disease comprising the fractional extract of melissa leaf as effective component can be administered in the conventional way by oral, rectal, intravenous, intra-arterial, intraperitoneal, intramuscular, intra-sternal, transdermal, topical, intraocular or intradermal routes, and specifically by oral route.

When the angiogenesis-related disease or MMP-mediated disease is inflammatory bowel disease, a pharmaceutical composition for use in the prevention or treatment of angiogenesis-related disease or MMP (Matrix metalloproteinase)-mediated disease comprising the fractional extract of melissa leaf as effective component is preferably to be administered at a dose of 0.001 mg/kg to 50 mg/kg when administered once or several times a day.

The matters mentioned in the pharmaceutical composition for use in the prevention or treatment of angiogenesis-related diseases or matrix metalloproteinase (MMP)-mediated diseases are equally applied to inflammatory bowel disease, which is an example of angiogenesis-related disease or MMP-mediated disease, unless they contradict each other.

## Angiogenesis-related disease or MMP-mediated disease: Alzheimer's

According to the embodiment of the present invention, the angiogenesis-related disease or MMP-mediated disease may be Alzheimer's.

According to the present invention, the term "Alzheimer" refers to the most common cause of dementia, which causes dementia symptoms due to degenerative changes in brain cells.

According to the present invention, a pharmaceutical composition for use in the prevention or treatment of angiogenesis-related disease or MMP (Matrix metalloproteinase)-mediated disease comprising the fractional extract of melissa leaf as effective component is effective in preventing or treating Alzheimer's by improving cognitive function.

According to the embodiment of the present invention, when the angiogenesis-related disease or MMP-mediated disease is Alzheimer's, a pharmaceutical composition for use in the prevention or treatment of angiogenesis-related disease or MMP (Matrix metalloproteinase)-mediated disease comprising the fractional extract of melissa leaf as effective component comprises a fractional extract of melissa leaf in an amount of 20 weight % to 80 weight % based on the total weight.

According to the present invention, when the angiogenesis-related disease or MMP-mediated disease is Alzheimer's, a pharmaceutical composition for use in the prevention or treatment of angiogenesis-related disease or MMP (Matrix metalloproteinase)-mediated disease comprising the fractional extract of melissa leaf as effective component can be administered in the conventional way by oral, rectal, intravenous, intra-arterial, intraperitoneal, intramuscular, intra-sternal, transdermal, topical, intraocular or intradermal routes, and specifically by oral route.

According to the embodiment of the present invention, when the angiogenesis-related disease or MMP-mediated disease is Alzheimer's, a pharmaceutical composition for use in the prevention or treatment of angiogenesis-related disease or MMP (Matrix metalloproteinase)-mediated disease comprising the fractional extract of melissa leaf as effective component is preferably to be administered at a dose of 0.001 mg/kg to 50 mg/kg when administered once or several times a day.

The matters mentioned in the pharmaceutical composition for use in the prevention or treatment of angiogenesis-related diseases or matrix metalloproteinase (MMP)-mediated diseases are equally applied to Alzheimer's, which is an example of angiogenesis-related disease or MMP-mediated disease, unless they contradict each other.

## Angiogenesis-related disease or MMP-mediated disease: periodontal disease

According to the embodiment of the present invention, the angiogenesis-related disease or MMP-mediated disease may be periodontal disease.

According to the present invention, the term "periodontal disease" refers to a disease that appears in the tissues surrounding the teeth such as the gingiva, periodontal ligament, and alveolar bone.

According to the present invention, a pharmaceutical composition for use in the prevention or treatment of angiogenesis-related disease or MMP (Matrix metalloproteinase)-mediated disease comprising the fractional extract of melissa leaf as effective component has a significant therapeutic effect on periodontal disease by effectively regenerating gum tissues due to increased expression of pro-collagen.

According to the embodiment of the present invention, when the angiogenesis-related disease or MMP-mediated disease is periodontal disease, a pharmaceutical composition for use in the prevention or treatment of angiogenesis-related disease or MMP (Matrix metalloproteinase)-mediated disease comprising the fractional extract of melissa leaf as effective component comprises a fractional extract of melissa leaf in an amount of 20 weight % to 80 weight % based on the total weight.

When the angiogenesis-related disease or MMP-mediated disease is periodontal disease, a pharmaceutical composition for use in the prevention or treatment of angiogenesis-related disease or MMP (Matrix metalloproteinase)-mediated disease comprising the fractional extract of melissa leaf as effective component can be administered in the conventional way by oral, rectal, intravenous, intra-arterial, intraperitoneal, intramuscular, intra-sternal, transdermal, topical, intraocular or intradermal routes, and specifically by oral route.

When the angiogenesis-related disease or MMP-mediated disease is periodontal disease, a pharmaceutical composition for use in the prevention or treatment of angiogenesis-related disease or MMP (Matrix metalloproteinase)-mediated disease comprising the fractional extract of melissa leaf as effective component is preferably to be administered at a dose of 0.001 mg/kg to 50 mg/kg when administered once or several times a day.

The matters mentioned in the pharmaceutical composition for use in the prevention or treatment of angiogenesis-related diseases or matrix metalloproteinase (MMP)-mediated diseases are equally applied to periodontal disease, which is an example of angiogenesis-related disease or MMP-mediated disease, unless they contradict each other.

## Angiogenesis-related disease or MMP-mediated disease: diabetic retinopathy

According to the embodiment of the present invention, the angiogenesis-related disease or MMP-mediated disease may be diabetic retinopathy.

According to the present invention, the term "diabetic retinopathy" means a state in which the blood vessels in the retina of our eyes are damaged by diabetes.

According to the present invention, a pharmaceutical composition for use in the prevention or treatment of angiogenesis-related disease or MMP (Matrix metalloproteinase)-mediated disease comprising the fractional extract of melissa leaf as effective component is effective in preventing or treating diabetic retinopathy by excellent inhibitory effect on retinal vascular leakage.

When the angiogenesis-related disease or MMP-mediated disease is diabetic retinopathy, a pharmaceutical composition for use in the prevention or treatment of angiogenesis-related disease or MMP (Matrix metalloproteinase)-mediated disease comprising the fractional extract of melissa leaf as effective component comprises a fractional extract of melissa leaf in an amount of 20 weight % to 80 weight % based on the total weight.

When the angiogenesis-related disease or MMP-mediated disease is diabetic retinopathy, a pharmaceutical composition for use in the prevention or treatment of angiogenesis-related disease or MMP (Matrix metalloproteinase)-mediated disease comprising the fractional extract of melissa leaf as effective component can be administered in the conventional way by oral, rectal, intravenous, intra-arterial, intraperitoneal, intramuscular, intra-sternal, transdermal, topical, intraocular or intradermal routes, and specifically by oral route.

When the angiogenesis-related disease or MMP-mediated disease is diabetic retinopathy, a pharmaceutical composition for use in the prevention or treatment of angiogenesis-related disease or MMP (Matrix metalloproteinase)-mediated disease comprising the fractional extract of melissa leaf as effective component is preferably to be administered at a dose of 0.001 mg/kg to 50 mg/kg when administered once or several times a day.

The matters mentioned in the pharmaceutical composition for use in the prevention or treatment of angiogenesis-related diseases or matrix metalloproteinase (MMP)-mediated diseases are equally applied to diabetic retinopathy, which is an example of angiogenesis-related disease or MMP-mediated disease, unless they contradict each other.

## Angiogenesis-related disease or MMP-mediated disease: Sjogren's syndrome

According to the embodiment of the present invention, the angiogenesis-related disease or MMP-mediated disease may be Sjogren's syndrome.

According to the present invention, the term "Sjogren's syndrome" refers to an autoimmune disease in which the body's immune system attacks glands that produce moisture (fluids).

According to the present invention, a pharmaceutical composition for use in the prevention or treatment of angiogenesis-related disease or MMP (Matrix metalloproteinase)-mediated disease comprising the fractional extract of melissa leaf as effective component is effective in preventing or treating Sjogren's syndrome by increasing the amount of salivary and tear secretion.

When the angiogenesis-related disease or MMP-mediated disease is Sjogren's syndrome, a pharmaceutical composition for use in the prevention or treatment of angiogenesis-related disease or MMP (Matrix metalloproteinase)-mediated disease comprising the fractional extract of melissa leaf as effective component comprises a fractional extract of melissa leaf in an amount of 20 weight % to 80 weight % based on the total weight.

When the angiogenesis-related disease or MMP-mediated disease is Sjogren's syndrome, a pharmaceutical composition for use in the prevention or treatment of angiogenesis-related disease or MMP (Matrix metalloproteinase)-mediated disease comprising the fractional extract of melissa leaf as effective component can be administered in the conventional way by oral, rectal, intravenous, intra-arterial, intraperitoneal, intramuscular, intra-sternal, transdermal, topical, intraocular or intradermal routes, and specifically by oral route.

When the angiogenesis-related disease or MMP-mediated disease is Sjogren's syndrome, a pharmaceutical composition for use in the prevention or treatment of angiogenesis-related disease or MMP (Matrix metalloproteinase)-mediated disease comprising the fractional extract of melissa leaf as effective component is preferably to be administered at a dose of 0.001 mg/kg to 50 mg/kg when administered once or several times a day.

The matters mentioned in the pharmaceutical composition for use in the prevention or treatment of angiogenesis-related diseases or matrix metalloproteinase (MMP)-mediated diseases are equally applied to Sjogren's syndrome, which is an example of angiogenesis-related disease or MMP-mediated disease, unless they contradict each other.

# EP 4 134 091 B1

**Angiogenesis-related disease or MMP-mediated disease: glaucoma**

According to the embodiment of the present invention, the angiogenesis-related disease or MMP-mediated disease may be glaucoma.

According to the present invention, the term "glaucoma" refers to a disease occurring in the optic nerve including retinal plexus cells while taking the form of optic nerve atrophy.

According to the present invention, a pharmaceutical composition for use in the prevention or treatment of angiogenesis-related disease or MMP (Matrix metalloproteinase)-mediated disease comprising the fractional extract of melissa leaf as effective component is effective in preventing or treating glaucoma by remarkably reducing the elevated intraocular pressure.

When the angiogenesis-related disease or MMP-mediated disease is glaucoma, a pharmaceutical composition for use in the prevention or treatment of angiogenesis-related disease or MMP (Matrix metalloproteinase)-mediated disease comprising the fractional extract of melissa leaf as effective component comprises a fractional extract of melissa leaf in an amount of 20 weight % to 80 weight % based on the total weight.

When the angiogenesis-related disease or MMP-mediated disease is glaucoma, a pharmaceutical composition for use in the prevention or treatment of angiogenesis-related disease or MMP (Matrix metalloproteinase)-mediated disease comprising the fractional extract of melissa leaf as effective component can be administered in the conventional way by oral, rectal, intravenous, intra-arterial, intraperitoneal, intramuscular, intra-sternal, transdermal, topical, intraocular or intradermal routes, and specifically by oral route.

When the angiogenesis-related disease or MMP-mediated disease glaucoma, a pharmaceutical composition for use in the prevention or treatment of angiogenesis-related disease or MMP (Matrix metalloproteinase)-mediated disease comprising the fractional extract of melissa leaf as effective component is preferably to be administered at a dose of 0.001 mg/kg to 50 mg/kg when administered once or several times a day.

The matters mentioned in the pharmaceutical composition for use in the prevention or treatment of angiogenesis-related diseases or matrix metalloproteinase (MMP)-mediated diseases are equally applied to glaucoma, which is an example of angiogenesis-related disease or MMP-mediated disease, unless they contradict each other.

**Food composition comprising a fractional extract of melissa leaf as effective component**

The present invention can be used to provide a food composition comprising a fractional extract of melissa leaf as effective component comprising caffeic acid, EDPA, RME and rosmarinic acid.

The fractional extract of melissa leaf and its preparation method thereof are the same as described above.

The food composition may be used for preventing or improving angiogenesis-related diseases or MMP-mediated diseases.

The angiogenesis-related disease or MMP-mediated disease may be any one of obesity, age-related macular degeneration, psoriasis, endometriosis, cancer growth or cancer metastasis, arteriosclerosis, arthritis, inflammatory bowel disease, Alzheimer's disease, periodontal disease, diabetic retinopathy, Sjogren's syndrome, or glaucoma, but is not limited thereto.

The cancer may be any one of lung cancer, non-small cell lung cancer (NSCL), bronchoalveolar cell lung cancer, stomach cancer, gastrointestinal cancer, liver cancer, bone cancer, pancreatic cancer, skin cancer, head and neck cancer, skin or eye melanoma, ovarian cancer, rectal cancer, colorectal cancer, colon cancer, breast cancer, fallopian tube carcinoma, endometrial carcinoma, vaginal carcinoma, vulvar carcinoma, esophageal cancer, laryngeal cancer, small intestine cancer, thyroid cancer, parathyroid cancer, soft tissue sarcoma, urethral cancer, penile cancer, prostate cancer, multiple myeloma, chronic or acute leukemia, childhood solid tumor, lymphoma, bladder cancer, kidney cancer, renal cell carcinoma, renal pelvic carcinoma, axial contraction tumors, brainstem glioma, or pituitary adenoma, and specifically colorectal cancer, but is not limited thereto.

The arthritis may be any one of osteoarthritis, degenerative arthritis, dissociative osteochondritis, joint ligament injury, psoriatic arthritis, ankylosing spondylitis, and rheumatoid arthritis, specifically osteoarthritis and rheumatoid arthritis, but is not limited thereto.

When the fractional extract of melissa leaf is used as an additive in a food composition, it can be added as it is or used with other foods or food ingredients, and can be appropriately used according to a conventional method. The mixing amount of the effective component may be appropriately determined according to each purpose of use, such as prevention, health or treatment.

The formulation of the food composition may be possible in any form of general food or beverage as well as powder, granule, pill, tablet, and capsule.

There is no particular limitation on the type of the food, and examples of the food to which the substance can be added include meat, sausage, bread, chocolate, candy, snacks, confectionery, pizza, ramen, other noodles, gums, and dairy products including ice cream, various soups, beverages, teas, alcoholic beverages, and vitamin complex, and may include all foods in a conventional sense.

In general, in the manufacture of food or beverage, the fractional extract of melissa leaf may be added in an amount of 15 parts by weight or less, preferably 10 parts by weight or less, based on 100 parts by weight of the raw material. However, in the case of long-term intake for health and hygiene or health control, the amount may be less than or equal to the above range. In addition, since there is no problem in terms of safety using a fraction from a natural product in the present invention, it can also be used in an amount above the range.

Beverages in the food composition may comprise various flavoring agents or natural carbohydrates as additional ingredients like conventional beverages. The above-mentioned natural carbohydrates may be monosaccharides such as glucose and fructose, disaccharides such as maltose and sucrose, polysaccharides such as dextrin and cyclodextrin, and sugar alcohols such as xylitol, sorbitol, and erythritol. As the sweetener, natural sweeteners such as thaumatin and stevia extract, or synthetic sweeteners such as saccharin and aspartame, and the like may be used. The ratio of the natural carbohydrate may be about 0.01 to 0.04 g, preferably about 0.02 to 0.03 g per 100 mL of the beverage according to the present invention.

In addition to the above, the food composition can include various nutrients, vitamins, electrolytes, flavoring agents, coloring agents, pectic acid and its salts, alginic acid and its salts, organic acids, protective colloidal thickeners, pH adjusters, stabilizers, preservatives, glycerin, alcohol, and carbonating agents used in carbonated beverages. In addition, the food composition comprising a fractional extract of melissa leaf as effective component may comprise natural fruit juice, and fruit pulp for making fruit juice and vegetable beverages. These components may be used independently or in combination. The ratio of these additives is not limited, but is generally selected in the range of 0.01 to 0.1 parts by weight relative to 100 parts by weight of the food composition of the present invention.

## Use in a method for prevention or treatment

The present invention can be used in a method for preventing or treating angiogenesis-related diseases or matrix metalloproteinase (MMP)-mediated diseases by administering a therapeutically effective amount of a fractional extract of melissa leaf to a subject in need of treatment.

Specifically, according to the type of angiogenesis-related diseases or MMP (Matrix metalloproteinase)-mediated diseases, the following prevention or treatment method can be used.

In a method for preventing or treating obesity a therapeutically effective amount of a fractional extract of melissa leaf may be administered to a subject in need of treatment.

In a method for preventing or treating age-related macular degeneration a therapeutically effective amount of a fractional extract of melissa leaf may be administered to a subject in need of treatment.

In a method for preventing or treating psoriasis a therapeutically effective amount of a fractional extract of melissa leaf may be administered to a subject in need of treatment.

In a method for preventing or treating endometriosis a therapeutically effective amount of a fractional extract of melissa leaf may be administered to a subject in need of treatment.

In a method for preventing or treating cancer a therapeutically effective amount of a fractional extract of melissa leaf may be administered to a subject in need of treatment.

In a method for preventing or treating arteriosclerosis a therapeutically effective amount of a fractional extract of melissa leaf may be administered to a subject in need of treatment.

In a method for preventing or treating arthritis a therapeutically effective amount of a fractional extract of melissa leaf may be administered to a subject in need of treatment.

A method for preventing or treating inflammatory bowel disease is provided by administering a therapeutically effective amount of a fractional extract of melissa leaf may be administered to a subject in need of treatment.

In a method for preventing or treating Alzheimer's disease a therapeutically effective amount of a fractional extract of melissa leaf may be administered to a subject in need of treatment.

In a method for preventing or treating periodontal disease a therapeutically effective amount of a fractional extract of melissa leaf may be administered to a subject in need of treatment.

In a method for preventing or treating diabetic retinopathy a therapeutically effective amount of a fractional extract of melissa leaf may be administered to a subject in need of treatment.

In a method for preventing or treating Sjogren's syndrome a therapeutically effective amount of a fractional extract of melissa leaf may be administered to a subject in need of treatment.

In a method for preventing or treating glaucoma is provided by administering a therapeutically effective amount of a fractional extract of melissa leaf may be administered to a subject in need of treatment.

According to the embodiments of the present invention, the term "subject in need of treatment" refers to mammals including humans, and the term "administration" refers to providing a predetermined substance to a patient by any suitable method. The pharmaceutical composition for use in the prevention or treatment of angiogenesis-related diseases or MMP (Matrix metalloproteinase)-mediated diseases comprising the fractional extract of melissa leaf of the present invention as effective component may be administered in a conventional manner by oral, rectal, intravenous, intra-arterial, intraperitoneal, intramuscular, intra-sternal, transdermal, topical, intraocular or intradermal route.

In the method for preventing or treating obesity which is an example of angiogenesis-related disease or MMP-mediated disease, the fractional extract of melissa leaf of the present invention may be administered in a conventional manner by oral, rectal, intravenous, intra-arterial, intraperitoneal, intramuscular, intra-sternal, transdermal, topical, intraocular or intradermal route, specifically oral route.

In the method for preventing or treating age-related macular degeneration which is an example of angiogenesis-related disease or MMP-mediated disease, the fractional extract of melissa leaf of the present invention may be administered in a conventional manner by oral, rectal, intravenous, intra-arterial, intraperitoneal, intramuscular, intra-sternal, transdermal, topical, intraocular or intradermal route, specifically oral route.

In the method for preventing or treating psoriasis which is an example of angiogenesis-related disease or MMP-mediated disease, the fractional extract of melissa leaf of the present invention may be administered in a conventional manner by oral, rectal, intravenous, intra-arterial, intraperitoneal, intramuscular, intra-sternal, transdermal, topical, intraocular or intradermal route, specifically oral route.

In the method for preventing or treating endometriosis which is an example of angiogenesis-related disease or MMP-mediated disease, the fractional extract of melissa leaf of the present invention may be administered in a conventional manner by oral, rectal, intravenous, intra-arterial, intraperitoneal, intramuscular, intra-sternal, transdermal, topical, intraocular or intradermal route, specifically oral route.

In the method for preventing or treating cancer which is an example of angiogenesis-related disease or MMP-mediated disease, the fractional extract of melissa leaf of the present invention may be administered in a conventional manner by oral, rectal, intravenous, intra-arterial, intraperitoneal, intramuscular, intra-sternal, transdermal, topical, intraocular or intradermal route, specifically oral route.

In the method for preventing or treating arteriosclerosis which is an example of angiogenesis-related disease or MMP-mediated disease, the fractional extract of melissa leaf of the present invention may be administered in a conventional manner by oral, rectal, intravenous, intra-arterial, intraperitoneal, intramuscular, intra-sternal, transdermal, topical, intraocular or intradermal route, specifically oral route.

In the method for preventing or treating arthritis which is an example of angiogenesis-related disease or MMP-mediated disease, the fractional extract of melissa leaf of the present invention may be administered in a conventional manner by oral, rectal, intravenous, intra-arterial, intraperitoneal, intramuscular, intra-sternal, transdermal, topical, intraocular or intradermal route, specifically oral route.

In the method for preventing or treating inflammatory bowel disease which is an example of angiogenesis-related disease or MMP-mediated disease, the fractional extract of melissa leaf of the present invention may be administered in a conventional manner by oral, rectal, intravenous, intra-arterial, intraperitoneal, intramuscular, intra-sternal, transdermal, topical, intraocular or intradermal route, specifically oral route.

In the method for preventing or treating Alzheimer's disease which is an example of angiogenesis-related disease or MMP-mediated disease, the fractional extract of melissa leaf of the present invention may be administered in a conventional manner by oral, rectal, intravenous, intra-arterial, intraperitoneal, intramuscular, intra-sternal, transdermal, topical, intraocular or intradermal route, specifically oral route.

In the method for preventing or treating periodontal disease which is an example of angiogenesis-related disease or MMP-mediated disease, the fractional extract of melissa leaf of the present invention may be administered in a conventional manner by oral, rectal, intravenous, intra-arterial, intraperitoneal, intramuscular, intra-sternal, transdermal, topical, intraocular or intradermal route, specifically oral route.

In the method for preventing or treating diabetic retinopathy which is an example of angiogenesis-related disease or MMP-mediated disease, the fractional extract of melissa leaf of the present invention may be administered in a conventional manner by oral, rectal, intravenous, intra-arterial, intraperitoneal, intramuscular, intra-sternal, transdermal, topical, intraocular or intradermal route, specifically oral route.

In the method for preventing or treating Sjogren's syndrome which is an example of angiogenesis-related disease or MMP-mediated disease, the fractional extract of melissa leaf of the present invention may be administered in a conventional manner by oral, rectal, intravenous, intra-arterial, intraperitoneal, intramuscular, intra-sternal, transdermal, topical, intraocular or intradermal route, specifically oral route.

In the method for preventing or treating glaucoma which is an example of angiogenesis-related disease or MMP-mediated disease, the fractional extract of melissa leaf of the present invention may be administered in a conventional manner by oral, rectal, intravenous, intra-arterial, intraperitoneal, intramuscular, intra-sternal, transdermal, topical, intraocular or intradermal route, specifically oral route.

According to the present invention, the term "therapeutically effective amount" refers to the amount of an effective component or a pharmaceutical composition that induces biological or medical response in a tissue system, animal or human that is considered by researchers, veterinarians, medical doctors, or other clinician, which includes an amount that induces amelioration of the symptoms of the disease or disorder being treated. It is apparent to those skilled in the art that the therapeutically effective dosage and frequency of administration for the effective component of the present invention will vary depending on the desired effect. Therefore, the optimal dosage to be administered can be easily determined by those skilled in the art, and can be adjusted by various factors including the type of disease, the severity of the disease, the content of effective components and other ingredients comprised in the composition, the type of formulation, and patient's age, weight, general health, sex and diet, administration time, administration route and secretion rate of the composition, treatment period, and concomitant drugs.

In the method for preventing or treating obesity which is an example of angiogenesis-related disease or MMP-mediated disease, the fractional extract of melissa leaf of the present invention is preferably to be administered to adults at a dose of 0.001 mg/kg to 50 mg/kg when administered once or several times a day.

In the method for preventing or treating age-related macular degeneration which is an example of angiogenesis-related disease or MMP-mediated disease, the fractional extract of melissa leaf of the present invention is preferably to be administered to adults at a dose of 0.001 mg/kg to 50 mg/kg when administered once or several times a day.

In the method for preventing or treating psoriasis which is an example of angiogenesis-related disease or MMP-mediated disease, the fractional extract of melissa leaf of the present invention is preferably to be administered to adults at a dose of 0.001 mg/kg to 50 mg/kg when administered once or several times a day.

In the method for preventing or treating endometriosis which is an example of angiogenesis-related disease or MMP-mediated disease, the fractional extract of melissa leaf of the present invention is preferably to be administered to adults at a dose of 0.001 mg/kg to 50 mg/kg when administered once or several times a day.

In the method for preventing or treating cancer which is an example of angiogenesis-related disease or MMP-mediated disease, the fractional extract of melissa leaf of the present invention is preferably to be administered to adults at a dose of 0.001 mg/kg to 50 mg/kg when administered once or several times a day.

In the method for preventing or treating arteriosclerosis, which is an example of angiogenesis-related disease or MMP-mediated disease, the fractional extract of melissa leaf of the present invention is preferably to be administered to adults at a dose of 0.001 mg/kg to 50 mg/kg when administered once or several times a day.

In the method for preventing or treating arthritis, which is an example of angiogenesis-related disease or MMP-mediated disease, the fractional extract of melissa leaf of the present invention is preferably to be administered to adults at a dose of 0.001 mg/kg to 50 mg/kg when administered once or several times a day.

In the method for preventing or treating inflammatory bowel disease, which is an example of angiogenesis-related disease or MMP-mediated disease, the fractional extract of melissa leaf of the present invention is preferably to be administered to adults at a dose of 0.001 mg/kg to 50 mg/kg when administered once or several times a day.

In the method for preventing or treating Alzheimer's disease, which is an example of angiogenesis-related disease or MMP-mediated disease, the fractional extract of melissa leaf of the present invention is preferably to be administered to adults at a dose of 0.001 mg/kg to 50 mg/kg when administered once or several times a day.

In the method for preventing or treating periodontal disease, which is an example of angiogenesis-related disease or MMP-mediated disease, the fractional extract of melissa leaf of the present invention is preferably to be administered to adults at a dose of 0.001 mg/kg to 50 mg/kg when administered once or several times a day.

In the method for preventing or treating diabetic retinopathy, which is an example of angiogenesis-related disease or MMP-mediated disease, the fractional extract of melissa leaf of the present invention is preferably to be administered to adults at a dose of 0.001 mg/kg to 50 mg/kg when administered once or several times a day.

In the method for preventing or treating Sjogren's syndrome, which is an example of angiogenesis-related disease or MMP-mediated disease, the fractional extract of melissa leaf of the present invention is preferably to be administered to adults at a dose of 0.001 mg/kg to 50 mg/kg when administered once or several times a day.

In the method for preventing or treating Sjogren's syndrome, which is an example of angiogenesis-related disease or MMP-mediated disease, for adults, the fractional extract of melissa leaf of the present invention is preferably to be administered once to several times a day at a dose of 0.001 mg/kg to 50 mg/kg.

In the method for preventing or treating glaucoma, which is an example of angiogenesis-related disease or MMP-mediated disease, for adults, the fractional extract of melissa leaf of the present invention is preferably to be administered once to several times a day at a dose of 0.001 mg/kg to 50 mg/kg.

According to the present invention, the term "prevention" refers to any action of suppressing or delaying angiogenesis-related diseases or MMP-mediated diseases such as obesity, age-related macular degeneration, psoriasis, endometriosis, cancer growth or cancer metastasis, arteriosclerosis, arthritis, inflammatory bowel disease, Alzheimer's disease, periodontal disease, diabetic retinopathy, Sjogren's syndrome, and glaucoma by administration of the fractional extract of melissa leaf of the present invention.

According to the present invention, the term "treatment" refers to any action of improving or beneficially changing angiogenesis-related diseases or MMP-mediated diseases such as obesity, age-related macular degeneration, psoriasis, endometriosis, cancer growth or cancer metastasis, arteriosclerosis, arthritis, inflammatory bowel disease, Alzheimer's disease, periodontal disease, diabetic retinopathy, Sjogren's syndrome, and glaucoma by administration of the fractional extract of melissa leaf of the present invention.

**A pharmaceutical composition comprising a fractional extract of melissa leaf as effective component for use in the prevention or treatment of angiogenesis-related diseases or MMP (Matrix metalloproteinase)-mediated diseases**

The present invention provides a pharmaceutical composition comprising a fractional extract of melissa leaf as effective component for use in the prevention or treatment of angiogenesis-related diseases or MMP (Matrix metalloproteinase)-mediated diseases,

Specifically, it provides a pharmaceutical composition for use in preventing or treating angiogenesis-related diseases or MMP (Matrix metalloproteinase)-mediated diseases as described below depending on the type of angiogenesis-related diseases or MMP (Matrix metalloproteinase)-mediated diseases.

According to the embodiment of the present invention, it is to provide the pharmaceutical composition comprising a fractional extract of melissa leaf as effective component for use in the prevention or treatment of obesity which is an example of angiogenesis-related disease or MMP-mediated disease.

According to the embodiment of the present invention, it is to provide the pharmaceutical composition comprising a fractional extract of melissa leaf as effective component for use in the prevention or treatment of age-related macular degeneration which is an example of angiogenesis-related disease or MMP-mediated disease.

According to the embodiment of the present invention, it is to provide the pharmaceutical composition comprising a fractional extract of melissa leaf as effective component for use in the prevention or treatment of psoriasis which is an example of angiogenesis-related disease or MMP-mediated disease.

According to the embodiment of the present invention, it is to provide the pharmaceutical composition comprising a fractional extract of melissa leaf as effective component for use in the prevention or treatment of endometriosis which is an example of angiogenesis-related disease or MMP-mediated disease.

According to the embodiment of the present invention, it is to provide the pharmaceutical composition comprising a fractional extract of melissa leaf as effective component for use in the prevention or treatment of cancer which is an example of angiogenesis-related disease or MMP-mediated disease.

According to the embodiment of the present invention, it is to provide the pharmaceutical composition comprising a fractional extract of melissa leaf as effective component for use in the prevention or treatment of arteriosclerosis which is an example of angiogenesis-related disease or MMP-mediated disease.

According to the embodiment of the present invention, it is to provide the pharmaceutical composition comprising a fractional extract of melissa leaf as effective component for use in the prevention or treatment of arthritis which is an example of angiogenesis-related disease or MMP-mediated disease.

According to the embodiment of the present invention, it is to provide the pharmaceutical composition comprising a fractional extract of melissa leaf as effective component for use in the prevention or treatment of inflammatory bowel disease which is an example of angiogenesis-related disease or MMP-mediated disease.

According to the embodiment of the present invention, it is to provide the pharmaceutical composition comprising a fractional extract of melissa leaf as effective component for use in the prevention or treatment of Alzheimer's disease which is an example of angiogenesis-related disease or MMP-mediated disease.

According to the embodiment of the present invention, it is to provide the pharmaceutical composition comprising a fractional extract of melissa leaf as effective component for use in the prevention or treatment of periodontal disease which is an example of angiogenesis-related disease or MMP-mediated disease.

According to the embodiment of the present invention, it is to provide the pharmaceutical composition comprising a fractional extract of melissa leaf as effective component for use in the prevention or treatment of diabetic retinopathy which is an example of angiogenesis-related disease or MMP-mediated disease.

According to the embodiment of the present invention, it is to provide the pharmaceutical composition comprising a fractional extract of melissa leaf as effective component for use in the prevention or treatment of Sjogren's syndrome which is an example of angiogenesis-related disease or MMP-mediated disease.

According to the embodiment of the present invention, it is to provide the pharmaceutical composition comprising a fractional extract of melissa leaf as effective component for use in the prevention or treatment of glaucoma which is an example of angiogenesis-related disease or MMP-mediated disease.

**A pharmaceutical composition comprising a fractional extract of melissa leaf as effective component can be used for the manufacture of a medicament for preventing or treating angiogenesis-related diseases or MMP (Matrix metalloproteinase)-mediated diseases**

The present invention provides a pharmaceutical composition comprising a fractional extract of melissa leaf as effective component that can be used for the manufacture of a medicament for preventing or treating angiogenesis-related diseases or MMP (Matrix metalloproteinase)-mediated diseases.

Specifically, a pharmaceutical composition comprising a fractional extract of melissa leaf as effective component can be used for the manufacture of a medicament for preventing or treating angiogenesis-related diseases or MMP (Matrix metalloproteinase)-mediated diseases as described below depending on the type of angiogenesis-related diseases or MMP (Matrix metalloproteinase)-mediated diseases.

A pharmaceutical composition comprising a fractional extract of melissa leaf as effective component can be used for the manufacture of a medicament for preventing or treating obesity which is an example of angiogenesis-related disease or MMP-mediated disease.

A pharmaceutical composition comprising a fractional extract of melissa leaf as effective component can be used for the manufacture of a medicament for preventing or treating age-related macular degeneration which is an example of angiogenesis-related disease or MMP-mediated disease.

A pharmaceutical composition comprising a fractional extract of melissa leaf as effective component can be used for the manufacture of a medicament for preventing or treating psoriasis which is an example of angiogenesis-related disease or MMP-mediated disease.

A pharmaceutical composition comprising a fractional extract of melissa leaf as effective component for the manufacture of a medicament can be used for preventing or treating endometriosis which is an example of angiogenesis-related disease or MMP-mediated disease.

A pharmaceutical composition comprising a fractional extract of melissa leaf as effective component can be used for the manufacture of a medicament for preventing or treating cancer which is an example of angiogenesis-related disease or MMP-mediated disease.

A pharmaceutical composition comprising a fractional extract of melissa leaf as effective component can be used for the manufacture of a medicament for preventing or treating arteriosclerosis which is an example of angiogenesis-related disease or MMP-mediated disease.

A pharmaceutical composition comprising a fractional extract of melissa leaf as effective component can be used for the manufacture of a medicament for preventing or treating arthritis which is an example of angiogenesis-related disease or MMP-mediated disease.

A pharmaceutical composition comprising a fractional extract of melissa leaf as effective component can be used for the manufacture of a medicament for preventing or treating inflammatory bowel disease which is an example of angiogenesis-related disease or MMP-mediated disease.

A pharmaceutical composition comprising a fractional extract of melissa leaf as effective component can be used for the manufacture of a medicament for preventing or treating Alzheimer's disease which is an example of angiogenesis-related disease or MMP-mediated disease.

A pharmaceutical composition comprising a fractional extract of melissa leaf as effective component can be used for the manufacture of a medicament for preventing or treating periodontal disease which is an example of angiogenesis-related disease or MMP-mediated disease.

A pharmaceutical composition comprising a fractional extract of melissa leaf as effective component can be used for the manufacture of a medicament for preventing or treating diabetic retinopathy which is an example of angiogenesis-related disease or MMP-mediated disease.

A pharmaceutical composition comprising a fractional extract of melissa leaf as effective component can be used for the manufacture of a medicament for preventing or treating Sjogren's syndrome which is an example of angiogenesis-related disease or MMP-mediated disease.

A pharmaceutical composition comprising a fractional extract of melissa leaf as effective component can be used for the manufacture of a medicament for preventing or treating glaucoma which is an example of angiogenesis-related disease or MMP-mediated disease.

A pharmaceutical composition comprising a fractional extract of melissa leaf as effective component can be used for the manufacture of a medicament for preventing or treating angiogenesis-related disease or MMP-mediated disease may be admixed with an acceptable carrier or the like, and may further comprise other agents.

Matters mentioned in the pharmaceutical composition, food composition, treatment method, to be used according to the present invention are equally applied unless they contradict each other.

[Advantageous Effects]

The fractional extract of melissa leaf of the present invention exhibits superior effects on the prevention and treatment of non-alcoholic steatohepatitis and non-alcoholic fatty liver disease compared with the extract of melissa leaf extract extracted by other extraction methods. Therefore, the composition comprising the fractional extract of melissa leaf as effective component of the present invention can be useful for the prevention or treatment of non-alcoholic steatohepatitis and non-alcoholic fatty liver disease, which has significantly fewer side effects and exhibits excellent pharmacological effects.

Specifically, the pharmaceutical composition according to the present invention, comprising the fractional extract of melissa leaf as effective component for use in the prevention or treatment of non-alcoholic steatohepatitis is effective in preventing or treating non-alcoholic steatohepatitis by significant inhibition of fibrosis and protein expression levels of Col1A2, TGF beta, IL-6 and IL-10.

The pharmaceutical composition according to the present invention, comprising the fractional extract of melissa leaf as effective component for use in the prevention or treatment of non-alcoholic fatty liver disease is effective in preventing or treating non-alcoholic fatty liver disease by showing the inhibitory effects on hepatic fat accumulation, blood triglycerides, blood sugar, and total cholesterol.

The fractional extract of melissa leaf of the present invention as effective component comprised in the pharmaceutical composition for use in the prevention or treatment of angiogenesis-related diseases or MMP (matrix metalloproteinase)-mediated diseases, is superior to the fractional extract of melissa leaf extracted by other extraction methods in preventing or treating obesity, age-related macular degeneration, psoriasis, endometriosis, cancer growth or cancer metastasis, arteriosclerosis, arthritis, inflammatory bowel disease, Alzheimer's disease, periodontal disease, diabetic retinopathy, Sjogren's syndrome, and glaucoma. Therefore, the pharmaceutical composition comprising the fractional extract of melissa leaf as effective component for use in the prevention or treatment of angiogenesis-related diseases or MMP (Matrix metalloproteinase)-mediated diseases of the present invention can be useful for preventing or treating obesity, age-related macular degeneration, psoriasis, endometriosis, cancer growth or cancer metastasis, arteriosclerosis, arthritis, inflammatory bowel disease, Alzheimer's disease, periodontal disease, diabetic retinopathy, Sjogren's syndrome, or glaucoma, which has significantly fewer side effects and exhibits excellent pharmacological effects.

Specifically, the pharmaceutical composition according to the present invention, comprising the fractional extract of melissa leaf as effective component for use in the prevention or treatment of angiogenesis-related diseases or matrix metalloproteinase (MMP)-mediated diseases is effective in preventing or treating obesity by exhibiting significant suppression of weight, blood triglycerides, blood sugar, and total cholesterol.

The pharmaceutical composition according to the present invention, comprising the fractional extract of melissa leaf as effective component for use in the prevention or treatment of angiogenesis-related diseases or matrix metalloproteinase (MMP)-mediated diseases is effective in preventing or treating age-related macular degeneration by suppressing the size of macular degeneration lesions.

The pharmaceutical composition according to the present invention, comprising the fractional extract of melissa leaf as effective component for use in the prevention or treatment of angiogenesis-related diseases or matrix metalloproteinase (MMP)-mediated diseases is effective in preventing or treating psoriasis by suppressing significant epidermal thickness, inflammatory cell infiltration of ear tissue, and mRNA expression level in ear tissue.

The pharmaceutical composition according to the present invention, comprising the fractional extract of melissa leaf as effective component for use in the prevention or treatment of angiogenesis-related diseases or matrix metalloproteinase (MMP)-mediated diseases is effective in preventing or treating endometriosis by reducing the size of the uterine lesions significantly.

The pharmaceutical composition according to the present invention, comprising the fractional extract of melissa leaf as effective component for use in the prevention or treatment of angiogenesis-related diseases or matrix metalloproteinase (MMP)-mediated diseases is effective in preventing or treating cancer by reducing the size of cancerous tumors significantly.

The pharmaceutical composition according to the present invention, comprising the fractional extract of melissa leaf as effective component for use in the prevention or treatment of angiogenesis-related diseases or matrix metalloproteinase (MMP)-mediated diseases is effective in preventing or treating arteriosclerosis by inhibiting the increase in atherosclerotic plaques significantly

The pharmaceutical composition according to the present invention, comprising the fractional extract of melissa leaf as effective component for use in the prevention or treatment of angiogenesis-related diseases or matrix metalloproteinase (MMP)-mediated diseases is effective in preventing or treating arthritis by exhibiting excellent arthritis treatment effect and excellent weight-bearing effect.

The pharmaceutical composition according to the present invention, comprising the fractional extract of melissa leaf as effective component for use in the prevention or treatment of angiogenesis-related diseases or matrix metalloproteinase (MMP)-mediated diseases is effective in preventing or treating inflammatory bowel disease by showing a low disease activity index.

The pharmaceutical composition according to the present invention, comprising the fractional extract of melissa leaf as effective component for use in the prevention or treatment of angiogenesis-related diseases or matrix metalloproteinase (MMP)-mediated diseases is effective in preventing or treating Alzheimer's by improving cognitive function.

The pharmaceutical composition according to the present invention, comprising the fractional extract of melissa leaf as effective component for use in the prevention or treatment of angiogenesis-related diseases or matrix metalloproteinase (MMP)-mediated diseases is effective in preventing or treating periodontal disease by effectively regenerating gum tissues due to increased expression of pro-collagen.

The pharmaceutical composition according to the present invention, comprising the fractional extract of melissa leaf as effective component for the prevention or treatment of angiogenesis-related diseases or matrix metalloproteinase (MMP)-mediated diseases is effective in preventing or treating diabetic retinopathy by excellent inhibitory effect on retinal vascular leakage.

The pharmaceutical composition according to the present invention, comprising the fractional extract of melissa leaf as effective component for use in the prevention or treatment of angiogenesis-related diseases or matrix metalloproteinase (MMP)-mediated diseases is effective in preventing or treating Sjogren's syndrome by increasing the amount of salivary and tear secretion.

The pharmaceutical composition according to the present invention, comprising the fractional extract of melissa leaf as effective component for use in the prevention or treatment of angiogenesis-related diseases or matrix metalloproteinase (MMP)-mediated diseases is effective in preventing or treating glaucoma by remarkably reducing the elevated intraocular pressure.

**[Description of Drawings]**

Figure 1 is the result of Picrosirius red staining to detect fibrosis.

Figure 2 is a graph showing SMA deposition in the liver.

Figure 3 is a graph showing the protein expression level of Col1A2, which is known as a protein associated with liver fibrosis.

Figure 4 is a graph showing the protein expression level of TGF beta, which is known as a protein associated with liver fibrosis.

Figure 5 is a graph showing the protein expression level of IL-6, which is known as a protein associated with liver fibrosis.

Figure 6 is a graph showing the protein expression level of IL-10, which is known as embodiment a protein associated with liver fibrosis.

Figure 7 is a graph showing the serum concentrations of AST and ALT.

Figure 8 is a graph showing the quantification of the degree of fat accumulation in the liver.

Figure 9 shows that single ingredients of rosmarinic acid, caffeic acid, RME and EDPA have angiogenesis inhibitory effects, showing the strong angiogenesis inhibitory effect of EDPA at the same concentration.

Figure 10 confirms that, among compositions comprising EDPA, the angiogenesis inhibitory effect significantly increases as the content of EDPA increases.

Figure 11 is a graph showing the change in body weight of high-fat diet-induced obese rats.

Figure 12 is a graph showing the change in food intake in high-fat diet-induced obese rats.

Figure 13 is a graph showing the change in the concentration of triglycerides in the blood of high-fat diet-induced obese rats.

in the blood of high-fat diet-induced obese rats.

Figure 14 is a graph showing the change in the concentration of blood sugar in high-fat diet-induced obese rats.

Figure 15 is a graph showing changes in blood total cholesterol concentration in high-fat diet-induced obese rats.

Figure 16 is a graph showing the size of a choroidal neovascularization lesion in a laser-induced CNV model.

Figure 17 is a graph showing the thickness of the epidermis of the excised ear tissue.

Figure 18 is a graph confirming the volume of uterine lesions in the experimental model in which the fractional extract of melissa leaf of the embodiment was administered to the treatment group and was not administered to the control group.

Figure 19 is a graph showing the growth curve of tumor size in nude mice injected with DLD1 cells.

Figure 20 is a graph showing the results of measuring and evaluating the formation of atherosclerotic plaques in the aorta in an ApoE-deficient mouse model.

Figure 21 is a graph comparing the treatment effect on arthritis in a collagen-induced arthritis (CIA) model.

Figure 22 is a graph measuring the weight bearing rate in an osteoarthritis model induced by MIA.

Figure 23 is a graph measuring the disease activity index (DAI) in an animal model of inflammatory bowel disease induced by dextran sodium sulfate (DSS).

Figure 24 is a graph showing reference memory during a water maze test when the fractional extract of melissa leaf according to the embodiment was administered.

Figure 25 is a graph showing the improvement rate of the probe test during the underwater maze test when the fractional extract of melissa leaf according to the embodiment was administered.

Figure 26 is a graph showing the RT-PCR result of analyzing the gene expression level of pro-collagen indicating tissue regeneration in a periodontal disease model induced by ligation.

Figure 27 is a graph showing the results of testing the retinal blood vessel leakage effect in a rat model of diabetic retinopathy induced by streptozotocin.

Figure 28 is a graph analyzing the amount of tear secretion using NOD/ShiLt which is an animal model of Sjogren's syndrome.

Figure 29 is a graph showing the effect on lowering intraocular pressure in a hypertonic saline-induced scar glaucoma model.

**[Best Mode for Carrying out the Invention ]**

The terms used in the embodiments have been selected as currently widely used general terms as possible while considering the functions in the present invention, but these may vary depending on the intention or precedent of a person skilled in the art, the emergence of new technology, and the like. In addition, in a specific case, there is a term arbitrarily selected by the applicant, and in this case, the meaning will be described in detail in the description of the corresponding invention. Therefore, the term used in the present invention should be defined based on the meaning of the term and the overall content of the present invention, rather than the name of a simple term.

Unless otherwise defined, all terms used herein including technical or scientific terms have the same meaning as commonly understood by one of ordinary skill in the art to which the present invention pertains. Terms such as those defined in a commonly used dictionary should be interpreted as having a meaning consistent with the meaning in the context of the related art, and should not be interpreted in an ideal or excessively formal meaning unless explicitly defined in the present application.

Numerical ranges are inclusive of the values defined in that range. Every maximum numerical limitation given throughout the embodiments includes all lower numerical limitations as if the lower numerical limitation was expressly written. Every minimum numerical limitation given throughout the embodiments includes all higher numerical limitations as if the higher numerical limitation was expressly written. Any numerical limitation given throughout the embodiments shall include all numerical ranges within the broader numerical range, as if the narrower numerical limitation was expressly written.

Examples and manufacturing examples are presented to help the understanding of the present invention. The following examples and manufacturing examples are only provided for easier understanding of the present invention, and the content of the present invention is not limited by the examples and manufacturing examples.

**Examples**

**Example 1. Preparation of a fractional extract of melissa leaf**

400.4 kg of dried melissa leaves (origin: Europe) were extracted using 4000 L of 75% (v/v) ethanol under reflux at 81°C for 4 hours, and after 35 minutes of reflux extraction filtered with a 10μm of cartridge filter for 55 minutes to obtain 3100 L of the first extract.

The melissa leaf residue was extracted under reflux at 82°C for 4 hours using 4000 L of 75 (v/v) % ethanol, and after 35 minutes of reflux extraction filtered with a 10μm cartridge filter for 55 to 65 minutes to obtain 4100 L of the second extract.

After mixing the first and second extracts (a total of 7200 L of extract), concentration was performed to obtain 600 L of the first concentrate.

<The first concentration conditions>

Temperature: 56~58 °C
Pressure: -0.066 to -0.070 MPa
Time: 9 hours
600 L of the first concentrate was concentrated to obtain 250 L of the second concentrate.

<The second concentration conditions>

Temperature: 56~58 °C
Pressure: -0.063 to -0.065 MPa
Time: 5 hours and 50 minutes

250 L of the second concentrate and 200 L of purified water were mixed to obtain a suspension (total 450 L), and 450 L of ethyl acetate were added to the suspension to obtain 900 L of a mixture.

900 L of the mixture were stirred for 1 hour, and then left for 2 hours to separate the ethyl acetate layer (450 L) to obtain the first fraction.

Then, 450 L of ethyl acetate (ratio of mixture and ethyl acetate (v/v) = 1: 1) were added to the remaining suspension, followed by stirring for 1 hour, and then standing for 2 hours to separate the ethyl acetate layer (450 L) to obtain the second fraction.

After combining the first fraction and the second fraction (900 L), concentration was performed thereon to obtain 41 L (42.5 kg) of the third concentrate.

<The third concentration conditions>

Temperature: 58 °C
Pressure: -0.064 MPa
Time: 3 hours and 40 minutes

The third concentrate was collected in a stainless collector for 20 minutes (42.5 kg).

Thereafter, after complete drying at 80 ~ 82°C for 36 hours using a hot air dryer, the dried material (19.1 kg, 44.94% (w/w) of the concentrate) was put into a grinder and pulverized for 3 hours and 50 minutes to obtain a fractional extract of melissa leaf (yield: 18.9 kg) which is an ethyl acetate fraction of ethanol extract of melissa leaves.

### Example 2. Ingredients of a fractional extract of melissa leaf

(1) Analytical method

The active ingredients of a fractional extract of melissa leaf were analyzed using High Performance Liquid Chromatography (HPLC) at the wavelength of 285 nm. The fractional extract of melissa leaf prepared above was injected into a Zorbax Eclipse Plus C18 (150 X 4.6 mm) column and mobile phase A (5% aqueous formic acid solution) and mobile phase B (methanol) were delivered at a flow rate of 1 ml per minute according to the ratio shown in Table 1 below

[Table 1]

| Time (min) | Mobile Phase A | Mobile Phase B |
|---|---|---|
| 0 | 100 | 0 |
| 5 | 100 | 0 |
| 25 | 0 | 100 |
| 35 | 0 | 100 |
| 35.1 | 100 | 0 |
| 45 | 100 | 0 |

(2) Result

The fractional extract of melissa leaf obtained according to Example 1 comprised caffeic acid, EDPA, RME and rosmarinic acid according to the analytical method above and their contents were as follows.

Caffeic acid: 0.96 wt%
EDPA: 0.71 wt%
RME: 0.11 wt%
Rosmarinic acid: 16.1 wt%

In addition, rutin was not shown in the HPLC results of the fractional extract of melissa leaf obtained according to Example 1. Therefore, it was found that the fractional extract of melissa leaf obtained according to Example 1 did not comprise rutin.

**Experimental examples**

**Experimental Example 1. Inhibitory effect of the fractional extract of melissa leaf on non-alcoholic steatohepatitis in methionine/choline deficiency high-fat diet model**

(1) Experimental method

The therapeutic effect of the fractional extract of melissa leaf according to the Example on non-alcoholic steatohepatitis was evaluated in a methionine/choline deficient high-fat diet animal model.

The study was conducted using C57Bl/6 mice fed methionine/choline deficiency high-fat diet (MCD-HFD), which were divided into the treatment group administered with the fractional extract of melissa leaf according to the Example, and the control group administered with 0.5% CMC.

For the administration method, while feeding the MCD-HFD, the fractional extract of melissa leaf according to the Example was orally administered once a day at a dose of 200 mg/kg, and to the control group 0.5% CMC was administered at the same time. The administration period was for 9 weeks. In the normal group, non-alcoholic steatohepatitis was not induced by feeding the normal diet.

To confirm the degree of fibrosis in the liver, Picrosirius red staining was performed on liver tissue. For picrosirius red staining, liver tissue samples fixed in 10% formalin were embedded in paraffin, cut into 5 $\mu$m sections, and the tissue sections were incubated in 0.5% thiosemicarbazide for 10 minutes. Then, it was stained with 0.1% Sirius Red F3B in saturated picric acid for 1 hour and then washed with 0.5% acetic acid solution. To observe myofibroblasts which are the marker of liver fibrosis, immunohistochemical tests were performed using an alpha-smooth muscle actin (alpha-SMA) antibody. Paraffin-embedded liver tissue was cut into 4 $\mu$m sections, attached to a slide glass treated with poly-L-lysine, deparaffinized, and sequentially immersed in ethanol. After 10 minutes of treatment in 3% $H_2O_2$/methanol solution to remove endogenous peroxidase, it was reacted with normal goat serum in a moisture chamber for 40 minutes to eliminate non-specific reactions, and then washed with 0.01M phosphate-buffered saline solution (PBS, pH 7.4) containing 0.05% non-fat dry milk and 0.3% Triton X-100. The primary antibody was reacted for 1 hour, respectively, and the secondary antibody was reacted for 60 minutes, followed by color development. Western blot was performed to confirm the protein expression levels of Col1A2, TGF beta, IL-6 and IL-10. Specifically, liver tissue was dissolved in a buffer containing a protease inhibitor cocktail, and then the protein was extracted. After the extracted protein was quantified using a BCA assay kit, it was mixed with SDS gel loading buffer, and boiled at 100°C for 5 minutes for denaturation. Proteins electrophoresed on SDS-PAGE gel were transferred to a nitrocellulose membrane, and then the membrane was left in 5% skim milk for 1 hour to block non-specific protein binding. A general immunoblot was performed by treating antibodies of ColiA2, TGF beta, IL-6, IL-10 and beta-actin as primary antibodies, and then the intensity of the band finally appeared was measured with gel documentation software and graphed.

(2) Results

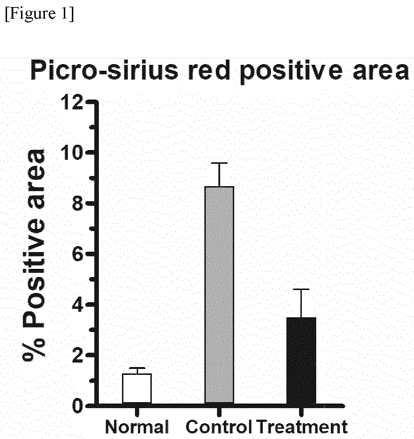

Figure 1 is a result of Picrosirius red staining to detect fibrosis. Referring to FIG. 1, fibrosis induced by methionine/choline-deficient high-fat diet in non-alcoholic steatohepatitis animal model was inhibited in the treatment group to which the fractional extract of melissa leaf according to the Example was orally administered.

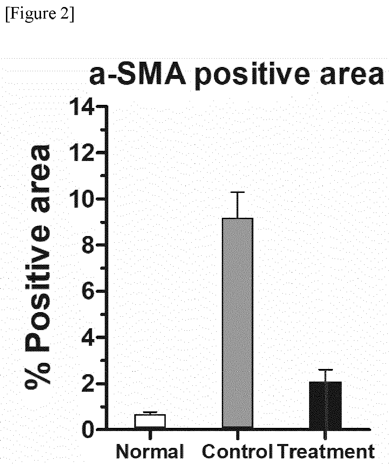

Figure 2 is a graph showing SMA expression in the liver. Referring to FIG. 2 , fibrosis induced by methionine/choline-deficient high-fat diet in non-alcoholic steatohepatitis animal model was inhibited in the treatment group to which the fractional extract of melissa leaf according to the Example was orally administered.

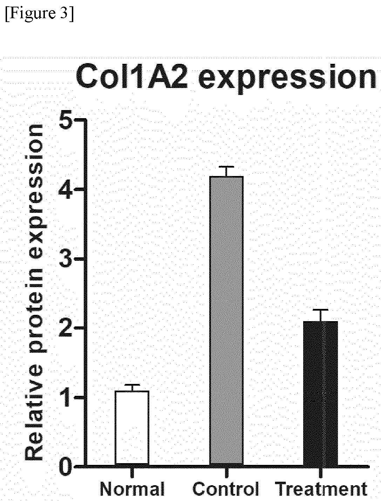

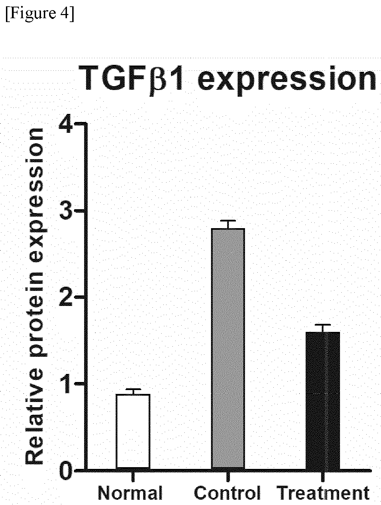

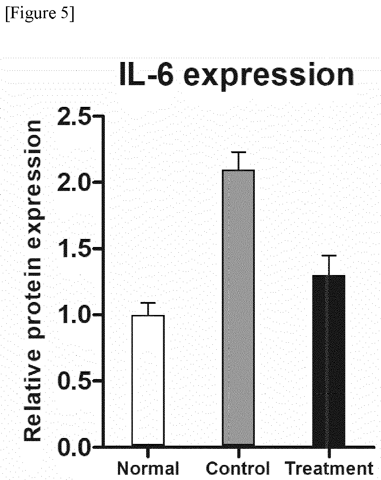

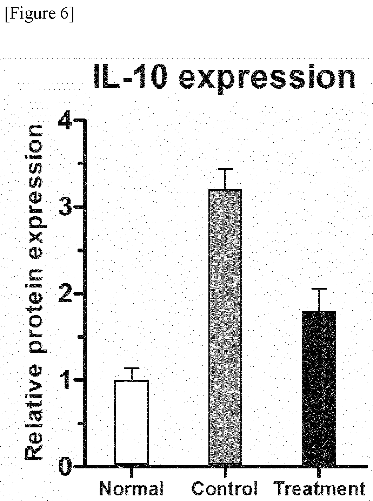

Figure 3 to 6 are graphs showing protein expression levels of Col1A2, TGF beta, IL-6 and IL-10, respectively which are known proteins associated with liver fibrosis. Referring to FIG. 3 to 6, it was found that all of these proteins increased in the control group, and the increased expression levels of these proteins were inhibited by treatment with the fractional extract of melissa leaf according to Example in the treatment group.

**Experimental Example 2. Inhibitory effect of a fractional extract of melissa leaf on non-alcoholic fatty liver disease in high-fat diet model**

(1) Experimental method

As experimental animals, thirty SD rats were randomly divided into three groups of ten rats each after acclimatization for one week.

For each of the three groups, a group fed a standard chow diet (purchased from Saeron Bio) was divided into the normal group, a group fed a high-fat diet (HFD) and administered with the fractional extract of melissa leaf of the Example was divided into the treatment group, and a group fed a high-fat diet (HFD) and administered with 0.5% CMC was divided into the control group.

The normal group was fed a standard chow diet. The treatment group was fed a HFD instead of a standard chow diet, and while fed a HFD the fractional extract of melissa leaf according to the Example was orally administered once a day at a dose of 100 mg/kg. The same experiment was performed in the control group as the treatment group except that 0.5% CMC was administered instead of the fractional extract of melissa leaf of the Example. The administration period was 10 weeks.

To measure the serum levels of AST and ALT of the normal group, the treatment group, and the control group, respectively blood samples were taken from the abdominal aorta by autopsy of the rats. The collected blood samples were centrifuged to obtain serum. Serum levels of AST and ALT were measured using an automatic biochemical analyzer.

Three rats were randomly selected from each of the normal group, the treatment group, and the control group to determine the degree of fat accumulation in the liver tissues of each of the normal group, the treatment group, and the control group. After preparing 4 slides stained with Oil red on the frozen liver tissue for each individual of the selected rats, the degree of fat accumulation in the liver tissue was quantified using an analysis program, ImageJ.

(2) Results

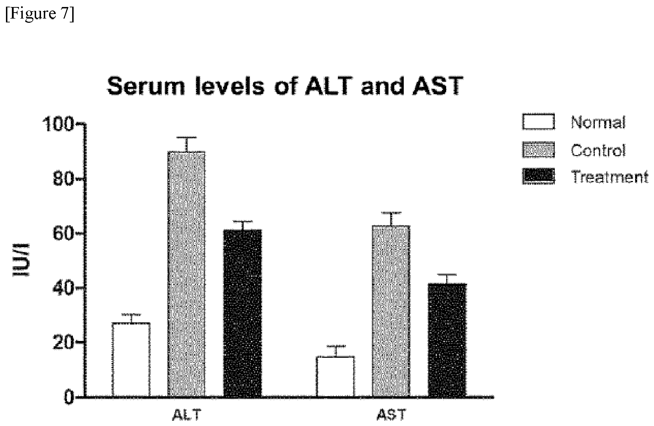

Figure 7 is the measured value (unit: IU/L) of serum levels of AST and ALT for the normal group, the treatment group, and the control group. Referring to FIG. 7, it was confirmed fatty liver did not occur in the normal group as the normal group showed low serum levels of AST and ALT. The treatment group administered with the fractional extract of melissa leaf according to the Example showed lower serum levels of AST and ALT than the control group confirming that fatty liver was inhibited compared with the control group.

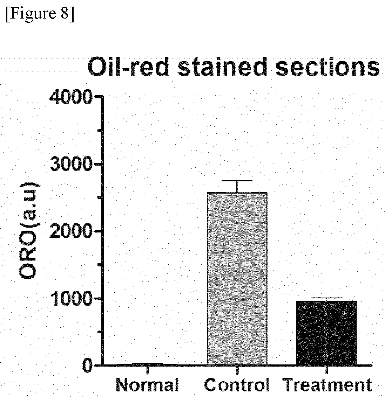

Figure 8 is a graph showing the quantification of the degree of fat accumulation for each of the treatment group and the control group using an analysis program, ImageJ. Referring to FIG. 8, it was confirmed that the degree of fat accumulation in the liver tissue was insignificant in the normal group. It was confirmed that the treatment group administered with the fractional extract of melissa leaf according to the Example suppressed the degree of fat accumulation in the liver tissue compared with the control group administered with CMC.

**Experimental Example 3. Inhibitory effect on angiogenesis**

To confirm the inhibitory effect of the fractional extract of melissa leaf according to the Example on angiogenesis, an experiment was conducted using the following method.

(1) Experimental method

A 48-well plate was coated with 200 μl of 10% matrigel, and incubated at 4°C. After 4 hours the temperature was raised to 37°C, and 1 hour later, the test substances listed in Tables 2 and 3 below were added to a final concentration of 50 μg/ml, and then 5 X $10^4$ HUVECs (human umbilical vein endothelial cells) were added to each well, and pictures were taken the next day.

[Table 2]

| | Sample |
|---|---|
| 1 | DMSO |
| 2 | RME |
| 3 | Caffeic acid |
| 4 | Rosmarinic acid |
| 5 | EDPA |

[Table 3]

| | Sample | Content of EDPA |
|---|---|---|
| 1 | ALS-T20003 | 0.85% |
| 2 | ALS-T20006 | 0.66% |
| 3 | ALS-T20009 | 0.45% |

(2) Results

**1) Effect of single ingredient (Rosmarinic acid, caffeic acid, RME (Rosmarinic acid methyl ester) and EDPA (Ethyl 2-(3,4-dihydrophenyl acetate)) on HUVEC tube formation, an angiogenesis inhibitory activity assay**

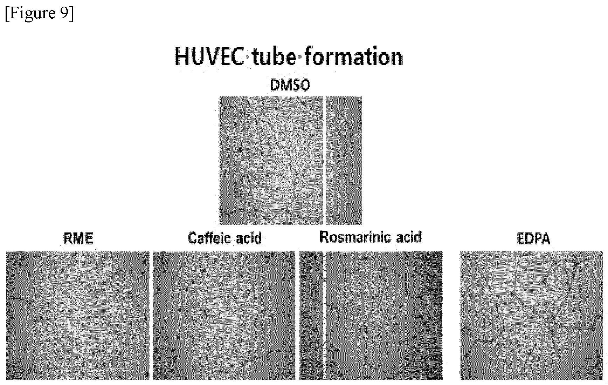

As shown in Figure 9, Rosmarinic acid, caffeic acid, RME and EDPA which are single ingredient comprised in the fractional extract of melissa leaf had an angiogenesis inhibitory activities in HUVEC tube formation assay and EDPA showed the highest angiogenesis inhibitory activity at the same concentration by inhibiting tube formation the most.

**2) Angiogenesis inhibitory activity of a composition comprising EDPA**

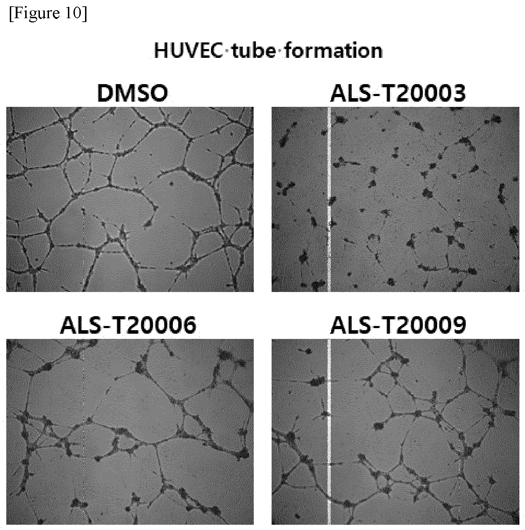

As shown in Figure 10 and Table 4, when angiogenesis inhibitory activities were measured with fractional extracts of melissa leaf having different EDPA contents (referring to the contents of Table 3), it was confirmed that the higher the EDPA content, the more angiogenesis inhibitory activity increased significantly.

[Table 4]

| No. | Sample | Concentration ($\mu$g/ml) | Inhibition of Tube Formation (%) |
|---|---|---|---|
| 1 | DMSO | | 0 |
| 2 | ALS-T20003 | 50 | 90.6 |
| 3 | ALS-T20006 | 50 | 28.7 |
| 4 | ALS-T20009 | 50 | 14.1 |

**Experimental Example 4. MMP inhibitory activity**

An experiment was conducted to confirm the MMP (Matrix metalloproteinase) inhibitory activity of the fractional extract of melissa leaf according to the Example with the following method.

(1) Experimental method

The MMP inhibitory activities were measured using a spectrofluorometer (PerkinElmer LS50B) to determine whether the fractional extract of melissa leaf has an inhibitory effect on MMPs. MMP-2 and MMP-9 were activated with 1 mM APMA (p-aminophenyl mercuric acetate) before use for activity measurement. As substrates for MMP-2 and MMP-9, fluorescent substrate for MMP-2/MMP-9 (Calbiochem) was used. As a control, 2 ml of a buffer solution [50 mM Tricine (pH 7.5), 10 mM CaCl$_2$, 200 mM NaCl] containing 1 uM of the substrate was added to a 2 ml cuvette, and MMP-2, or -9 was added thereto. Fluorescence intensity was measured by a spectrofluorometer at 2-minute intervals for 20 minutes at room temperature using a 328 nm excitation wavelength and a 400 nm emission wavelength. In the case of the treatment group, each test substance listed in Table 3 was added to the buffer solution containing the substrate and MMP in the same manner as the control group to a final concentration of 20 ug/ml, and then fluorescence intensity was measured at 2-minute intervals for 20 minutes at room temperature.

(2) Results

[Table 5]

| Concentration (20 $\mu$g/ml) | MMP-2 inhibition (%) | MMP-9 inhibition (%) |
|---|---|---|
| ALS-T20003 | 78 | 89 |
| ALS-T20006 | 54 | 62 |
| ALS-T20009 | 48 | 53 |

Referring to Table 5, it was confirmed that the inhibitory activities of MMP-2 and MMP-9 significantly increased as the content of EDPA increased among the compositions containing EDPA.

## Experimental Example 5. Anti-obesity effect in high-fat diet-induced obese rats

### (1) Experimental method

The anti-obesity effect of the fractional extract of melissa leaf according to the Example was evaluated in a high-fat diet-induced obese animal model.

Obesity was induced by feeding a high-fat diet (Saeron Bio Co., Ltd.) to a male Sprague-Dawley (SD) rat (JoongAng Experimental Animals Co., Ltd.), and the study was conducted by dividing into the treatment group administered with the fractional extract of melissa leaf according to the Example, and the control group administered with 0.5% carboxymethyl cellulose (CMC).

The control group and the treatment group were acclimatized to the environment with standard diet (Saeron Bio Co., Ltd.) for 1 week before feeding with an experimental high-fat diet, and then fed with an experimental diet for 10 weeks.

For the administration method, the fractional extract of melissa leaf according to the Example was orally administered once a day at a dose of 100 mg/kg, and 0.5% CMC was administered to the control group. Body weight and food intake were measured twice a week on the same day. For the measurement of food intake, a certain amount of food was filled in a powder feed box at 3 pm and the total weight is measured, and the weight of the reduced food was measured at 3 pm the next day.

This was calculated as the amount of food consumed for 24 hours, and was carried out constantly until the end of the test. Blood samples were collected at 5 and 10 weeks from the test start date. Blood was collected after anesthesia with ether by ocular bleed method in which a heparin-treated glass capillary tube was inserted into the orbital venous plexus of SD rats. Total cholesterol, triglyceride and insulin concentration were measured from the collected blood.

### (2) Results

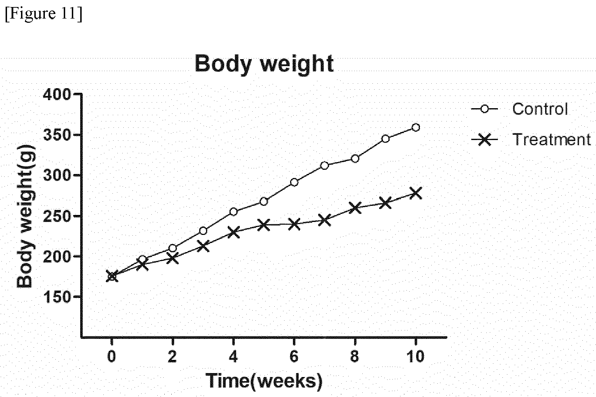

Figure 11 is a graph showing the change in body weight of high-fat diet-induced obese rats. Referring to FIG. 11, continuous weight gain was observed in the control group, and this weight gain continued until the 10th week of the end of the experiment. But in the treatment group the body weight was significantly reduced in high-fat diet-induced obese rats.

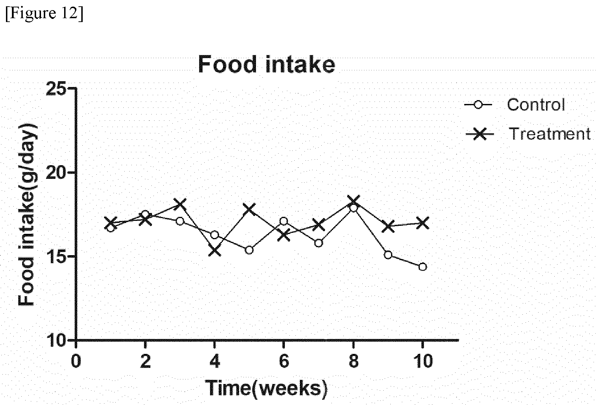

Figure 12 is a graph showing changes in food intake in high-fat diet-induced obese rats. Referring to Fig. 12, no significant change in food intake was observed in the control group and the treatment group.

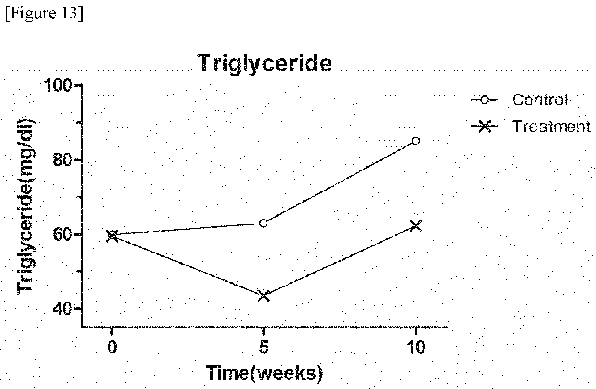

Figure 13 is a graph showing changes in triglyceride concentrations in the blood of high-fat diet-induced obese rats. Referring to FIG. 13, in the treatment group blood triglycerides were significantly reduced in high-fat diet-induced obese rats. In particular, the levels of triglycerides in the blood did not increase in the experimental group even after 10 weeks of ingestion of a high-fat diet.

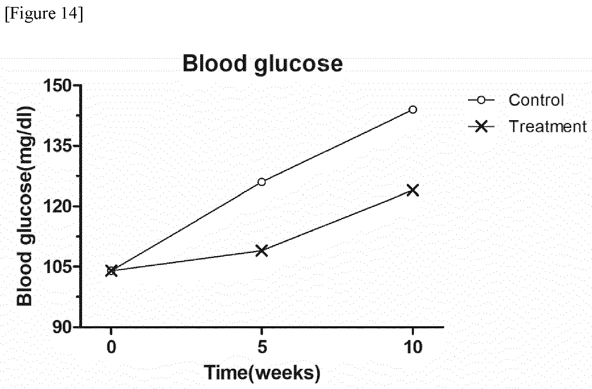

Figure 14 is a graph showing changes in the concentration of blood glucose in high-fat diet-induced obese rats. Referring to FIG. 14, in the treatment group blood glucose was significantly reduced in high-fat diet-induced obese rats.

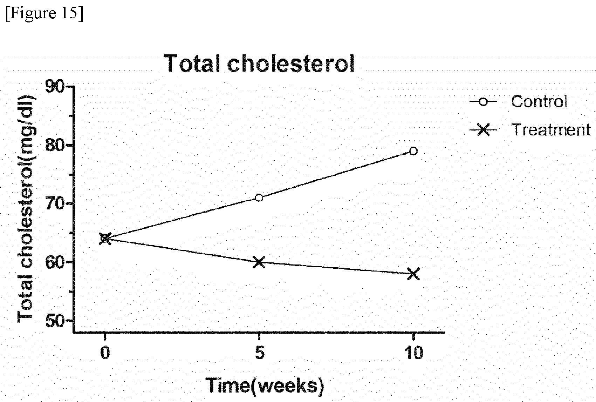

Figure 15 is a graph showing changes in blood concentration of total cholesterol in high-fat diet-induced obese rats. Referring to FIG. 15, in the treatment group total cholesterol was significantly reduced in high-fat diet-induced obese rats.

## Experimental Example 6. Inhibitory effect of the fractional extract of melissa leaf on age-related macular degeneration in laser-induced choroidal neovascularization (CNV) model

### (1) Experimental method

The treatment effect of the fractional extract of melissa leaf according to Example on age-related macular degeneration was evaluated in a laser-induced choroidal neovascularization (CNV) animal model.

A laser-induced CNV model was prepared with 6-week-old C57BL/6 mice by damaging the retinal optic nerve area using a diode green laser (532 nm, 150 mW, 0.1 sec).

In the laser-induced CNV model, the study was conducted by dividing into the treatment group administered with the fractional extract of melissa leaf according to the Example, and the control group administered with 0.5% CMC.

The fractional extract of melissa leaf according to the Example was orally administered once a day at a dose of 100 mg/kg two days before laser treatment, and 0.5% CMC was administered to the control group. The dosing period continued for 10 days after laser treatment.

Ten days after laser treatment, the retina-choroid flat mount was performed to confirm the effect of reducing the size of CNV lesions. Mice were anesthetized and retro-orbital injection of 25 mg/ml of FITC-dextran was performed. After 30 minutes, the mice were euthanized, the eyes were removed, fixed with 10% formalin, and the cornea and lens were removed and flat mounted on a cover glass. The size of the macular degeneration lesion stained with FITC-dextran was measured.

(2) Results

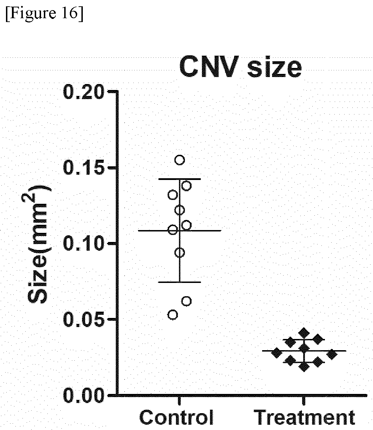

Figure 16 is a graph showing the size of a macular degeneration lesion in a laser-induced CNV model. Referring to FIG. 16, it was shown that the size of the macular degeneration lesion by laser treatment increased in the control group, and the size of the neovascularization was significantly reduced in the treatment group in the laser-induced choroidal neovascularization model.

## Experimental Example 7. Inhibitory effect of the fractional extract of melissa leaf on psoriasis in IL-23 induced psoriasis animal model

(1) Experimental method

The effect of the fractional extract of melissa leaf according to the Example on psoriasis was evaluated in an IL-23 induced psoriasis animal model according to Ma's method [J. Clin. Cell Immunol. 4:6.(2013)].

An IL-23 induced psoriasis animal model was prepared in which psoriasis was induced by intradermal injection of 500 ng of IL-23 to the ears of C57BL/6 mice (6 weeks old, Orient Bio) every other day for 16 days.

The study was conducted in the IL-23 induced psoriasis animal model by dividing into the treatment group administered with the fractional extract of melissa leaf according to the Example, and the control group administered with 0.5% CMC, and the PBS control group treated with PBS instead of IL-23

For the administration method, the fractional extract of melissa leaf according to the Example was orally administered once a day at a dose of 100 mg/kg, and 0.5% CMC was administered to the control group. The oral administration started from one day before the IL-23 injection and continued for a total of 16 days, and the experiment was ended on the 17th day after the start of administration.

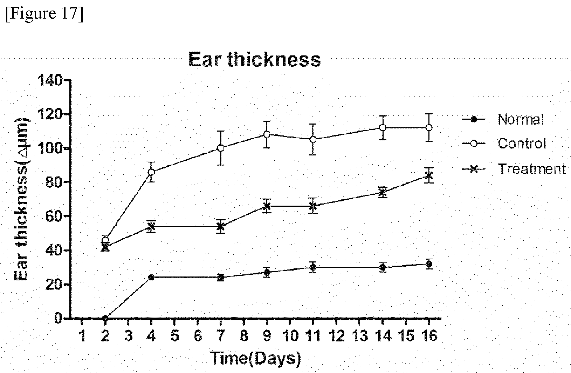

After the experiment was finished, the ear thickness of the mice was measured, and the results were shown in FIG. 17.

(2) Results

Figure 17 is a graph showing the thickness of the epidermis of the excised ear tissue. Referring to FIG. 17, in the treatment group, the epidermal thickness was significantly reduced in an IL-23 induced psoriasis animal model.

## Experimental Example 8. Inhibitory effect of the fractional extract of melissa leaf on endometrial growth in endometrial autograft model

(1) Experimental method

After housing SD rats, in order to match the female estrus cycle, bedding soaked in male urine was placed in a female cage every 5 days, and vaginal cytology was performed daily for a week before surgery to confirm the menstrual cycle.

Before surgery, the surgical site was shaved with an electric razor and disinfected with 70% ethanol. After making an incision about 1 from 0.5 cm to 1.0 cm from the vaginal opening,
the left uterus was exposed. Both parts of the exposed left uterus at a position of 6 cm to 8 cm were tightly tied with 5-0 sutures. Cut the uterus between the tied positions and place it in a sterile glass Petri dish with 100 $\mu$L of PBS containing penicillin (100U/ml) and streptomycin (100$\mu$g/ml). After removing the fat, the uterus was incised vertically, and cut into three pieces of 2mm size.

After finding and pulling out the intestines from the incision site, the intestines were spread on pre-wetted gauze, where three arteries between the mesentery were identified and the uterine fragment was gently sutured. Then, the operated intestine was inserted into the abdominal cavity, the abdominal wall was sewn using a suture, and the skin was closed with an autoclip. After surgery, heat was applied to SD rats to prevent body temperature drop. The surgical site was disinfected with povidone, and acetaminophen, an analgesic, was mixed with drinking water at 2 mg/ml and administered.

The auto clip was removed 7 days after the operation, and the fractional extract of melissa leaf according to the Example was orally administered to the treatment group (n = 5) once a day for 4 weeks. The administered dose of the fractional extract of melissa leaf was 100 mg/kg. Group separation was performed so that the average size of uterine lesions in each group was the same.

0.5% CMC was orally administered to the control group (n = 5).

Four weeks after surgery, SD rats were euthanized and the peritoneum was incised. Then, endometriotic lesions transplanted to the mesentery were isolated and the size and weight were measured.

(2) Results

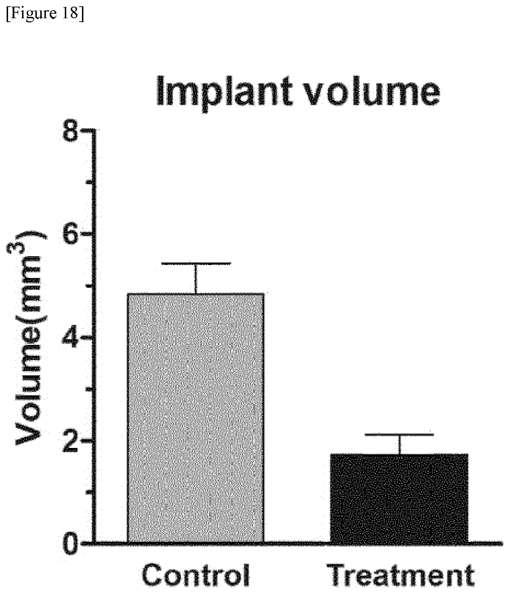

Figure 18 is a graph confirming the volume of endometriotic lesions transplanted to the mesentery in the experimental model wherein the fractional extract of melissa leaf of the Example was administered to the treatment group and was not administered to the control group. In Figure 18, the average volumes of the endometriotic lesions of the treatment group and the control group were described.

Table 6 showed the size and volume of endometriotic lesions transplanted into the mesentery of each of the treatment group and the control group.

[Table 6]

| | | Width (mm) | Length (mm) | Volume (mm$^3$) |
|---|---|---|---|---|
| Treatment group | SD rat 1 | 1.82 | 1.51 | 2.16 |
| | SD rat 2 | 1.75 | 1.53 | 2.13 |
| | SD rat 3 | 1.59 | 1.39 | 1.60 |
| | SD rat 4 | 1.63 | 1.28 | 1.39 |
| | SD rat 5 | 1.99 | 1.16 | 1.39 |
| Control group | SD rat 1 | 2.56 | 2.01 | 5.38 |
| | SD rat 2 | 2.39 | 2.06 | 5.27 |
| | SD rat 3 | 2.47 | 1.98 | 5.04 |
| | SD rat 4 | 2.27 | 1.82 | 3.91 |
| | SD rat 5 | 2.45 | 1.89 | 4.55 |

Referring to Table 6, the average volume of the endometriotic lesions transplanted into the mesentery of the treatment group was about 1.73 mm$^3$, and the standard deviation was about 0.38. The average volume of the endometriotic lesions of the control group was about 4.83 mm$^3$ and the standard deviation was about 0.61. Referring to Figure 18 and Table 6, it was confirmed that the volume of the endometriotic lesions of the treatment group was significantly smaller than that of the control group. As a result of this it could be confirmed that the fractional extract of melissa leaf according to the Example has an excellent inhibitory effect on endometrial tissue growth.

**Experimental Example 9. Inhibitory effect of the fractional extract of melissa leaf on cancer growth in tumorigenicity model in nude mice**

(1) Experimental Method

The anticancer effect of the fractional extract of melissa leaf according to the Example was evaluated in a tumorigenicity model in nude mice.

Colorectal cancer cells (DLD1) were cultured in RPMI-1640 medium, and 6-week-old male nude mice (Orient Bio) were housed in an aseptic facility. The cultured DLD1 cells were resuspended in DMEM medium, and $5 \times 10^6$ cells were injected subcutaneously into the right flank of nude mice, which were divided into the treatment group administered with the fractional extract of melissa leaf according to the Example, and the control group administered with 0.5% CMC.

For the administration method, the fractional extract of melissa leaf was orally administered once a day at a dose of 100 mg/kg from the day of tumor injection, and 0.5% CMC was administered to the control group. The administration period was 28 days, and the tumor size was measured every 3 days with a caliper.

(2) Results

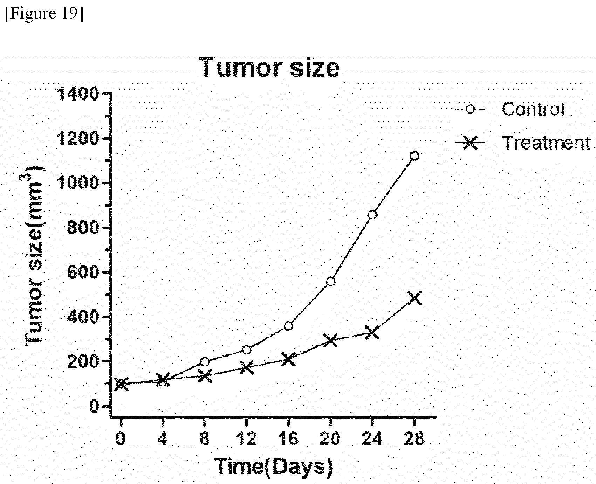

Figure 19 is a graph showing the growth curve of tumor size in nude mice injected with DLD1 cells. In the control rats, tumors grew exponentially until the 28th day, but in the treatment group to which the fractional extract of melissa leaf according to the Example was orally administered, the tumors significantly decreased in size, thereby exhibiting a strong anticancer effect.

**Experimental Example 10. Inhibitory effect of the fractional extract of melissa leaf on arteriosclerosis in Apolipoprotein E (ApoE)-deficient mouse model**

(1) <u>Experimental Method</u>

The anti-atherosclerotic effect of the fractional extract of melissa leaf according to the Example was evaluated in an ApoE-deficient mouse model. Mice in which atherosclerosis was induced by a high-fat diet for 16 weeks were selected, and those in which atherosclerosis was not induced by a standard diet instead of a high-fat diet were classified as normal group.

The control group was administered with 0.5% CMC, and the treatment group was administered with the fractional extract of melissa leaf according to the Example.

Mice were given ad libitum access to food and water for 16 weeks, and the fractional extract of melissa leaf according to the Example was orally administered once a day at a dose of 100 mg/kg to the treatment group of mice induced arteriosclerosis by a high-fat diet for 16 weeks, and 0.5 % CMC was administered to the control group.

After 16 weeks, mice were sacrificed, resection from the heart and ascending aorta to the thoracic aorta was performed and fixed in 10% neutral buffered formalin solution. After the fixed tissue is trimmed with a razor, it was embedded in a frozen tissue embedding agent (OCT compound) and frozen in a deep freezer. Slides were prepared by cutting aortic arch into 10 $\mu$m thick with a cryostat microtome. For fat staining, slides were immersed in distilled water and treated with absolute propylene glycol for 1 minute, stained in an oil-red solution for 16 hours, and then treated in 85% propylene glycol for 2 minutes. Slides were washed with distilled water, sealed with an aqueous encapsulant, and observed under an optical microscope.

(2) <u>Results</u>

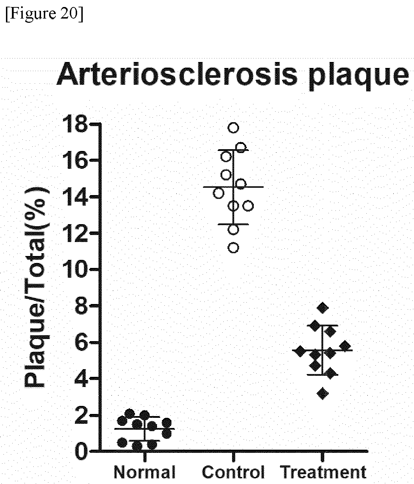

Figure 20 is a graph showing the results of measuring and evaluating the formation of atherosclerotic plaques in the aorta in an ApoE-deficient mouse model. It was confirmed that the formation of atherosclerotic plaques increased in the control group compared to the normal group, and the increase in atherosclerotic plaques was significantly inhibited in the treatment group.

**Experimental Example 11. Efficacy of the fractional extract of melissa leaf in collagen-induced arthritis animal model**

(1) <u>Experimental method</u>

The efficacy of the fractional extract of melissa leaf according to the Example was evaluated in a collagen-induced arthritis model.

The collagen-induced arthritis (CIA) animal model was created by inducing an immune response by injecting collagen, which is considered to be the cause of rheumatoid arthritis.

4 mg of chicken type II collagen was mixed with 1 ml of 100 mM acetic acid and dissolved at 4°C for one day. This was mixed with 1 ml of Freund's complete adjuvant containing 4 mg/ml of Mycobacterium tuberculosis, emulsified, and then 150 $\mu$l (300 $\mu$g) was intradermally injected into the tail of 6-week-old Lewis rats for immunization. On the 7th day after the primary immunization, a secondary immune (boosting) reaction was induced by intradermal injection of chicken type II collagen again. Arthritis symptoms were confirmed after 1-2 weeks after secondary immunization.

The control group was administered with 0.5% CMC, and the treatment group was administered with the fractional extract of melissa leaf according to the Example.

The fractional extract of melissa leaf according to the Example was orally administered once a day for 47 days from the next day after induction of the secondary immune response at a dose of 100 mg/kg, and 0.5% CMC was administered to the control group.

In order to verify the therapeutic effect, the degree of swelling and erythema of the joints and nodes of each rat's paws were observed, and clinical scores were calculated according to the scoring index described in Table 7 below.

[Table 7]

| Score | Clinical observations |
|---|---|
| 0 | no evidence of erythema and swelling |
| 1 | erythema and mild swelling confined to the tarsals or ankle joint |
| 2 | erythema and mild swelling extending from the ankle to the tarsals |
| 3 | erythema and moderate swelling extending from the ankle to the metatarsal joints |
| 4 | erythema and severe swelling encompassing the ankle, foot, and digits |

(2) Results

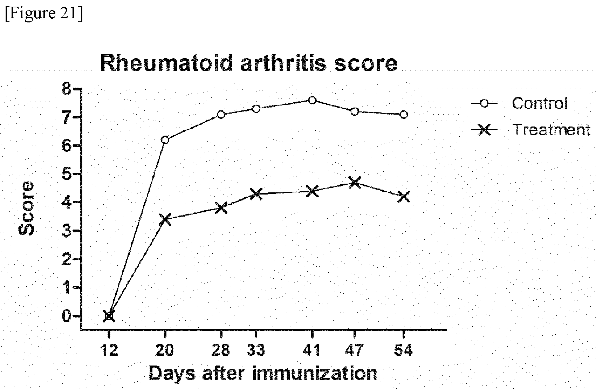

Figure 21 is a graph comparing the treatment efficacy of arthritis in a collagen-induced arthritis (CIA) model. Referring to FIG. 21, arthritis symptoms appeared from about 20 days after the CIA-induced immune response. The treatment group showed a significant therapeutic effect in a collagen-induced arthritis model.

**Experimental Example 12**. **Efficacy of the fractional extract of melissa leaf in MIA (Monosodium iodoacetate)-induced Osteoarthritis animal model**

(1) Experimental Method

The efficacy of the fractional extract of melissa leaf according to the Example was evaluated in a MIA-induced osteoarthritis model.

Osteoarthritis was induced by injection of 50 $\mu$l of MIA diluted to a concentration of 60 mg/ml with 0.9% saline into the joint cavity of right hind limb of 7-week-old SD rats and selected.

0.5% CMC was administered to the control group, and the fractional extract of melissa leaf according to the Example was administered to the treatment group.

For the administration method, the fractional extract of melissa leaf was orally administered once a day for 21 days at a dose of 100 mg/kg, and 0.5% CMC was administered to the control group. Weight bearing rate was measured at intervals of 7 days after administration. The group that did not induce osteoarthritis was classified as the normal group.

Hind paw weight bearing was measured using an Incapacitance tester. In the holder of the tester, the osteoarthritis-induced rat stood on the normal hind paw without MIA treatment due to pain, so the weight of both paws was out of balance, and the weight of the paw treated with MIA was relatively light compared to the weight of the normal paw. When measuring the weight of the paws, the weight (g) of both paws was measured in the state that the SD rat's belly did not touch the sensor of the device.

Using the measured weight of the paw, the weight-bearing rate (%) was calculated by Equation 1 below.

$$\text{Weight bearing rate (\%)} = [\text{weight of osteoarthritis-induced hind limb/(weight of hind limb of both paws)}] \times 100 \qquad \text{[Equation 1]}$$

(2) Results

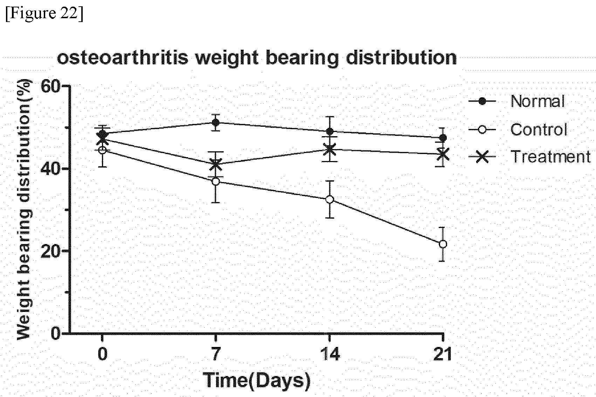

Figure 22 is a graph measuring the weight bearing rate in the osteoarthritis model induced by MIA. Referring to Fig. 22, it was confirmed that the weight-bearing rate (%) of the control group decreased statistically significantly compared with the normal group as the period elapsed. In contrast, the treatment group showed an excellent weight-bearing effect (therapeutic effect of osteoarthritis) by significantly increasing the reduced weight-bearing rate.

**Experimental Example 13. Inhibitory effect of the fractional extract of melissa leaf on inflammatory bowel disease induced by dextran sodium sulfate (DSS)**

(1) Experimental Method

The therapeutic effect of the fractional extract of melissa leaf according to the Example on ulcerative colitis was evaluated in an animal model induced by sodium dextran sulfate.

Seven-week-old C57BL/6J mice (Central Lab. Animal Inc.) were given ad libitum access to food and water and acclimatized for one week, and then randomly divided into the control group, the normal group, and the treatment group.

The fractional extract of melissa leaf according to the Example was orally administered to the treatment group once a day, at a dose of 200 mg/kg for 11 days after group separation and 0.5% CMC was administered to the control group. Inflammatory bowel disease was induced by adding 3% DSS in drinking water instead of water from the third day of group separation. On the other hand, in the normal group, inflammatory bowel disease was not induced because DSS was not administered.

During the experimental period, the appearance of stool was observed by inducing defecation of mice once a day, and the presence or absence of occult blood was observed using coulter hemoccult single slides, and the scores were indicated according to the criteria in Table 8 below.

[Table 8]

| Score | Stool consistency | Stool color |
|---|---|---|
| 0 | Normal | Normal colored stool |
| 1 | Soft but maintains morphology | Brown stool |
| 2 | Soft | Reddish stool |
| 3 | Very soft | Bloody stool |
| 4 | Diarrhea | Gross bleeding |

[Formula 2]

DAI (disease activity index) = stool concentration + stool color

(2) Results

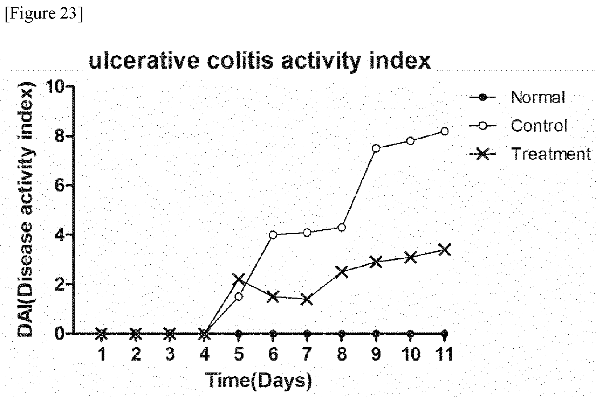

Figure 23 is a graph measuring the disease activity index (DAI) in an animal model of inflammatory bowel disease induced by DSS. At this time, the DAI of the normal group represents 0. As a result of measuring the DAI of the rats with ulcerative colitis induced by DSS, the control group showed high DAI, whereas the treatment group showed a remarkable therapeutic effect with low DAI score.

**Experimental Example 14. The efficacy of improving cognitive function in scopolamine-induced dementia animal model**

(1) Experimental Method

For cognitive function test, five 9-week-old male ICR mice were used in each group. The treatment group was administered with the fractional extract of melissa leaf, and the control group was administered with physiological saline for 2 weeks.

The treatment group was orally administered at a dose of 200 mg/kg seven times a week for two weeks and 60 minutes before the start of the test, scopolamine was orally administered to treatment group and control group, respectively at 1 mg/kg to induce memory impairment, and a water maze test was conducted.

The reference memory test during the water maze test was conducted after acclimatization by allowing them to swim freely in a water tank without a platform for 60 seconds one day before the start of the test. The reference memory test was performed by measuring the time of escape latency (unit: seconds) to find a platform submerged in water, 4 to 5 times a day for 5 days, and the maximum allowable time was limited to 60 seconds. If the location of the platform was not found until the second day of the test, mice were guided to find the platform within the time limit of 60 seconds and when they climbed on the platform, they were allowed to stay there for 10 seconds.

24 hours after the end of the reference memory test, a probe test was performed by removing the platform from the water tank and allowing them to swim freely for 60 seconds to measure the length of time they stayed at the location where the platform was.

The reference memory test was performed for 5 days after administration of the fractional extract of melissa leaf according to the Example, and the average of time to find the platform for the treatment group and the control group was compared.

(2) Results

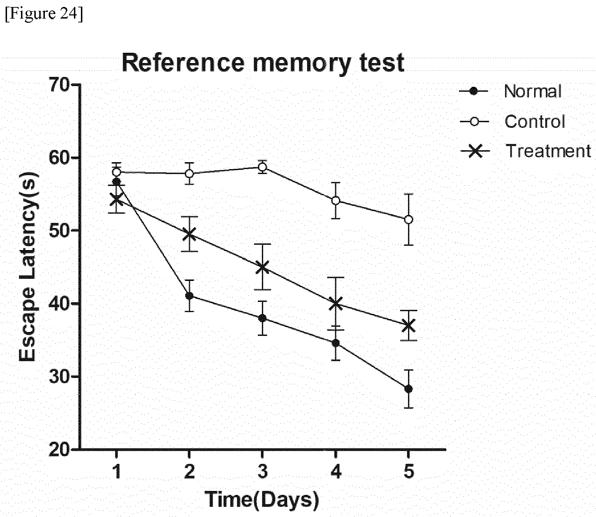

Referring to Figure 24, when the fractional extract of melissa leaf according to the Example was administered, the effect on improving cognitive function was confirmed ($37.2 \pm 2.1$).

In the probe test conducted after the reference memory test was completed, the average time of staying on the platform in the control group was compared with the average staying time of the treatment group administered with the fractional extract of melissa leaf according to the Example, and the improvement rate was expressed.

[Formula 3]

$$\text{Probe test improvement rate} = (tA - ts)/ts \times 100(\%)$$

The ts is the average time that the control group stayed at the platform, and tA is the average time the treatment group administered with the fractional extract of melissa leaf according to the Example stayed at the platform.

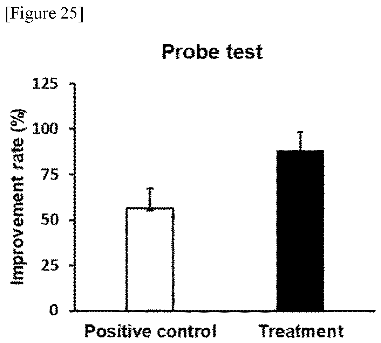

Referring to Fig. 25, also in the probe test the treatment group stayed on the platform for a longer time than the control group, and the treatment group (88.3±9.9%) showed an excellent improvement rate compared with the control group.

**Experimental Example 15. Inhibitory effect on periodontal disease in ligature-induced periodontal disease model**

(1) Experimental Method

7-week-old male SD rats were used as the experimental animals, which were given ad libitum access to general lab animal food and water and after acclimatization for one week they were used in the experiment.

Periodontal disease was induced by ligating the mandibular first molar with sterile sutures (3-0, nylon thread) after general anesthesia. After confirming only the cervical part, rats that were not ligated were classified as the normal group

Among the group with periodontal disease induced, the fractional extract of melissa leaf according to the Example was administered to the treatment group.

The treatment group was orally administered once a day at a dose of 100 mg/kg and the control group was administered with 0.5% CMC.

After sacrificing the experimental animals, the surrounding tissues of the excised mandible were removed, and then the gene expression level of procollagen indicating tissue regeneration was confirmed.

In periodontal disease, regeneration and recovery of periodontal tissue made of collagen is important. The gene expression level of procollagen from the gums was confirmed through RT-PCR.

cDNA was synthesized using RT-PCR (reverse transcriptase-polymerase chain reaction) from 10 $\mu$g of RNA obtained by pulverizing the extracted gum tissue with a homogenizer using the Trizol method. The nucleotide sequences of the PCR primers for each gene are shown in Table 9 below.

[Table 9]

| Gene | Primer sequences | |
| --- | --- | --- |
| | Forward primer | Reverse primer |
| GAPDH | ggc atg gac tgt ggt cat ga | ttc acc acc atg gag aag gc |
| Pro-collagen | tct act ggc gaa acc tgt atc cg | caa gga agg gca ggc gtg at |

(2) Results

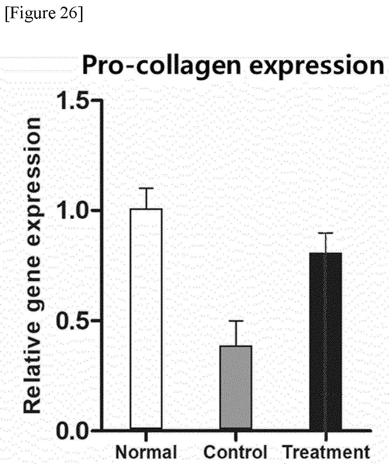

Figure 26 is a graph showing the RT-PCR result of analyzing the gene expression level of pro-collagen indicating tissue regeneration in a periodontal disease model induced by ligation. Referring to Fig. 26, the administration of the fractional extract of melissa leaf according to the Example increased the expression of pro-collagen to regenerate the gum tissue effectively, thereby showing a significant therapeutic effect on periodontal disease.

**Experimental Example 16. Inhibitory effect of the fractional extract of melissa leaf on diabetic retinopathy in streptozotocin (STZ)-induced diabetic retinopathy rat model**

(1) Experimental Method

The therapeutic effect of the fractional extract of melissa leaf according to the Example was evaluated in a streptozotocin-induced diabetic retinopathy rat model.

A streptozotocin solution (100 mM) dissolved in citrate buffer (100 mM, pH 4.5) was injected into the abdominal cavity of rats at 150 mg/kg, and 10% sucrose was sufficiently supplied to prevent hypoglycemic shock. After 2 days, blood glucose level was measured using a blood glucose meter, and a diabetic animal in which non-fasting blood glucose was maintained over 300 mg/dl within 1 to 2 weeks were used.

Among the diabetes-induced rats, 0.5% CMC was administered to the control group, and the fractional extract of melissa leaf according to the Example was administered to the treatment group, and diabetes was not induced in the normal group.

For the administration method, diabetes-induced rats were selected and the fractional extract of melissa leaf according to the Example was orally administered once a day at a dose of 100 mg/kg for 16 weeks from 2 weeks after streptozotocin administration, and 0.5% CMC was administered to the control group. After 16 weeks to analyze retinal vascular leakage quantitatively, 1.25 mg of 500-kDa FITC-dextran (Sigma-Aldrich) was injected into the left ventricle of a rat, and stained by circulating blood for 5 minutes. Eyes were enucleated and immediately fixed with 4% paraformaldehyde for 45 minutes. The retina was incised from the fixed eyeball, cut in the shape of a Maltese cross, and the cut retina was placed on a slide glass, and then observed using a confocal microscope. Retinal vascular leakage was quantified by measuring the strength of FITC-dextran exuding from the entire retinal tissue.

(2) Results

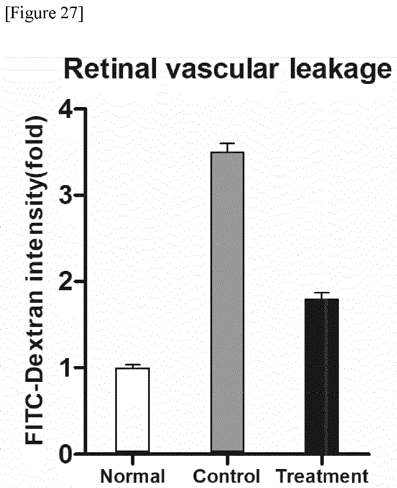

Figure 27 is a result of testing the inhibitory effect on retinal vascular leakage in the streptozotocin-induced diabetic retinopathy rat model. Referring to Fig. 27, it was confirmed that retinal vascular leakage was high in the control group, and in the treatment group retinal vascular leakage was significantly inhibited in the streptozotocin-induced diabetic retinopathy rat model.

**Experimental Example 17. Efficacy of the fractional extract of melissa leaf in Sjogren's Syndrome animal model**

(1) Experimental Method

Experiments were performed using NOD/ShiLt mice, an animal model of Sjogren's syndrome.

In NOD/ShiLt, an animal model of Sjogren's syndrome, 0.5% CMC was administered to the control group, and the fractional extract of melissa leaf according to the Example was administered to the treatment group.

For the administration method, the tear secretion of the mice was analyzed after administering the fractional extract of melissa leaf at 100 mg/kg for 12 weeks. After anesthetizing NOD/ShiLt mice with isoflurane, the tear secretion was measured by using cotton wool soaked in phenol red according to the method of Zoukhri et al. (Exp Eye Res. 2007; 84:894-904).

(2) Results

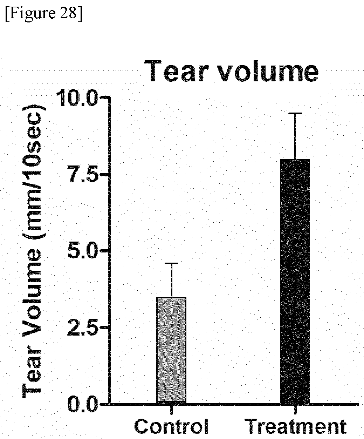

Figure 28 is a graph analyzing tear secretion when the fractional extract of melissa leaf according to the Example was administered using NOD/ShiLt mice, an animal model of Sjogren's syndrome. Referring to Figure 28, tear secretion was significantly increased in the treatment group.

With the above result, it was confirmed that Sjogren's syndrome was improved by administering the fractional extract of melissa leaf according to the Example to increase tear secretion.

**Experimental Example 18. Inhibitory effect of the fractional extract of melissa leaf on glaucoma in a hypertonic saline-induced scar glaucoma model**

(1) Experimental Method

The efficacy of the fractional extract of melissa leaf according to the Example was evaluated in a hypertonic saline-induced scar glaucoma model.

Rats were anesthetized by injecting ketamine-xylazine intraperitoneally, and injected with 50 µl of 1.8 M hypertonic saline into the episcleral veins of the left eye to induce scar tissue in the trabecular meshwork, which induced elevation of intraocular pressure due to resistance of the aqueous humor outflow pathway. The right eye was used as a control, and intraocular pressures was measured in the left and right eyes before glaucoma induction and at intervals of 2 days after glaucoma induction for 2 weeks.

After selecting the rats induced with glaucoma, 0.5% CMC was administered to the control group, and the fractional extract of melissa leaf according to the Example was administered to the treatment group, and glaucoma was not induced in the normal group.

For the administration method, the fractional extract of melissa leaf according to the Example was orally administered to treatment group once a day for 2 weeks from the glaucoma induction date at a dose of 100 mg/kg, and 0.5% CMC was administered to the control group.

(2) Results

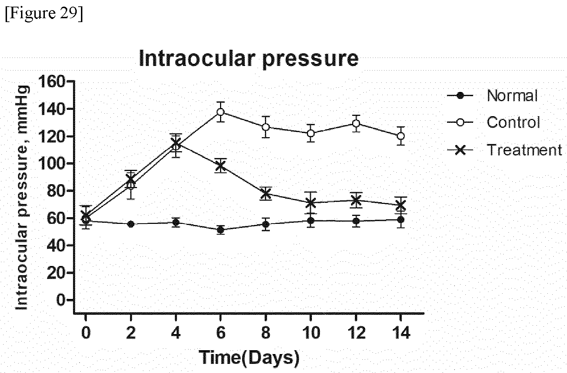

Figure 29 is a graph showing that the fractional extract of melissa leaf according to the Example reduced intraocular pressure in a scar glaucoma model induced by hypertonic saline. Referring to Fig. 29, it was confirmed that in the control group, the intraocular pressure reached the maximum value on the 6th day after the hypertonic saline injection and continued to be maintained, and in the treatment group, the elevated intraocular pressure was significantly alleviated in a glaucoma model with scar in the trabecular meshwork induced by hypertonic saline.

**Claims**

1. A fractional extract of melissa leaf comprising caffeic acid, ethyl 2-(3,4-dihydroxyphenyl) acetate (EDPA), rosmarinic acid methyl ester (RME) and rosmarinic acid, wherein the fractional extract of melissa leaf comprising 0.1 to 5% by weight of caffeic acid, 0.05 to 6% by weight of EDPA, 0.01 to 2% by weight of RME, and 5 to 50% by weight of rosmarinic acid, based on the total of a fractional extract of melissa leaf, and wherein the fractional extract of melissa leaf does not contain rutin.

2. The fractional extract of melissa leaf of claim 1,
wherein the fractional extract of melissa leaf is obtained by an extraction process comprising extracting and concentrating the melissa leaf with 50% to 100% alcohol, suspending in water, and fractionating with ethyl acetate, and the fractional extract of melissa leaf comprises 0.05 to 6% by weight of EDPA (Ethyl 2 -(3,4-dihydroxyphenyl) acetate).

3. A pharmaceutical composition for use in the prevention or treatment of non-alcoholic steatohepatitis or non-alcoholic fatty liver disease comprising a fractional extract of melissa leaf as effective component, wherein the fractional extract of melissa leaf comprises caffeic acid, Ethyl 2-(3,4-dihydroxyphenyl)acetate (EDPA), Rosmarinic acid methyl ester(RME), and rosmarinic acid, wherein the fractional extract of melissa leaf comprising 0.1 to 5% by weight of caffeic acid, 0.05 to 6% by weight of EDPA, 0.01 to 2% by weight of RME, and 5 to 50% by weight of rosmarinic acid, based on the total of a fractional extract of melissa leaf, and wherein the fractional extract of melissa leaf does not contain rutin.

4. A pharmaceutical composition for use in the prevention or treatment of angiogenesis-related diseases or MMP (Matrix metalloproteinase)-mediated diseases, comprising the fractional extract of melissa leaf containing caffeic acid, ethyl 2-(3,4-dihydroxyphenyl) acetate (EDPA), rosmarinic acid methyl ester (RME) and rosmarinic acid as effective component, wherein the fractional extract of melissa leaf comprising 0.1 to 5% by weight of caffeic acid, 0.05 to 6% by weight of EDPA, 0.01 to 2% by weight of RME, and 5 to 50% by weight of rosmarinic acid, based on the total of a fractional extract of melissa leaf, and wherein the fractional extract of melissa leaf does not contain rutin.

5. The pharmaceutical composition for use according to claim 4, wherein the fractional extract of melissa leaf is obtained by an extraction process comprising extracting and concentrating the melissa leaf with 50% to 100% alcohol, suspending in water, and fractionating with ethyl acetate, and the fractional extract of melissa leaf comprises 0.05 to 6% by weight of EDPA (Ethyl 2 -(3,4-dihydroxyphenyl) acetate).

6. The pharmaceutical composition for use according to claim 4, wherein the angiogenesis-related disease or MMP-mediated disease is obesity.

7. The pharmaceutical composition for use according to claim 4, wherein the angiogenesis-related disease or MMP-mediated disease is any one of age-related macular degeneration, diabetic retinopathy, Sjogren's syndrome, and glaucoma.

8. The pharmaceutical composition for use according to claim 4, wherein the angiogenesis-related disease or MMP-mediated disease is any one of endometriosis, psoriasis, arteriosclerosis, inflammatory bowel disease, Alzheimer's disease, periodontal disease, and arthritis, wherein the arthritis is any one of osteoarthritis, degenerative arthritis, dissociative osteochondritis, joint ligament damage, psoriatic arthritis, ankylosing spondylitis, and rheumatoid arthritis.

9. The pharmaceutical composition for use according to claim 4, wherein the angiogenesis-related disease or MMP-mediated disease is cancer growth or cancer metastasis, wherein the cancer is any one of lung cancer, non-small cell lung cancer (NSCL), bronchoalveolar cell lung cancer, stomach cancer, gastrointestinal cancer, liver cancer, bone cancer, pancreatic cancer, skin cancer, head and neck cancer, skin or eye melanoma, ovarian cancer, rectal cancer, colorectal cancer, colon cancer, Breast cancer, fallopian tube carcinoma, endometrial carcinoma, vaginal carcinoma, vulvar carcinoma, esophageal cancer, laryngeal cancer, small intestine cancer, thyroid cancer, parasitic adenocarcinoma, soft tissue sarcoma, urethral cancer, penile cancer, prostate cancer, multiple myeloma, chronic or acute leukemia, childhood Solid tumor, lymphoma, bladder cancer, kidney cancer, renal cell carcinoma, renal pelvic carcinoma, contractile tumor, brainstem glioma and pituitary adenoma.

**Patentansprüche**

1. Fraktionierter Extrakt aus Melissenblättern, umfassend Kaffeesäure, Ethyl-2-(3,4-dihydroxyphenyl)-acetat (EDPA), Rosmarinsäuremethylester (RME) und Rosmarinsäure, wobei der fraktionierte Extrakt aus Melissenblättern 0,1 bis 5 Gew.-% Kaffeesäure, 0,05 bis 6 Gew.-% EDPA, 0,01 bis 2 Gew.-% RME und 5 bis 50 Gew.-% Rosmarinsäure, bezogen auf die Gesamtmenge des fraktionierten Extrakts aus Melissenblättern, umfasst und wobei der fraktionierte Extrakt aus Melissenblättern kein Rutin enthält.

2. Fraktionierter Extrakt aus Melissenblättern nach Anspruch 1, wobei der fraktionierte Extrakt aus Melissenblättern erhalten wird durch ein Extraktionsverfahren umfassend Extrahieren und Konzentrieren der Melissenblätter mit 50 % bis 100 % Alkohol, Suspendieren in Wasser und Fraktionieren mit Ethylacetat, und der fraktionierte Extrakt aus Melissenblättern 0,05 bis 6 Gew.-% EDPA (Ethyl-2-(3,4-dihydroxyphenyl)-acetat) umfasst.

3. Pharmazeutische Zusammensetzung zur Anwendung bei der Vorbeugung oder Behandlung von nichtalkoholischer Steatohepatitis oder nichtalkoholischer Fettlebererkrankung, umfassend einen fraktionierten Extrakt aus Melissenblättern als wirksamen Bestandteil, wobei der fraktionierte Extrakt aus Melissenblättern Kaffeesäure, Ethyl-2-(3,4-dihydroxyphenyl)-acetat (EDPA), Rosmarinsäuremethylester (RME) und Rosmarinsäure umfasst, wobei der fraktionierte Extrakt aus Melissenblättern 0,1 bis 5 Gew.-% Kaffeesäure, 0,05 bis 6 Gew.-% EDPA, 0,01 bis 2 Gew.-% RME und 5 bis 50 Gew.-% Rosmarinsäure, bezogen auf die Gesamtmenge eines fraktionierten Extrakts aus Melissenblättern, umfasst, und wobei der fraktionierte Extrakt aus Melissenblättern kein Rutin enthält.

4. Pharmazeutische Zusammensetzung zur Anwendung bei der Vorbeugung oder Behandlung von Angiogenese-bezogenen Erkrankungen oder von durch MMP (Matrix-Metalloproteinase) vermittelten Erkrankungen, umfassend den fraktionierten Extrakt aus Melissenblättern, der Kaffeesäure, Ethyl-2-(3,4-dihydroxyphenyl)-acetat (EDPA), Rosmarinsäuremethylester (RME) und Rosmarinsäure als wirksame Komponente enthält, wobei der fraktionierte Extrakt aus Melissenblättern 0,1 bis 5 Gew.-% Kaffeesäure, 0,05 bis 6 Gew.-% EDPA, 0,01 bis 2 Gew.-% RME und 5 bis 50 Gew.-% Rosmarinsäure, bezogen auf die Gesamtmenge eines fraktionierten Extrakts aus Melissenblättern, umfasst und wobei der fraktionierte Extrakt aus Melissenblättern kein Rutin enthält.

5. Pharmazeutische Zusammensetzung zur Anwendung nach Anspruch 4, wobei der fraktionierte Extrakt aus Melissenblättern erhalten wird durch ein Extraktionsverfahren umfassend Extrahieren und Konzentrieren der Melissenblätter mit 50 % bis 100 % Alkohol, Suspendieren in Wasser und Fraktionieren mit Ethylacetat, und wobei der fraktioniserte Extrakt aus Melissenblättern 0,05 bis 6 Gew.-% EDPA (Ethyl-2-(3,4-dihydroxyphenyl)-acetat) umfasst.

6. Pharmazeutische Zusammensetzung zur Anwendung nach Anspruch 4, wobei die Angiogenese-bezogene Erkrankung oder die MMP-vermittelte Erkrankung Adipositas ist.

7. Pharmazeutische Zusammensetzung zur Anwendung nach Anspruch 4, wobei die Angiogenese-bezogene Erkrankung oder MMP-vermittelte Erkrankung eine von altersbedingter Makuladegeneration, diabetischer Retinopathie, Sjögren-Syndrom und Glaukom ist.

8. Pharmazeutische Zusammensetzung zur Anwendung nach Anspruch 4, wobei die Angiogenese-bezogene Erkrankung oder die MMP-vermittelte Erkrankung eine von Endometriose, Psoriasis, Arteriosklerose, entzündliche Darmerkrankung, Alzheimer-Krankheit, Parodontitis und Arthritis ist, wobei die Arthritis eine von Osteoarthritis, degenerative Arthritis, dissoziative Osteochondritis, Gelenkbänderschaden, Psoriasis-Arthritis, Spondylitis ankylosans und rheumatoide Arthritis ist.

9. Pharmazeutische Zusammensetzung zur Anwendung nach Anspruch 4, wobei die Angiogenese-bezogene Erkrankung oder die MMP-vermittelte Erkrankung Krebswachstum oder Krebsmetastasierung ist, wobei der Krebs einer von Lungenkrebs, nicht-kleinzelligem Lungenkrebs (NSCL), bronchoalveolärem Lungenkrebs, Magenkrebs, Magen-Darm-Krebs, Leberkrebs, Knochenkrebs, Bauchspeicheldrüsenkrebs, Hautkrebs, Kopf- und Halskrebs, Haut- oder Augenmelanom, Eierstockkrebs, Rektumkarzinom, kolorektalem Karzinom, Kolonkarzinom, Brustkrebs, Eileiterkrebs, Gebärmutterkrebs, Vaginalkrebs, Vulvakrebs, Speiseröhrenkrebs, Kehlkopfkrebs, Dünndarmkrebs, Schilddrüsenkrebs, parasitärem Adenokarzinom, Weichteilsarkom, Harnröhrenkrebs, Peniskrebs, Prostatakrebs, multiplem Myelom, chronischer oder akuter Leukämie, soliden Tumoren im Kindesalter, Lymphom, Blasenkrebs, Nierenkrebs, Nierenzellkarzinom, Nierenbeckenkarzinom, kontraktilem Tumor, Hirnstammgliom und Hypophysenadenom ist.

## Revendications

1. Un extrait fractionnaire de feuille de mélisse comprenant de l'acide caféique, de l'éthyl-2-(3,4-dihydroxyphényl)-acétate (EDPA), de l'ester méthylique d'acide rosmarinique (RME) et de l'acide rosmarinique, dans lequel l'extrait fractionnaire de feuille de mélisse comprend 0,1 à 5 % en poids d'acide caféique, 0,05 à 6 % en poids d'EDPA, 0,01 à 2 % en poids de RME, et 5 à 50 % en poids d'acide rosmarinique, en se basant sur le total d'un extrait fractionnaire de feuille de mélisse, et dans lequel l'extrait fractionnaire de feuille de mélisse ne contient pas de rutine.

2. L'extrait fractionnaire de feuille de mélisse selon la revendication 1,
dans lequel l'extrait fractionnaire de feuille de mélisse est obtenu par un procédé d'extraction comprenant une extraction et une concentration de la feuille de mélisse avec 50 % à 100 % d'alcool, une mise en suspension dans de l'eau, et un fractionnement avec de l'acétate d'éthyle, et l'extrait fractionnaire de feuille de mélisse comprend 0,05 à 6 % en poids d'EDPA (éthyl-2-(3,4-dihydroxyphényl)-acétate).

3. Une composition pharmaceutique pour une utilisation dans la prévention ou le traitement de la stéatohépatite non alcoolique ou la stéatose hépatique non alcoolique comprenant un extrait fractionnaire de feuille de mélisse en tant que composant efficace, dans laquelle l'extrait fractionnaire de feuille de mélisse comprenant de l'acide caféique, de l'éthyl-2-(3,4-dihydroxyphényl)-acétate (EDPA), de l'ester méthylique d'acide rosmarinique (RME) et de l'acide rosmarinique, dans laquelle l'extrait fractionnaire de feuille de mélisse comprend 0,1 à 5 % en poids d'acide caféique, 0,05 à 6 % en poids d'EDPA, 0,01 à 2 % en poids de RME, et 5 à 50 % en poids d'acide rosmarinique, en se basant sur le total d'un extrait fractionnaire de feuille de mélisse, et dans laquelle l'extrait fractionnaire de feuille de mélisse ne contient pas de rutine.

4. Une composition pharmaceutique pour une utilisation dans la prévention ou le traitement de maladies associées à l'angiogenèse ou de maladies médies par une MMP (métalloprotéinase matricielle), comprenant l'extrait fractionnaire de feuille de mélisse contenant de l'acide caféique, de l'éthyl-2-(3,4-dihydroxyphényl)-acétate (EDPA), de l'ester méthylique d'acide rosmarinique (RME) et de l'acide rosmarinique en tant que composant efficace, dans laquelle l'extrait fractionnaire de feuille de mélisse comprend 0,1 à 5 % en poids d'acide caféique, 0,05 à 6 % en poids d'EDPA, 0,01 à 2 % en poids de RME, et 5 à 50 % en poids d'acide rosmarinique, en se basant sur le total d'un extrait fractionnaire de feuille de mélisse, et dans laquelle l'extrait fractionnaire de feuille de mélisse ne contient pas de rutine.

5. La composition pharmaceutique pour une utilisation selon la revendication 4, dans laquelle l'extrait fractionnaire de feuille de mélisse est obtenu par un procédé d'extraction comprenant une extraction et une concentration de la feuille de mélisse avec 50 % à 100 % d'alcool, une mise en suspension dans de l'eau, et un fractionnement avec de l'acétate d'éthyle, et l'extrait fractionnaire de feuille de mélisse comprend 0,05 à 6 % en poids d'EDPA (éthyl-2-(3,4-dihydroxyphényl)-acétate).

6. La composition pharmaceutique pour une utilisation selon la revendication 4, dans laquelle la maladie associée à l'angiogenèse ou la maladie médiée par une MMP est l'obésité.

7. La composition pharmaceutique pour une utilisation selon la revendication 4, dans laquelle la maladie associée à l'angiogenèse ou la maladie médiée par une MMP est l'une quelconque de la dégénérescence maculaire liée à l'âge, la rétinopathie diabétique, le syndrome de Sjögren, et le glaucome.

8. La composition pharmaceutique pour une utilisation selon la revendication 4, dans laquelle la maladie associée à l'angiogenèse ou la maladie médiée par une MMP est l'une quelconque de l'endométriose, le psoriasis, l'artériosclérose, une maladie intestinale inflammatoire, la maladie d'Alzheimer, la parodontite, et l'arthrite, dans laquelle l'arthrite est l'une quelconque de l'arthrose, l'arthrite dégénérative, l'ostéochondrite disséquante, une lésion des ligaments articulaires, l'arthrite psoriasique, la spondylarthrite ankylosante, et la polyarthrite rhumatoïde.

9. La composition pharmaceutique pour une utilisation selon la revendication 4, dans laquelle la maladie associée à l'angiogenèse ou la maladie médiée par une MMP est une croissance cancéreuse ou une métastase cancéreuse, dans laquelle le cancer est l'un quelconque du cancer du poumon, le cancer du poumon non à petites cellules (CPNPC), le cancer du poumon broncho-alvéolaire, le cancer de l'estomac, le cancer gastro-intestinal, le cancer du foie, le cancer des os, le cancer du pancréas, le cancer de la peau, le cancer de la tête et du cou, un mélanome cutané ou oculaire, le cancer de l'ovaire, le cancer du rectum, le cancer colorectal, le cancer du côlon, le cancer du sein, un carcinome des trompes de Fallope, un carcinome de l'endomètre, un carcinome du vagin, un carcinome de la vulve, le cancer de l'œsophage, le cancer du larynx, le cancer de l'intestin grêle, le cancer de la thyroïde, un adénocarcinome parasitaire, un sarcome des tissus mous, le cancer de l'urètre, le cancer du pénis, le cancer de la prostate, le myélome multiple, la leucémie chronique ou aiguë, une tumeur solide pédiatrique, un lymphome, le cancer de la vessie, le cancer du rein, un carcinome à cellules rénales, un carcinome du bassinet du rein, une tumeur contractile, un gliome du tronc cérébral et un adénome hypophysaire.

[Figure 1]

**Picro-sirius red positive area**

[Figure 2]

[Figure 3]

Col1A2 expression

[Figure 4]

[Figure 5]

[Figure 6]

[Figure 7]

Serum levels of ALT and AST

[Figure 8]

Oil-red stained sections

[Figure 9]

HUVEC tube formation

[Figure 10]

HUVEC tube formation

[Figure 11]

**Body weight**

[Figure 12]

Food intake

[Figure 13]

[Figure 14]

[Figure 15]

**Total cholesterol**

[Figure 16]

[Figure 17]

**Ear thickness**

[Figure 18]

[Figure 19]

[Figure 20]

[Figure 21]

**Rheumatoid arthritis score**

[Figure 22]

osteoarthritis weight bearing distribution

[Figure 23]

ulcerative colitis activity index

[Figure 24]

Reference memory test

[Figure 25]

**Probe test**

[Figure 26]

[Figure 27]

**Retinal vascular leakage**

[Figure 28]

[Figure 29]

**Intraocular pressure**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 101055920 A
- WO 101292931 A
- KR 20150035245 A
- WO 2014014271 A1

### Non-patent literature cited in the description

- **FOLKMAN** ; **COTRAN**. Relation of vascular proliferation to tumor growth. *Int Rev Exp Pathol*, 1976, vol. 16, 207-248
- **POLVERINI PJ**. *Critical Reviews in Oral Biology*, 1995, vol. 6 (3), 230-247
- **ARUP DAS et al.** *Progress in Retinal and Eye Research*, 2003, vol. 22, 721-748
- **NICK DI GIROLAMO et al.** *IOVS*, August 2001, vol. 42 (9), 1963-1968
- **PATRICIA LEE et al.** *Survey of ophthalmology*, November 1998, vol. 43 (3), 245-269
- **D.B. HOLLAND et al.** *British Journal of Dermatology*, 2004, vol. 150, 72-81
- **ANTHONY H VAGNUCCI JR et al.** *The Lancet*, 15 February 2003, vol. 361, 605-606
- **BERISLAV V. ZLOKOVIC**. *Trends in Neuroscience*, April 2005, vol. 28 (4), 202-208
- **JAAP G. NEELS et al.** *The FASEB Journal*, 14 April 2004
- **D.L. CRANDALL et al.** *Microcirculation*, 1997, vol. 4, 211-232
- **G. VOROS et al.** *Endocrinology*, 2005, vol. 146, 4545-4554
- **M.A. RUPNICK et al.** *PNAS*, 2002, vol. 99, 10730-10735
- **E. BRAKENHIELM et al.** *Circ. Res.*, 2004, vol. 94, 1579-1588
- **H.R. LIJNEN et al.** *Arterioscler Thromb Vasc Biol.*, 2002, vol. 22, 374-379
- **D. DEMEULEMEESTER et al.** *Biochem. Biophys. Res. Commun.*, 2005, vol. 329, 105-110
- **ZOUKHRI et al.** *Exp Eye Res.*, 2007, vol. 84, 894-904